(19) Europäisches Patentamt
European Patent Office
Office européen des brevets

(11) EP 4 259 610 B1

(12) EUROPEAN PATENT SPECIFICATION

(45) Date of publication and mention of the grant of the patent:
**18.09.2024 Bulletin 2024/38**

(21) Application number: **21830673.6**

(22) Date of filing: **09.12.2021**

(51) International Patent Classification (IPC):
***C07D 265/16*** *(2006.01)* ***C07D 413/06*** *(2006.01)*
***C09J 177/00*** *(2006.01)* ***C08G 65/48*** *(2006.01)*
***C08G 73/02*** *(2006.01)*

(52) Cooperative Patent Classification (CPC):
**C07D 265/16; C07D 413/06; C08G 14/06; C08G 73/0233**

(86) International application number:
**PCT/EP2021/084927**

(87) International publication number:
**WO 2022/122882 (16.06.2022 Gazette 2022/24)**

(54) **CATALYSTS FOR BENZOXAZINE**

BENZOXAZIN-KATALYSATOREN

CATALYSEURS DE BENZOAXINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.12.2020 LU 102316**

(43) Date of publication of application:
**18.10.2023 Bulletin 2023/42**

(73) Proprietor: **Luxembourg Institute of Science and Technology (LIST)**
**4362 Esch-sur-Alzette (LU)**

(72) Inventors:
- **VERGE, Pierre**
**4362 Esch-sur-Alzette (LU)**
- **ADJAOUD, Antoine**
**4362 Esch-sur-Alzette (LU)**
- **PUCHOT, Laura**
**4362 Esch-sur-Alzette (LU)**

(74) Representative: **Lecomte & Partners**
**76-78, rue de Merl**
**2146 Luxembourg (LU)**

(56) References cited:
**WO-A1-2010/018198**

- **LIU CHAO ET AL: "Catalyst effects on the ring-opening polymerization of 1,3-benzoxazine and on the polymer structure", POLYMER, vol. 54, no. 12, 1 May 2013 (2013-05-01), GB, pages 2873 - 2878, XP055801631, ISSN: 0032-3861, DOI: 10.1016/j.polymer.2013.03.063**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 259 610 B1

## Description

### Field of invention

The invention is directed to the field of catalysts for benzoxazine compounds based on transesterification mechanism.

### Technical field

Benzoxazine gives thermosetting properties such as high-temperature and flammability performance, high strength, thermal stability, low water absorption, chemical resistance, low melt viscosities, and near-zero shrinkage. However, they require a lot of time and a high temperature to be polymerized. It impedes their use in many industrial sectors, as for instance for composite elaboration where high production cadence are required.

To tackle this drawback, catalysts can be added to decrease the requirements essential for the curing of benzoxazine. Among them lithium, zinc, sodium, or ammonium salts contribute to lower the onset of polymerization ($T_{onset}$) to reach for the best cases $T_{onset}$= 160°C.

However, these catalysts are not covalently linked to the network and can be released with time. In addition, most of them are harmful. Carboxylic acid-containing benzoxazine were also used as catalysts for thermal polymerization of benzoxazines. Other documents mention reaction of benzoxazine-base phenolic resins with strong and weak carboxylic acids and phenols as catalysts.

However, these catalytic systems are not enough stable under heating.

WO 2010/018198 A1 relates to polymerizable compositions, comprising at least two specific benzoxazines and cured products obtained from the polymerizable compositions, intended to increase the polymerization rate of polymerizable compositions via incorporation of at least one specific benzoxazine. Improvements remain, however, desirable.

LIU CHAO ET AL, "Catalyst effects on the ring-opening polymerization of 1,3-benzoxazine and on the polymer structure", POLYMER, GB, (20130501), vol. 54, no. 12, doi:10.1016/j.polymer.2013.03.063, ISSN 0032-3861, pages 2873 - 2878 discloses catalysts for the polymerization of phenoxazines which are phenoxazine derivatives. Improvements remain, however, also desirable.

### Disclosure of the invention

The invention has for technical problem to provide a solution to at least one above mentioned drawback. More specifically, the invention has for generic technical problem to provide a catalytic system for polymerization of benzoxazine monomers.

For this purpose, the invention is directed to a benzoxazine containing free aliphatic hydroxyl groups and a monoester of formula (I)

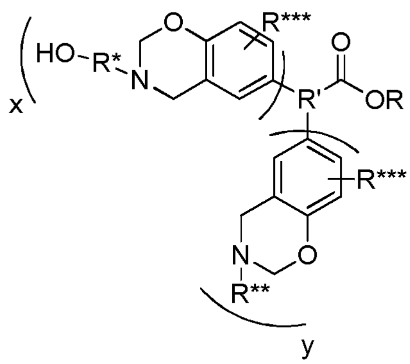

wherein

R is selected from the group consisting of a linear or branched $C_1$-$C_{12}$ alkyl or alkoxy group, a linear or branched $C_2$-$C_6$ alkenyl or alkylenoxy group, a substituted or unsubstituted linear or branched $C_2$-$C_6$ alkynyl group, a cyclo($C_3$-$C_6$ alkyl) group, a heteocyclo($C_3$-$C_6$ alkyl), a linear or branched $C_1$-$C_6$ alkyl or $C_2$-$C_6$ alkenyl substituted or unsubstituted phenyl group and a $(CH_2)_{n3}$-phenyl group, wherein n3 is an integer from 1 to 10;

R' is selected from the group consisting of at least one of -CH, a C-$(CH_2)_{n3}$-$CH_3$ group, a C-$(CH_2)_{n3}$-CH-$(CH_3)_2$

group, a $C-(CH_2)_{n3}-(CHZ)_{n4}-(CH_3)_2$ group, a $C-(CH_2)_{n3}-(CHZ)_{n4}-(CH_2)_{n3}-CH_3$ group, a $C-(CHZ)_{n4}-(CH_2)_{n3}-CH_3$ group, a $C-(CHZ)_{n4}-[(CH_2)_{n3}-CH_3]_2$ group, a C-substituted or unsubstituted $C_2$-$C_6$ linear or branched alkenyl group, a linear or branched $C_1$-$C_6$ alkyl substituted or unsubstituted phenyl or phenyl including at least one hetero atom selected from N, O and S, a $C-(CH_2)_{n3}-C_1-C_6$ linear or branched alkyl substituted or unsubstituted phenyl or phenyl including at least one heteroatom selected from N, O and S, a $C-(CH_2)_{n3}-C_1-C_6$ linear or branched alkyl substituted or unsubstituted phenyl or phenyl including at least one heteroatom selected from N, O and $S-(CH_2)_{n3}-CH_3$, a $C-(CH_2)_{n3}-C_1-C_6$ linear or branched alkyl substituted or unsubstituted phenyl or phenyl including at least one heteroatom selected from N, O and $S-(CH_2)_{n3}-CH-(CH_3)_2$, a $C-(CH_2)_{n3}-C_1-C_6$ linear or branched alkyl substituted or unsubstituted phenyl or phenyl including at least one heteroatom selected from N, O and $S-(CH_2)_{n3}-(CHZ)_{n4}-(CH_3)_2$ group, a $C-(CH_2)_{n3}-C_1-C_6$ linear or branched alkyl substituted or unsubstituted phenyl or phenyl including at least one heteroatom selected from N, O and $S-(CHZ)_{n4}-(CH_2)_{n3}-CH_3$ group and a $C-(CH_2)_{n3}-(CHZ)_{n4}-C_1-C_6$ linear or branched alkyl substituted or unsubstituted phenyl or phenyl including at least one heteroatom selected from N, O and $S-(CH_2)_{n3}-CH_3$ group, wherein n3 and n4, independently, are an integer from 1 to 10 and Z is selected from the group consisting in a linear or branched $C_1$-$C_6$ alkyl or alkoxy group, linear or branched $C_2$-$C_6$ alkenyl or alkylenoxy group and a linear or branched $C_1$-$C_6$ alkyl or $C_2$-$C_6$ alkenyl substituted or unsubstituted phenyl group, and at least one O atom is present or not between two adjacent C, or R' is omitted. "R' is omitted" means that the ester moiety is directly linked to the aromatic ring, with y = 0.

R* is selected from the group consisting of a linear or branched $C_1$-$C_{20}$, preferably $C_1$-$C_6$, alkyl or alkoxy group, a cyclo($C_3$-$C_6$ alkyl) group, a heteocyclo($C_3$-$C_6$ alkyl) group, wherein the hetero atom is selected from N, S, and O, linear or branched $C_2$-$C_6$ alkenyl or alkylenoxy group, substituted or unsubstituted linear or branched $C_2$-$C_6$ alkynyl group, a linear or branched $C_1$-$C_6$ alkyl or $C_2$-$C_6$ alkenyl substituted or unsubstituted phenyl group, a $(CH_2)_{n3}$-phenyl group and $-(CH_2)_{n3}-O-(CH_2)_{n4}$, wherein n3 and n4, independently, are an integer from 1 to 10;

R** is the same as R* and further includes a member selected from a O-, N- or $S-(CH_2)_{n3}-CH-(CH_3)_2$ group, a O-, N- or $S-(CH_2)_{n3}-(CHZ)_{n4}-(CH_3)_2$ group, a O-, N- or $S-(CH_2)_{n3}-(CHZ)_{n4}-(CH_2)_{n3}-CH_3$ group, a O-, N- or $S-(CHZ)_{n4}-(CH_2)_{n3}-CH_3$ group, a O-, N- or $S-(CHZ)_{n4}-[(CH_2)_{n3}-CH_3]_2$ group and a O-substituted or unsubstituted $C_2$-$C_6$ linear or branched alkynyl group, Z being as defined for R', a $-(CH_2)_{n3}-C\equiv N$ group, a polycyclic aromatic or heteroaromatic hydrocarbon, such as naphthalene, anthracene, fluorene, phenanthrene, optionally substituted by a linear or branched $C_1$-$C_6$ alkyl or alkoxy group, a cyclo($C_3$-$C_6$ alkyl) group, a heterocyclo($C_3$-$C_6$alkyl) group, a linear or branched $C_2$-$C_6$ alkenyl or alkylenoxy group, or by a substituted or unsubstituted linear or branched $C_2$-$C_6$ alkynyl group, wherein n3 and n4, independently, are an integer from 1 to 10;

R*** is selected from the group consisting in H, OH and a O-linear or branched $C_1$-$C_6$ alkyl group, and further includes a linear or branched $C_1$-$C_{15}$ alkyl group or a $C_2$-$C_{15}$ alkenyl group or

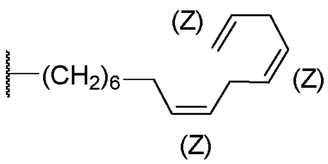 , 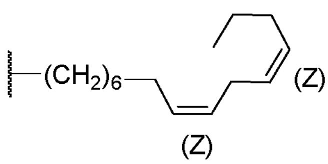 , 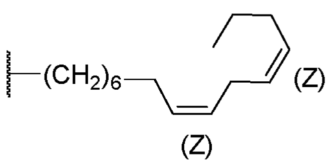

or

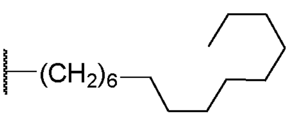 ;

and

x value is of from 0 to 1 and y value is 1-x, preferably of from 0,1 to 1, more preferentially from 0,5 to 1.

In the context of the invention, x and y represent the proportion between benzoxazine groups when prepared from an aminoalcohol and the other amine(s). In other words, x and y can be defined as

$$x = \frac{n_{aminoalcohol}}{n_{amines}^{total}}$$

$$y = \frac{n_{amines}}{n_{amines}^{total}}$$

wherein $n_{amines}^{total} = n_{amines} + n_{aminoalcohol}$, and $n_{aminoalcohol}$ being the number of aminoalcohol per molecules of catalyst, $n_{amines}$ represent the number of amines (excepting the number of aminoalcohol) per molecule of catalyst and $n_{amines}^{total}$ is the total number of amino groups per molecule of catalyst.

The monoester-benzoxazine-of formula (I) are including a hydroxyl group, an ester bond and a benzoxazine ring, combination of which is the essential feature of the invention. The Applicant has shown that said benzoxazine monomers may advantageously lead to the transesterification occurring between the OH and the ester bonds, triggering the polymerization of the benzoxazine. The characteristic tertiary amine of the benzoxazine ring will then catalyze the transesterification reaction, which will catalyze the benzoxazine Ring-Opening Polymerisation (ROP), leading to a polybenzoxazine derivative. It could be considered as a virtuous loop. Consequently, the monoester benzoxazine-containing free aliphatic hydroxyl groups is a catalyst for ROP reaction, as self-polymerisation. In addition, such monoester benzoxazine, once introduced in a traditional and commercial benzoxazine, triggers the polymerization at lower temperature and shorter times. Such monoester benzoxazines involve a green transesterification with high yield, which is solventless and not harmful.

According to the compounds of formula (I), R' is structure making the bridge between the ester bond and the phenolic ring, and R*** is a substituent of the phenolic ring. It is preferred that R*** is on *meta* position(s).

R may preferably be selected from the group consisting of a linear or branched $C_1$-$C_4$ alkyl or alkoxy group, a linear or branched $C_2$-$C_4$ alkenyl or alkylenoxy group, an unsubstituted linear or branched $C_2$-$C_4$ alkynyl group, an unsubstituted phenyl group and a $(CH_2)_{n3}$-phenyl group, wherein n3 is an integer from 1 to 6.

R' may preferably be selected from the group consisting of at least one of -CH, a $C$-$(CH_2)_{n3}$-$CH_3$ group, a $C$-$(CH_2)_{n3}$-$CH$-$(CH_3)_2$ group, a $C$-$(CH_2)_{n3}$-$(CHZ)_{n4}$-$(CH_3)_2$ group, $C$-$(CH_2)_{n3}$-$(CHZ)_{n4}$-$(CH_2)_{n3}$-$CH_3$ group, $C$-$(CHZ)_{n4}$-$(CH_2)_{n3}$-$CH_3$ group, a $C$-$(CHZ)_{n4}$-$[(CH_2)_{n3}$-$CH_3]_2$ group, a C-substituted or unsubstituted $C_2$-$C_4$ linear or branched alkenyl group, an unsubstituted phenyl or phenyl including at least one heteroatom selected from N, O and S, a $C$-$(CH_2)_{n3}$-unsubstituted phenyl or phenyl including at least one heteroatom selected from N, O and S, a $C$-$(CH_2)_{n3}$-unsubstituted phenyl or phenyl including at least one heteroatom selected from N, O and $S$-$(CH_2)_{n3}$-$CH_3$, a $C$-$(CH_2)_{n3}$-unsubstituted phenyl or phenyl including at least one heteroatom selected from N, O and $S$-$(CH_2)_{n3}$-$CH$-$(CH_3)_2$, a $C$-$(CH_2)_{n3}$-unsubstituted phenyl or phenyl including at least one heteroatom selected from N, O and $S$-$(CH_2)_{n3}$-$(CHZ)_{n4}$-$(CH_3)_2$ group, a $C$-$(CH_2)_{n3}$-unsubstituted phenyl or phenyl including at least one heteroatom selected from N, O and $S$-$(CHZ)_{n4}$-$(CH_2)_{n3}$-$CH_3$ group and a $C$-$(CH_2)_{n3}$-$(CHZ)_{n4}$-unsubstituted phenyl or phenyl including at least one heteroatom selected from N, O and $S$-$(CH_2)_{n3}$-$CH_3$ group, wherein n3 and n4, independently, are an integer from 1 to 6 and Z is selected from the group consisting in a linear or branched $C_1$-$C_4$ alkyl or alkoxy group, linear or branched $C_2$-$C_4$ alkenyl or alkylenoxy group and an unsubstituted phenyl group, and at least one O atom is present or not between two adjacent C.

R* may preferably be selected from the group consisting of a linear or branched $C_1$-$C_6$, preferably $C_1$-$C_6$, alkyl or alkoxy group, a linear or branched $C_2$-$C_4$ alkenyl or alkylenoxy group, an unsubstituted linear or branched $C_2$-$C_4$ alkynyl group, an unsubstituted phenyl group, a $(CH_2)_{n3}$-phenyl group and $-(CH_2)_{n3}$-$O$-$(CH_2)_{n4}$, wherein n3 and n4, independently, are an integer from 1 to 6;

Preferably, R** is the same as R* and may further include a member selected from O-, N- or $S$-$(CH_2)_{n3}$-$CH$-$(CH_3)_2$ group, a O-, N- or $S$-$(CH_2)_{n3}$-$(CHZ)_{n4}$-$(CH_3)_2$ group, a O-, N- or $S$-$(CH_2)_{n3}$-$(CHZ)_{n4}$-$(CH_2)_{n3}$-$CH_3$ group, a O-, N- or $S$-$(CHZ)_{n4}$-$(CH_2)_{n3}$-$CH_3$ group, a O-, N- or $S$-$(CHZ)_{n4}$-$[(CH_2)_{n3}$-$CH_3]_2$ group and a O-substituted or unsubstituted $C_2$-$C_4$ linear or branched alkynyl group, Z being as defined above, a - $(CH_2)_{n3}$-$C\equiv N$ group, a cyclo($C_3$-$C_4$ alkyl) group, a heteocyclo($C_3$-$C_4$ alkyl) group, a polycyclic aromatic or heteroaromatic hydrocarbon, wherein the hetero atom is selected from N, S, and O, such as naphthalene, anthracene, fluorene, furane, which may optionally be substituted by a linear or branched $C_1$-$C_4$ alkyl or alkoxy group, a linear or branched $C_2$-$C_4$ alkenyl or alkylenoxy group, or by a substituted or unsubstituted linear or branched $C_2$-$C_4$ alkynyl group, wherein n3 and n4, independently, are an integer from 1 to 6;

R*** may preferably be selected from the group consisting in H, OH and a O-linear or branched $C_1$-$C_4$ alkyl group, and may further include a linear or branched $C_1$-$C_{10}$ alkyl group or $C_2$-$C_{10}$ alkenyl group or

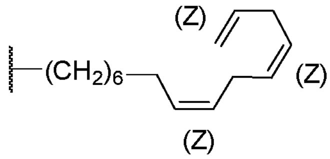,
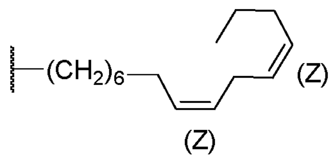,
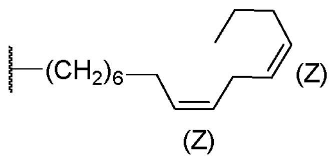

or

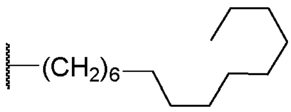.

More preferably, R may be selected from the group consisting of groups $-CH_3$, $-(CH_2)_{n3}-CH_3$, $-(CH_2)_{n3}-CH-[(CH_2)_{n3}-CH_3]_2$, $-C(CH_3)_3$, $-(CH_2)_{n3}-(C_6H_5)$, $-(CH_2)_{n3}-CH=CH_2$ and $-(CH_2)_{n3}-C\equiv CH$, wherein n3 is an integer from 1 to 5.

More preferably, R' may be selected from the group consisting of groups -CH, $C(CH_3)$, $-C-CH(CH_2CH_3)$, $-C(CH_2CH_2CH_3)$, $-C-CH_2(CH_2)_3CH_3$, $-C-CH_2(CH_2)_4CH_3$, $-C(C_6H_5)$, $-C(CH_3)CH_2$, $C(CH_3)CH_2CH_2$ and $-C(C_6H_5)CH_2-CH_3$.

More preferably, R* may be selected from the group consisting of groups $-CH_3$, $-(CH_2)_{n3}-CH_3$, $-(CH_2)_{n3}-CH-[(CH_2)_{n4}-CH_3]_2$, $-C(CH_3)_3$, $(CH_2)_{n3}-(C_6H_5)$, $-(CH_2)_{n3}-CH=CH_2$, $-(CH_2)_{n3}-C\equiv CH$, $-(CH_2)_{n3}-O-(CH_2)_{n4}$ wherein n3 and n4 independently are integer from 1 to 4, phenyl, and $-(CH_2)_3$-phenyl.

More preferably, R** can be the group R*, or may be selected from the group consisting of groups $CH_3$, $-(CH_2)_{n3}-CH_3$, $-(CH_2)_{n3}-CH-[(CH_2)_{n4}-CH_3]_2$, $-C(CH_3)_3$, $(CH_2)_{n3}-(C_6H_5)$, $-(CH_2)_{n3}-CH=CH_2$, $-(CH_2)_{n3}-C\equiv CH$, $O-(CH_2)_{n3}-C\equiv CH$, $O-(CH_2)_{n3}-C\equiv N$, $(CH_2)_{n3}-C\equiv N$, and $-(CH_2)_{n3}$-substituted or unsubstituted furan, phenyl, and wherein n3 and n4, independently, are integer from 1 to 4.

R*** may preferably be selected from the group consisting in H, OH and a O-linear or branched $C_1$-$C_3$ alkyl group, and may further include linear or branched $C_1$-$C_6$ alkyl group or $C_2$-$C_6$ alkenyl group or

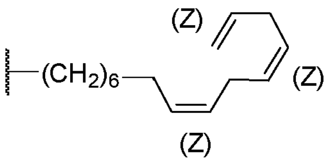,
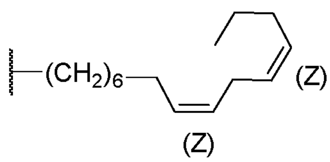,
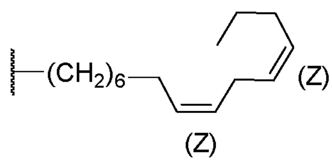

or

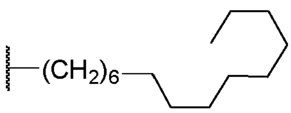.

More preferably R*** is H.

The depicted R, R', R*, R**, R*** and combination thereof may be used independently one from the other.

The expression "substituted" as defined above, relates to the presence of some linear or branched alkyl groups in $C_1$-$C_6$.

The invention also relates to a process (1) for producing a benzoxazine-containing free aliphatic hydroxyl groups and monoester of formula (I) comprising the following steps of:

a) reaction of a phenolic acid derivative of formula (II), comprising at least one R*** group,

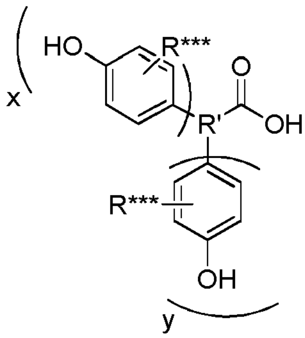

(II)

with a monofunctional oligomer or molecule of formula (III)

R-OH (III)

at a temperature of from 80°C to 200°C, during 12h-48h, in a presence of a Bronsted type acid catalyst, resulting in a phenol terminated oligomer or molecule of formula (IV)

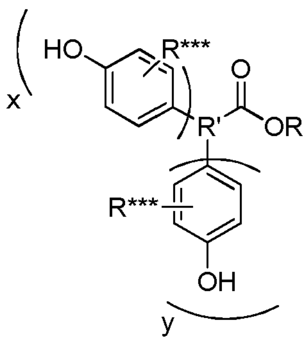

(IV)

and

b) reaction of the phenol terminated oligomer or molecule of formula (IV) with a mixture of

- an amino-alcohol of formula (V):

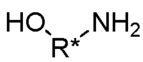

(V)

- a primary amine derivative of formula (VI),

$R^{**}-NH_2$ (VI),

and
- paraformaldehyde of formula (VII)

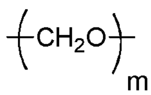

m=8-100

at a temperature range of from 80°C to 100°C, from 1h to 48h, under stirring, wherein R, R', R*, R**, R***, x and y are, independently, as defined above, with the proviso that when at least one R*** of the phenolic acid derivative is in *ortho* position with regard to -OH group, then R*** is H.

The monoester benzoxazine-containing free aliphatic hydroxyl groups of formula (I) is synthesized in two stages. The first step (step a)) corresponds to a Fischer esterification between a monofunctional molecule or oligomer terminated with an aliphatic hydroxyl group and a phenolic acid derivative in presence of Bronsted type acid catalyst introduced in catalytic amount. The reagents are reacted together at 80° to 200°C and under mechanical stirring for 12-48 hours. The second step (step b)) corresponds to a Mannich condensation of the freshly prepared ester and phenol functionalized molecule with paraformaldehyde, a linear bifunctional molecule amino-alcohol, and a primary amine derivative, wherein x is between 0 and 1, and y = 1-x.

The monoester-benzoxazine of the invention is advantageously suited for obtaining polybenzoxazine derivatives by a polymerization involving the benzoxazine ring opening and a self-polymerisation under heat.

Consequently, the Applicant has shown that said monoester-benzoxazines may advantageously lead to the transesterification occurring between the OH and the ester bonds trigger the opening of benzoxazine rings, leading to the formation of a tertiary amine. This tertiary amine will then catalyse the transesterification reaction, which will catalyse the benzoxazine Ring-Opening Polymerisation (ROP), leading to a polybenzoxazine derivative.

The Applicant has shown that the specific starting reactants are providing a benzoxazine monoester containing free aliphatic hydroxyl groups, which in turn, after polymerization, is giving the polybenzoxazine derivatives comprising polymerized benzoxazine.

The benzoxazine ring, obtained from the reaction of the specific derivatives which allows the material to be cross-linked (processed) upon heating, helps the reprocessing thanks to the exchangeable and reversible ester bonds, and free aliphatic hydroxyl groups. Also, the benzoxazine ring moiety gives thermosetting properties such as high-temperature and flammability performance, high strength, thermal stability, low water absorption, chemical resistance, low melt viscosities, and near-zero shrinkage.

The phenolic acid derivative (formula (II)) may include at least one R*** group, more preferably of from 1 to 4, related to the substitution of the phenolic ring, and the R group related to the nature of the bridge between the ester bonds and the phenolic ring.

It is advantageous that the phenolic acid derivative (formula (II)) bears R*** groups that does not interfere with the phenolic ortho-position to avoid steric hindrance that may adversely impact the kinetic of step a) or the oxazine ring closure of step b). Accordingly, R*** groups may then be advantageously selected to bear short chain groups, as previously defined, with the proviso that R*** in phenolic ortho-position is H.

In some embodiments, there could be two phenolic ortho-positions, each of which is H for the R*** group.

As an example, when the phenolic acid derivative is a monophenol, then x = 1 and y = 0, whereas when said phenolic acid derivative is a diphenol, then x = 0, 5 and y = 0,5.

In the context of the invention, "derivative" in "phenolic acid derivative" means a compound bearing phenol and carboxylic acid moieties. Accordingly, "phenolic acid derivative" also means an organic compound bearing phenol and carboxylic acid moieties without being limitative.

The phenolic acid derivative may be more preferably selected from the group consisting of mono-, di-, tri-hydroxybenzoic acid derivatives, anacardic acid derivatives, hydroxycinnamic acid derivatives, aliphatic X-hydroxyphenyl acid derivatives, wherein X is 2-4 and aliphatic diphenolic acid derivatives, or mixtures thereof.

Most preferred aliphatic mono-, di-, tri-hydroxybenzoic acid derivatives may be of formula (VIII)

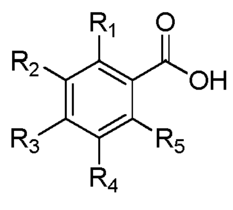

(VIII)

wherein R' is omitted, and the $R_1$ to $R_5$ groups corresponding to R***, and one among $R_1$-$R_5$ is a hydroxyl group, then at least one H is in phenolic ortho-position, the rest being defined above.

Especially, in formula (VIII), at least one combination of $R_1$ to $R_5$ may be selected from the group consisting of:

$R_1$= OH, $R_2$=H, $R_3$=$R_4$=$R_5$= H or $CH_3$ or $CH_2$-$CH_3$ or $CH_2$-$CH_2CH_3$ or $CH_2$-$CH(CH_3)_2$,

$R_2$= OH, $R_1$=$R_3$=H, $R_4$=$R_5$= H or $CH_3$ or $CH_2$-$CH_3$ or $CH_2$-$CH_2CH_3$ or $CH_2$-$CH(CH_3)_2$,

$R_3$= OH, $R_2$=$R_4$=H, $R_1$=$R_5$= H or $CH_3$ or $CH_2$-$CH_3$ or $CH_2$-$CH_2CH_3$ or $CH_2$-$CH(CH_3)_2$,

$R_4$= OH, $R_3$=$R_5$= H, $R_1$=$R_2$= H or $CH_3$ or $CH_2$-$CH_3$ or $CH_2$-$CH_2CH_3$ or $CH_2$-$CH(CH_3)_2$,

$R_5$ = OH, $R_1$=H, $R_2$=$R_3$=$R_4$= H or $CH_3$ or $CH_2$-$CH_3$ or $CH_2$-$CH_2CH_3$ or $CH_2$-$CH(CH_3)_2$.

Most preferred anacardic acid derivatives may be of formula (IX),

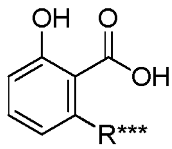

(IX)

wherein R' is omitted, and R*** is

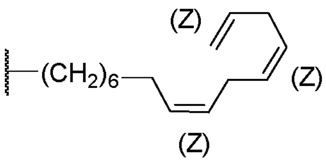

,

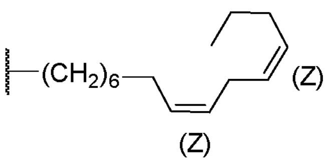

,

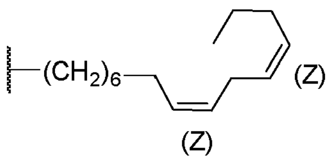

or

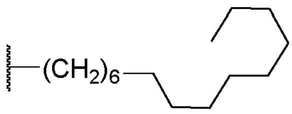

.

Most preferred hydroxycinnamic acid derivatives may be of formula (X)

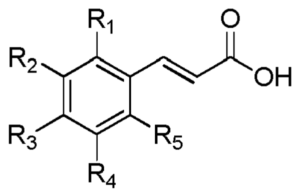

(X)

wherein $R_1$ to $R_5$ are corresponding to R***, and one among $R_1$-$R_5$ is a hydroxyl group and at least one H being in phenolic ortho-position, the rest being H and, optionally an aliphatic alkyl or alkoxy group of $C_1$-$C_6$.

Most preferred aliphatic X-hydroxyphenyl acid derivatives may be selected from the group consisting of aliphatic hydroxyphenyl acids (X= 1) di-hydroxyphenyl acids (X=2), aliphatic tri-hydroxyphenyl acids (X=3) and aliphatic tetra-hydroxyphenyl acids (X=4), or mixtures thereof, of formula (XI)

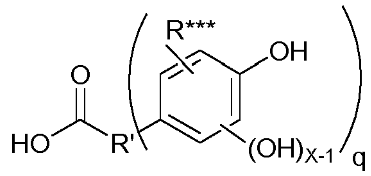

(XI)

wherein R' and R*** are as previously defined.

The number of R*** in the ring is depending on the number of hydroxyl groups in the ring, and at least one R***, preferably of from 1 to 3, is H towards the phenolic *ortho*-position, and the integer q is comprised between 1 and 3.

Most preferred diphenolic acid derivatives are of formula (XII)

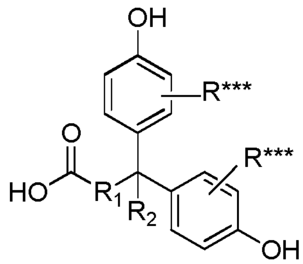

(XII)

wherein

in said formula, $-R_1-C-R_2-$ moiety is R';

on each respective phenolic cycle, at least one R***, preferably of from 1 to 3, is H towards the phenolic ortho-position, and otherwise R*** is as defined previously, and

$R_2$ is selected from the group consisting of $(CH_2)_{n5}CH_3$, $(CH_2)_{n4}$-(aliphatic $C_1$-$C_6$ aliphatic alkyl or alkoxy substituted or unsubstituted phenyl group), wherein $n_5$ is an integer from 1 to 12, preferably from 1 to 10, more preferably from 1 to 6, and $(CH_2)_{n5}(CH(CH_3)_2)$, and

$R_1$ is selected from the group consisting of $(CH_2)_{n6}$, wherein $n_6$ is an integer from 1 to 3, $CH(CH_2)_{n6}(CH_3)$, $CH(CH(CH_3)_2)$ and $C(CH_3)_2$, $(CH_2)_{n6}$ being the most preferred to lower the steric hindrance.

Most preferred is the 4,4-Bis(4-hydroxyphenyl)valeric acid (VA or DPA).

The monofunctional oligomer or molecule of formula (III) is an alcohol derivative, R-OH. R is as defined above.

The Bronsted acid type catalyst are those commonly used for a Fischer esterification include para-toluene sulfonic acid (p-TSA), anhydrous chlorhydric acid (HCl), phosphoric acid ($H_3PO_4$), methanoic acid ($CH_3$-$CO_2H$), sulfuric acid, tosylic acid, and Lewis acids such as scandium(III) triflate. The content of catalyst may typically be of from 0,5 wt% to 2 wt%.

The step a) may advantageously be carried out at a temperature in the range of 80°C to 150°C, most preferably of from 100°C to 140°C for the best synthesis yields of higher than 95%, the chosen temperature being dependent on the nature of the reactants, i.e. the melting temperature of said reactant medium.

Advantageously, step a) is performed of from 12h to 24h for the highest yield of at least 95%, and the duration is based on the kinetic of the reaction.

The respective stoichiometry of starting reactants on step a), phenolic acid derivative: monofunctional molecule or oligomer may preferably be 1,0-3,0 eq.:1,0 eq, resulting in an 1,0 eq. of phenol terminated oligomer or molecule.

The second step of the process, step b), corresponds to a Mannich condensation type reaction of the phenol terminated oligomer or molecule of step a) (formula (IV)) with an amino-alcohol (formula (V)), a primary amine derivative of formula (VI) and the paraformaldehyde, optionally in presence of a catalyst. Thus, since step b) does not require the use of an external catalyst, step b) is implemented in an easier way.

Advantageously, the amino-alcohol of formula (V) includes R* group, a linear amino-alcohol with a primary amine moiety and an aliphatic hydroxyl moiety for obtaining with the highest yield and the best reaction conditions the oxazine ring.

The amino-alcohol of formula (V) may be more preferably selected from the group consisting of 2-aminoethanol,

2-(2-aminoethoxy)ethanol, 2-amino-2-methylpropanol, 5-aminopentan-1-ol, heptaminol and diglycolamine, or mixtures thereof.

The primary amine derivative includes the R** group, as defined above.

In the context of the invention, "derivative" in "primary amine derivative" means a compound bearing a primary amine moiety. Accordingly, "primary amine derivative" also means an organic compound bearing a primary amine group without being limitative.

Primary amine derivatives bear R** having the definition of R* and may be further selected from the group consisting in allylamine, methylamine, ethylamine, propylamine, butylamine, isopropylamine, hexylamine, cyclohexylamine, stearylamine, 2-aminofluorene, aminophenyl acetylene, propargyl ether aniline, 4-aminobenzonitrile, furfurylamine and aniline, or mixtures thereof.

The temperature range of step b) may preferably be of from 80°C to 95°C, more allowing to obtain the highest conversion yields of at least 75%.

Advantageously, step b) is performed from 1h to 3h, for the highest yield of at least 75%.

One advantage of the invention, is that step b) is performed without any catalyst.

The respective stoichiometry of starting reactants on step b), phenol terminated oligomer or molecule:amino-alcohol:primary amine derivative:paraformaldehyde may preferably be 1,0 eq.:x(1,0 eq-18,0 eq): y(1,0 eq-18,0 eq):2,0-36,0 eq, resulting in an 1,0 eq. of the monoester-benzoxazine, wherein x and y are as previously defined. It is also assumed that the higher is x, the more efficient is the ROP.

The specific range stoichiometry is depending on the respective equivalent proportion of the amino-alcohol and of the primary amine derivative. It should be pointed out that there is a minimal quantity required for the reaction to occur. For instance, the relative molar % of amino-alcohol vs the relative molar % of primary amine derivative is 10 molar% vs 90 molar% respectively. It also means that primary amine may be omitted (0 molar%) and amino-alcohol may only be used (100 molar%). Besides, the selected stoichiometry ranges of both amino-alcohol/amine and paraformaldehyde preferably avoids the formation of either reaction linear and/or aliphatic by-products, such as oxazolidine, triaza derivatives, or condensation derivatives.

Preferentially, the whole process is performed with bio-based reactants.

The monoester-benzoxazine synthesis may most preferably be solventless, even though a solvent could be added for the dissolution of starting reactants. The process involves a one-step synthesis, which is one of the advantages of the invention.

Advantageously, the whole synthesis may generally not require any further monomer purification for the invention to be implemented. However, the purification of the monomer, if needed, may be performed by any known technic (vacuum, distillation etc.)

The reaction mixtures of both steps a) and b) are stirred using a classical mechanical stirrer, or any non-limitative means.

The process may be implemented by any known means known to the one skilled in the art, using appropriate vessel either at lab scale or at industrial scale.

The invention also relates to a benzoxazine Ring-Opening Polymerisation (ROP) catalyst comprising a benzoxazine containing free aliphatic hydroxyl groups monoester of formula (I).

The Applicant has shown that said monoester-benzoxazines may advantageously lead to the transesterification occurring between the OH and the ester bonds, triggering the polymerization of the benzoxazine, leading to the formation of a tertiary amine. This tertiary amine will then catalyse the transesterification reaction, which will catalyse the benzoxazine Ring-Opening Polymerisation (ROP), leading to a polybenzoxazine derivative. It could be considered as a virtuous loop.

The invention also relates to the use of a benzoxazine containing free aliphatic hydroxyl groups and monoester of formula (I) of the invention or as obtainable by the process (1) or of an ester containing benzoxazine monomer of formula (XX)

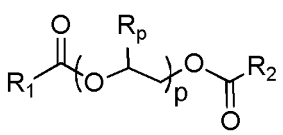

(XX)

wherein

$R_1$ is

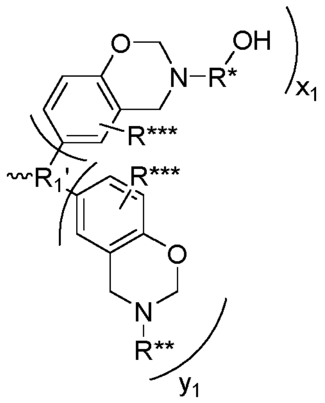 ; $R_2$ is 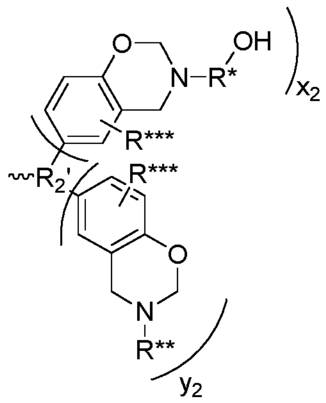 ;

and

$R_p$ is selected from the group consisting of H, a linear or branched $C_1$-$C_6$, preferably $C_1$-$C_4$, alkyl or alkoxy group, a linear or branched $C_2$-$C_6$, preferably $C_2$-$C_4$, alkenyl or alkylenoxy group, a substituted or unsubstituted linear or branched $C_2$-$C_6$, preferably $C_2$-$C_4$, alkynyl group, a linear or branched $C_1$-$C_6$, preferably $C_1$-$C_4$, alkyl or $C_2$-$C_6$, preferably $C_2$-$C_4$, alkenyl substituted or unsubstituted phenyl group and

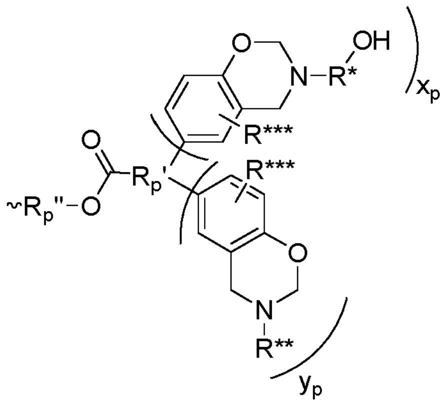

wherein

$R_1$ and $R_2$ of formula (XX) are identical or different;

$x_1$, $x_2$ and $x_p$, independently, are of from 0 to 1 and are not together 0;

$y_1$ = 1-$x_1$; $y_2$ = 1-$x_{2;}$ $y_p$ = 1-$x_p$;

p is 1-100;

$R_1$', $R_2$', and $R_p$', independently, are selected from the group consisting of a -C-linear or branched $C_1$-$C_6$ alkyl or alkoxy group, a -C-linear or branched $C_2$-$C_6$ alkenyl or alkylenoxy group, a -C-substituted or unsubstituted linear or branched $C_2$-$C_6$ alkynyl group, and a -C-linear or branched $C_1$-$C_6$ alkyl or $C_2$-$C_6$ alkenyl substituted or unsubstituted phenyl group;

$R_p$" is selected from the group consisting of a linear or branched $C_1$-$C_6$ alkyl or alkoxy group, a linear or branched $C_2$-$C_6$ alkenyl or alkylenoxy group, a substituted or unsubstituted linear or branched $C_2$-$C_6$ alkynyl group and a linear or branched $C_1$-$C_6$ alkyl or $C_2$-$C_6$ alkenyl substituted or unsubstituted phenyl group;

R*, R** and R*** are, independently, as defined above,

as a catalyst for benzoxazine polymerization.

Values of $x_1$, $x_2$ and xp, independently, are of from 0 to 1, and are not together 0, preferably of from 0,1 to 1, more preferentially of from 0,5 to 1, and $y_1$, $y_2$, and $y_p$ values are, respectively and independently, 1-$x_1$, 1-$x_2$ and 1-$x_p$. In some embodiments, $x_1$ and $x_2$ may not be together 0.

$x_1$, $x_2$, $x_p$ and $y_1$, $y_2$, $y_p$ represent the proportion between benzoxazine groups when prepared from an aminoalcohol and the other amine(s). In other words, $x_1$, $x_2$, $x_p$ and $y_1$, $y_2$, $y_p$ can be defined as

$$x_1 = \frac{n_{aminoalcohol\,(R1)}}{n^{total}_{amines\,(R1)}}$$

$$x_2 = \frac{n_{aminoalcohol\,(R2)}}{n^{total}_{amines\,(R2)}}$$

$$x_p = \frac{n_{aminoalcohol\,(Rp)}}{n^{total}_{amines\,(Rp)}}$$

$$y_1 = \frac{n_{amines\ (R1)}}{n^{total}_{amines\ (R1)}}$$

$$y_2 = \frac{n_{amines\ (R2)}}{n^{total}_{amines\ (R2)}}$$

$$y_p = \frac{n_{amines\,(Rp)}}{n^{total}_{amines\ (Rp)}}$$

wherein $n^{total}_{amine\,(R1)} = n_{amines(R1)} + n_{aminoalcohol(R1)}$, and $n_{aminoalcohol}(R1)$ being the number of aminoalcohol per $R_1$ group, $n_{amines}(R1)$represent the number of amines (excepting the number of aminoalcohol) per group $R_1$ and $n^{total}_{amine\,(R1)} = n_{amines(R1)} + n_{aminoalcohol(R1)}$ is the total number of amino groups per group $R_1$;

wherein $n^{total}_{amine\,(R2)} = n_{amines(R2)} + n_{aminoalcohol(R2)}$, and $n_{aminoalcohol(R2)}$ being the number of aminoalcohol per $R_2$ group, $n_{amines}(R2)$ represents the number of amines (excepting the number of aminoalcohol) per group $R_2$ and $n^{total}_{amine\,(R2)} = n_{amines(R2)} + n_{aminoalcohol(R2)}$ is the total number of amino groups per group $R_2$;

wherein $n^{total}_{amine\,(Rp)} = n_{amines(Rp)} + n_{aminoalcohol\,(Rp)}$, and $n_{aminoalcohol(Rp)}$ being the number of aminoalcohol per Rp group, $n_{amines(Rp)}$ represents the number of amines (excepting the number of aminoalcohol) per group $R_p$ and $n^{total}_{amine\,(Rp)} = n_{amines(Rp)} + n_{aminoalcohol\,(Rp)}$ is the total number of amino groups per group $R_p$.

The Applicant has surprisingly shown that such monoester-benzoxazine of formula (I) and/or such ester containing benzoxazine monomer of formula (XX) are the best suited for ROP of benzoxazine. Advantageously, the onset of the thermal polymerization of benzoxazine ring is lowered with the use of such catalyst(s).

Preferably, $R_1$', $R_2$', and $R_p$', independently, may be selected from the group consisting of a -C-a linear or branched $C_1$-$C_4$ alkyl or alkoxy group, a -C-linear or branched $C_2$-$C_4$ alkenyl or alkylenoxy group, a -C-substituted or unsubstituted linear or branched $C_2$-$C_4$ alkynyl group, and a -C-linear or branched $C_1$-$C_4$ alkyl or $C_2$-$C_4$ alkenyl substituted or unsubstituted phenyl group;

Preferably, $R_p$" may be selected from the group consisting of a linear or branched $C_1$-$C_4$ alkyl or alkoxy group, a linear or branched $C_2$-$C_4$ alkenyl or alkylenoxy group, a substituted or unsubstituted linear or branched $C_2$-$C_4$ alkynyl group and a linear or branched $C_1$-$C_4$ alkyl or $C_2$-$C_4$alkenyl substituted or unsubstituted phenyl group.

The invention also relates to a process (2) for synthesizing an ester-containing benzoxazine monomer of formula (XX) comprising the following steps consisting of:

a) reacting a phenolic acid derivative of formula (XXI), comprising at least one R*** group on the phenolic ring,

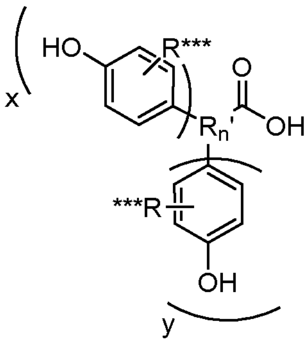

(XXI)

wherein x is of from 0 to 1, and y=1-x,
with a polyfunctional molecule or oligomer of formula (XXII)

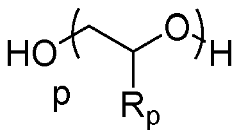

(XXII)

at a temperature of from 25°C to 200°C, during 1h-72h, in the presence of a catalyst of Bronsted acid type, resulting in a phenol terminated oligomer or molecule (compound ((XXIII)),

b) reacting the compound (XXIII) with a mixture of:

- an amino-alcohol of formula (V):

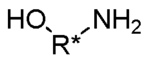

(V)

- a primary amine derivative of formula (VI), and

$R^{**}-NH_2$ (VI)

- paraformaldehyde of formula (VII)

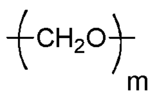

m=8-100

at a temperature range of from 80°C to 100°C, from 1h to 10h, under stirring, for obtaining the compound of formula (XX),
wherein $R_p$, R*, R**, R***, $R_n$' is $R_1$' and $R_2$', and p are, independently, as defined above, with the proviso that when at least one R*** of the phenolic acid derivative is in ortho-position with regard to -OH group, then R*** is H.

The process (2) for synthesizing an ester-containing benzoxazine monomer of formula (XX) uses polyfunctional oligomer or molecule of formula (XXII) while the process (1) to synthesize a monoester benzoxazine-containing free

aliphatic hydroxyl groups of formula (I) uses monofunctionnal oligomer or molecule of formula (III).

The polyfunctional molecule or oligomer compound of formula (XXII) is of importance for selecting the processing temperature of the benzoxazine polymer.

The compound of formula (XXII) may advantageously have 1-30, better 1-20, especially 1-10, p values, and may represent more preferably, when $R_p$=H, a polyethylene glycol (PEG) with a molecular weight (MW) in the range of from 4 MW of the $C_2H_4O$ unit to 50 MW of the $C_2H_4O$ unit, the MW of the $C_2H_4O$ unit being classically of about 44,05 g/Mol. It is preferable to use commercially available PEG, for example PEG 200 to PEG 2200, as being easily available.

In the compound of formula (XXII), when $R_p$=H, p values may be of from 1 (ethylene glycol) to 3 (triethylene glycol -TEG).

In some other embodiments, the compound of formula (XXII) may be glycerol ($R_p$=$CH_2OH$).

The preferred parameters, groups, stoechiometry and implementation conditions for step a) and step b) of the process (2) are the same as those described for step a) and step b) of the process (1), respectively.

The invention also relates to a process (3) for preparing a polybenzoxazine derivative comprising the step of polymerizing a composition comprising a benzoxazine containing free aliphatic hydroxyl groups monoester of formula (I) or as obtainable by the process (1) or an ester-containing benzoxazine monomer of formula (XX) or as obtainable by the process (2), or a mixture thereof, as a benzoxazine catalyst, and comprising of from 0 weight% to 99 weight% of a benzoxazine derivative, different from said benzoxazine catalyst, at temperatures within the range of from 100°C to 250°C for 1h to 24h, for obtaining polybenzoxazine derivatives.

In the context of the invention, "derivative" in "benzoxazine derivative" or "polybenzoxazine derivatives" means a compound bearing a benzoxazine moiety or issued from a compound bearing a benzoxazine moiety.

The monoester benzoxazine containing free aliphatic hydroxyl groups of formula (I) or the ester-containing benzoxazine monomer of formula (XX) as said benzoxazine catalyst may each react on itself, like the mixture thereof too, to produce the polybenzoxazine derivative, or react with a second benzoxazine derivative different of said benzoxazine catalyst.

The proportion of each of the benzoxazine containing free aliphatic hydroxyl groups and monoester of formula (I) or the ester-containing benzoxazine monomer of formula (XX) in the composition thereof is not limited. But it may advantageous that the proportion is within the range of 0,5 weight% to 95 weight%, better of from 1 to 50 wt%, most preferably of from 5 to 10 wt%.

According to the process for preparing the polybenzoxazine derivatives of the invention, using the compound (I) or the compound (XX), or mixture thereof, and optionally the benzoxazine derivative different from said benzoxazine catalyst, the polymerization step, which is a curing step, allows the benzoxazine ring to open and to react on itself or with another benzoxazine derivative to form a 3D network. Here, compounds of formula (I) or of formula (XX) as such can act on their self to produce the polybenzoxazine derivatives.

The polymerization duration is depending on the curing temperature and/or on the nature of the ester-containing benzoxazine monomer. The polymerization temperature is selected for a given monomer to be higher than the temperature needed to synthesize the monomer. Generally, the higher the polymerization temperature, the shorter the curing duration. For example, when the temperature of the polymerization is 250°C, the curing duration may be of at least 1h, and for a polymerization temperature of 100°C, the curing duration may be of no more than 24h. Preferably, the curing temperature may be of from 140°C to 200°C, more preferably of from 140°C to 180°C, the latter range providing curing duration of from 1,5h to 3h, preferably of from 1,5h to 2,5h. The polymerization may be performed by any known heating means, such as laser beam and infrared beam.

The process may also include a post-polymerization step consisting of a heating step which may preferably be carried out at higher temperature than that the polymerization heating step.

The benzoxazine derivative different from the catalyst may be the class of compounds selected from the group consisting of:

a 3,4-dihydro-2H-1,3-benzoxazine monomer having the formula

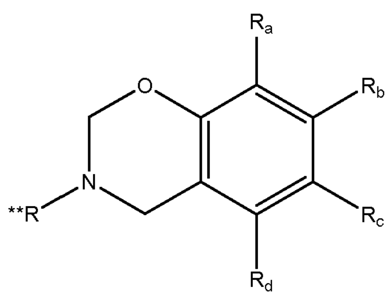

either wherein

$R_a = CH_3$

$R_b$ and $R_c$ together represent

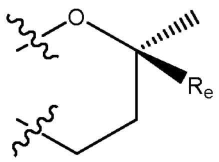

with

$R_e$ = 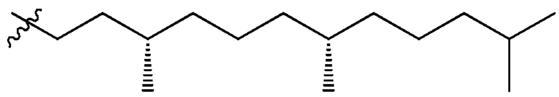

or

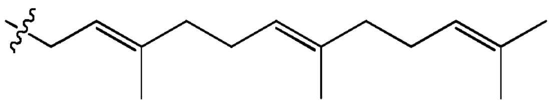,

and

$R_d$ = H, or $CH_3$,

or wherein

$R_d$ and $R_c$ together represent

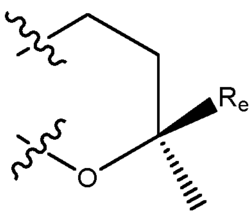

with

$R_e$ = 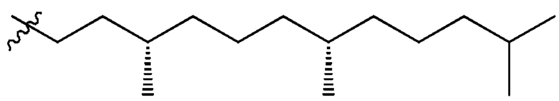

or

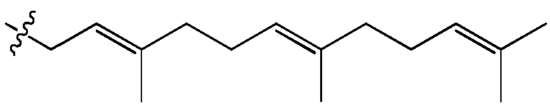,

and

$R_b = R_a = CH_3$,

or wherein
$R_c = R_d = H$,

$R_b$ = 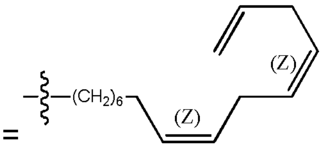, 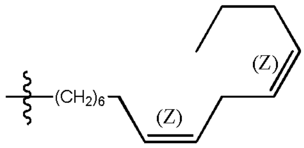, 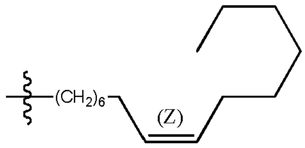

and/or

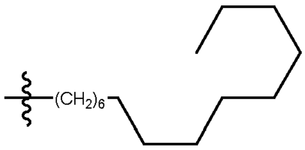

and $R_a$ = COOH,
or wherein
$R_a$ = H, $R_b$ = OH, $R_c$=H and

$R_d$ = 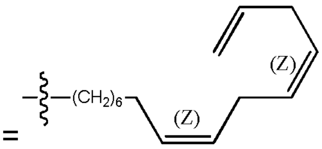, 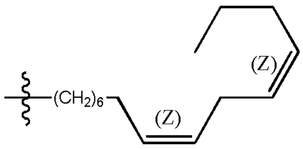, 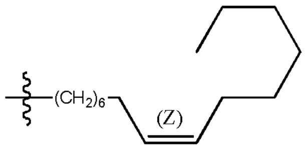

and/or

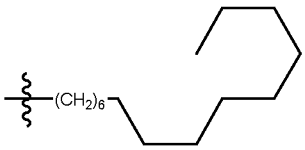

or wherein
$R_a$= H, and

$R_b$ = 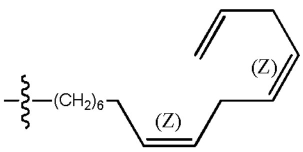, 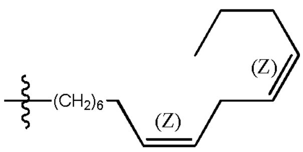, 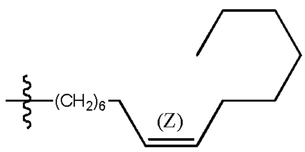

and/or

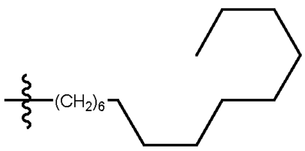

and $R_c$= H, $R_d$= OH,
or wherein
$R_a = CH_3$, $R_b$ = OH, $R_c$ = H

$R_d$ = 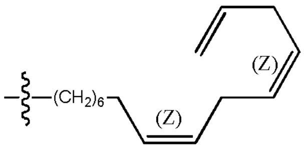 , 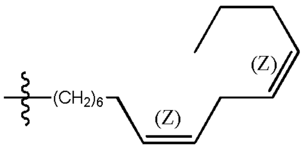 , 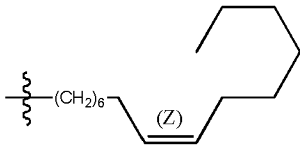

and/or

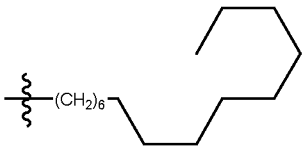

or wherein

$R_a$ = $R_c$ = $R_d$ = H and

$R_b$ = 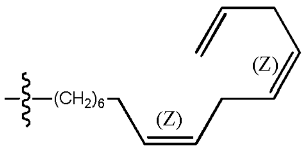 , 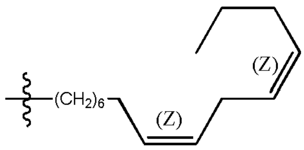 , 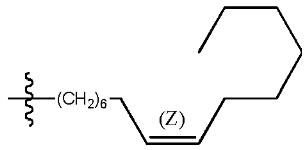

and/or

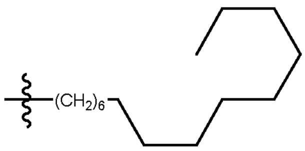

or wherein

$R_b$ = $R_d$ = H
$R_c$= $-CH_2-HC=CH_2$ and
$R_a$ = $OCH_3$;
or
$R_b$ = $R_d$ = H
$R_c$ = $-CH=HC-CH_3$ and
$R_a$ = $OCH_3$,
or a mixture thereof; R** being as defined above;

a compound of formula A, B or C, or mixture thereof, as follows Compound of formula A:

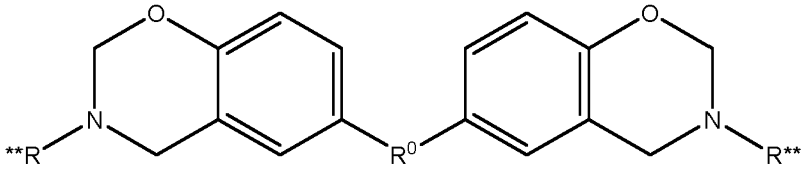

A

wherein

$R^0$ is a $-CH_2-$, $-C(CH_3)_2-$, $SO_2$, $-C(CF_3)_2-$, $-C(CH_3)(C_6H_5)-$, $-C(CH_3)(C_2H_5)-$, $-C(C_6H_5)_2-$ , $-CHCH_3-$, $-C_6H_{10}-$ or $-C(CH_3)CH_2CH_2COOH-$ group ;
R** is a $-CH_2CH_2OH$, vinyl, methyl, ethyl, propyl, isopropyl, butyl, hexyl, cyclohexyl, fluorene, phenylacetylene, phenyl propargyl ether, benzonitrile, furfuryl, phenylene or $-(CH_2)_{17}CH_3$ group;

compound of formula B

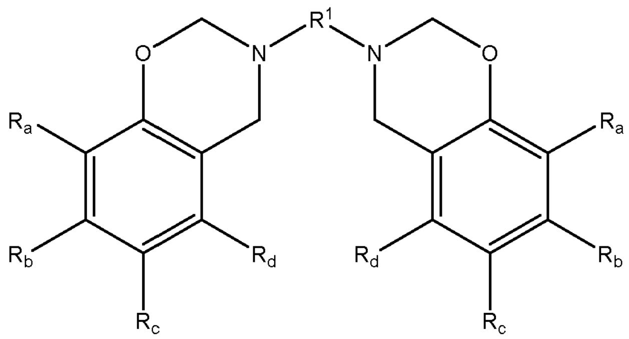

B

either wherein

$R_1$ is a $-(CH_2)_n-$ group, with n=1-10 or

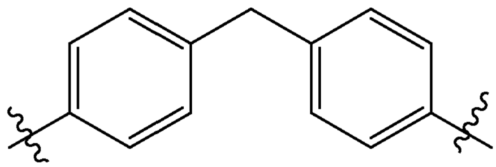

or

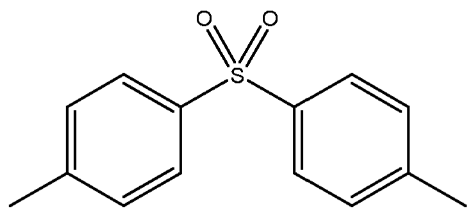

or

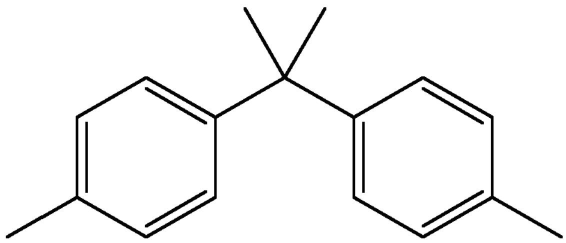

or

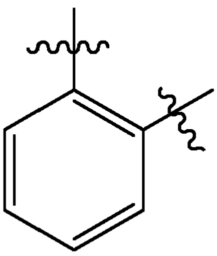

or

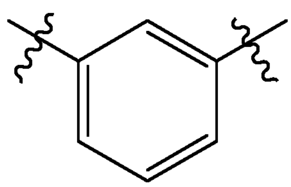

or

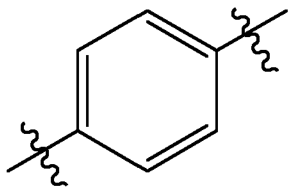

or

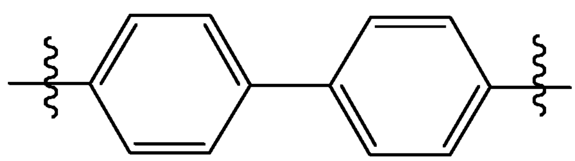

or

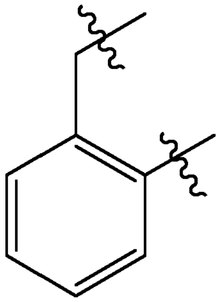

or

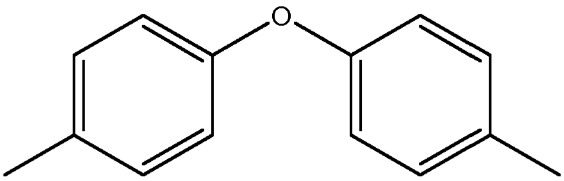

or

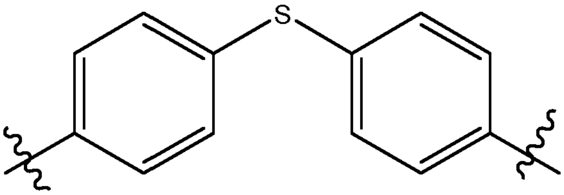

or

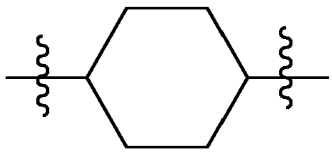

,

and wherein $R_a$, $R_b$, $R_c$, $R_d$ are selected from the group consisting of
$R_a = R_b = R_c = R_d = H$,
$R_a = OCH_3$, $R_b = R_d = H$, $R_c = CH_2CH{=}CH_2$,
$R_a = OCH_3$, $R_b = R_d = H$, $Re = CH{=}CHCH_3$,
$R_a = OCH_3$, $R_b = R_d = H$, $R_c = CHO$,
$R_a = OCH_3$, $R_b = R_c = R_d = H$,
$R_a = OCH_3$, $R_b = R_d = H$, $R_c = CHO$,
$R_a = OCH_3$, $R_b = R_d = H$, $R_c = CH_2CH_2COOH$,
$R_a = R_b = R_d = H$, $R_c = CH_2CH_2COOH$,
$R_a = R_b = R_d = H$, $R_c = CH{=}CHCOOH$, and
$R_a = OCH_3$, $R_b = R_d = H$, $R_c = CH{=}CHCOOH$,
or wherein
$R_c = R_d = H$,

$R_b$ =

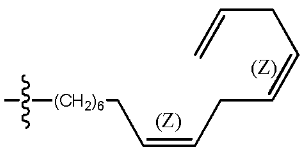

,

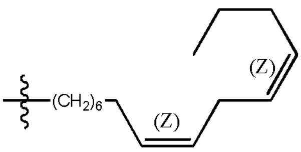

,

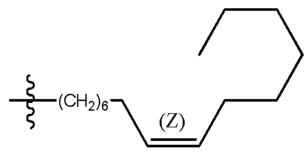

or

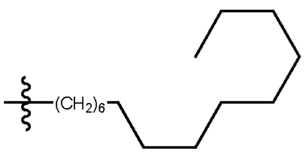

,

and $R_a = COOH$,
or wherein
$R_a = H$, $R_b = OH$, $R_c{=}H$ and

$R_d$ = 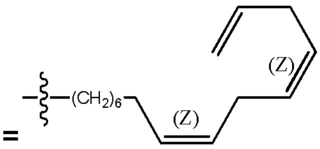, 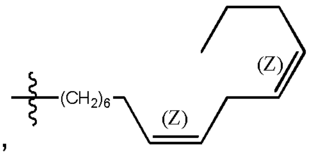, 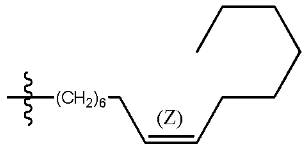

or

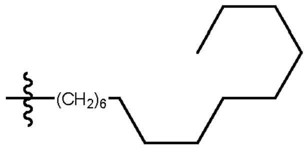,

or wherein
$R_a$ = H, and

$R_b$ = 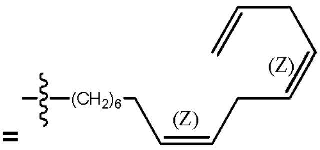, 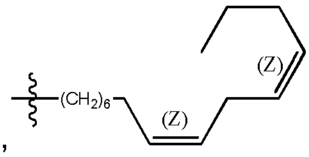, 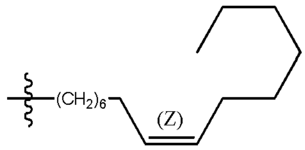

or

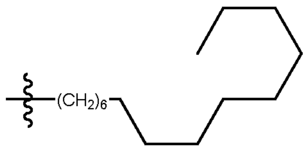,

and $R_c$= H, $R_d$= OH,
or wherein
$R_a$ = $CH_3$, $R_b$ = OH, $R_c$= H

$R_d$ = 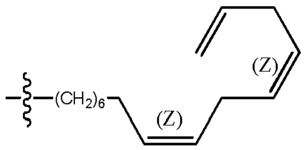, 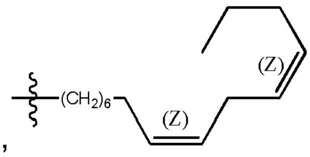, 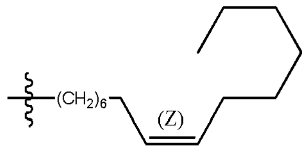

or

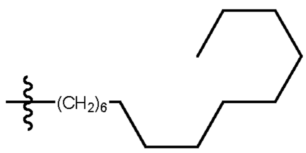,

or wherein
$R_a$ = $R_c$ = $R_d$ = H, and

$R_b$ = 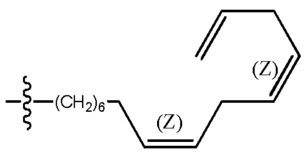, 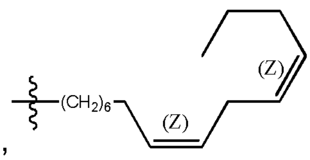, 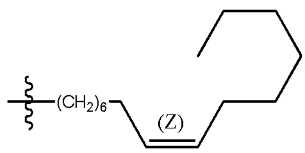

or

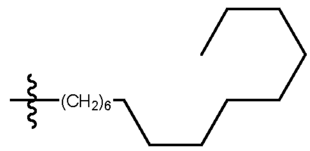

,

or wherein
$R_a = R_c = H$
$R_b = -CH_2HC{=}CH_2$, and $R_d = OCH_3$;
or
$R_a = R_c = H$
$R_b = -CH{=}HCCH_3$ and
$R_d = OCH_3$;
or $R_a = R_b = R_d = H$ and $R_c =$

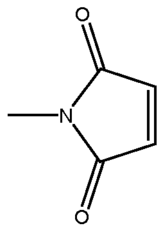

,

or $R_a = R_b = R_c = H$ and $R_d = CH_2CH{=}CH_2$
or $R_a = R_b = R_d = H$ and $R_c = CH_2CH{=}CH_2$
or $R_a = R_c = R_d = H$ and $R_b = CH_2CH{=}CH_2$;

a compound of formula C

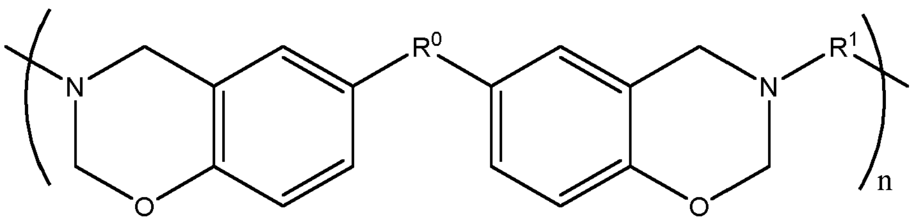

C

wherein $R^0$ is a $-CH_2-$, $-C(CH_3)_2-$, $SO_2$, $-C(CF_3)_2-$, $-C(CH_3)(C_6H_5)-$, $-C(CH_3)(C_2H_5)-$, $-C(C_6H_5)_2-$, $-CHCH_3-$, $-C_6H_{10}-$ or $-C(CH_3)CH_2CH_2COOH-$ group ;
and either within
n is an integer from 1 to 10;
$R_1$ is a $-(CH_2)_n-$ group, with n=1-10
or

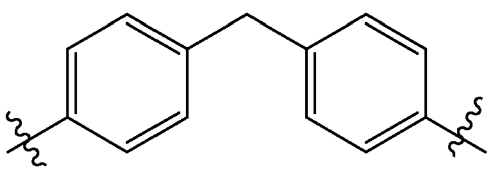

or

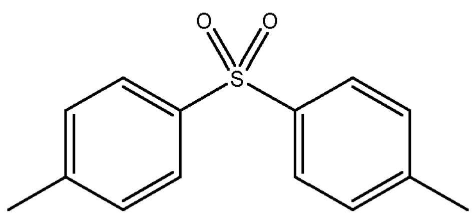

or

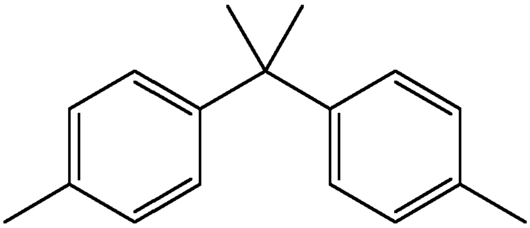
or
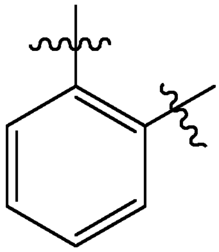

or

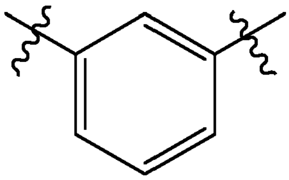
or
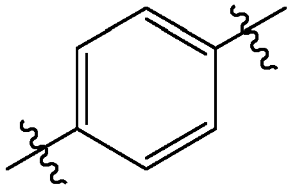

or

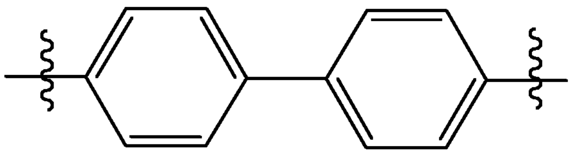
or
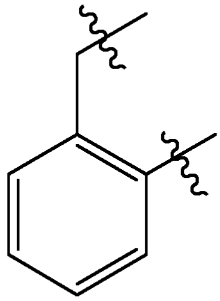

or

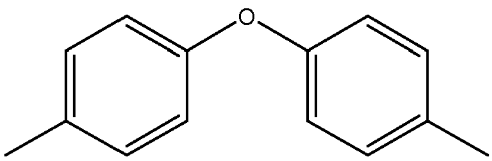
or
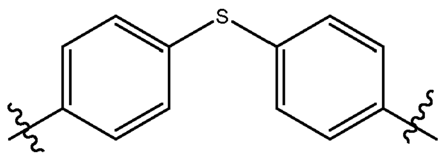

or

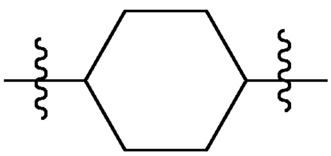
.

The benzoxazine derivatives: 3,4-dihydro-2H-1,3-benzoxazine monomer, compounds A-C, as described above, are known and synthesis thereof is detailed in WO2020/193293A1, as well as chemical and physical properties of polybenzoxazine derivatives thereof.

The invention also relates to a composition comprising:

- a benzoxazine containing free aliphatic hydroxyl groups and monoester of formula (I) or as obtainable by the process (1) or an ester-containing benzoxazine monomer of formula (XX) or as obtainable by the process (2), or a mixture thereof, as a benzoxazine catalyst, and

- of from 0 weight % to 99 weight% of a benzoxazine derivative, different of said benzoxazine catalyst and optionally at least one or more additional compounds of organic molecules types not containing benzoxazine moieties.

Preferably, the organic molecules types may be polymers not containing benzoxazine moieties, selected from the group consisting in epoxy resins, bismaleimide resins, phenolic resins or benzoxazine resins, polyurethanes, polyamides, polyolefins, polyesters and rubbers.

The composition may further comprise a material selected from the group consisting of fillers, fibers, pigments, dyes, and plasticizers, or mixture thereof.

Examples of such a material include at least one of carbon fibers, glass fibers, clays, carbon black, silica, carbon nanotubes, graphene, any known means for the thermal or the mechanical reinforcement of composites, or mixtures thereof.

The invention also concerns a use of the polybenzoxazine according to the invention as a reversible adhesive, sealant, coating or encapsulating systems for substrates selected from the group consisting of a metal, polymer, glass and ceramic material. Preferably, the metal and the polymer are as above defined.

Other features and advantages of the present invention will be readily understood from the following detailed description and drawings among them:

- Figure 1 shows a synthesis reaction of a monoester with a monofunctionnal phenolic acid for producing pentyl 3-(4-hydroxyphenyl)propanoate **(Pent-PA-mea);**

- Figure 2 shows a synthesis reaction of a monoester with a difunctionnal phenolic acid for producing pentyl 3-(4-hydroxyphenyl)propanoate (**Cyclo-DPA-mea**);

- Figure 3a) is a NMR spectrum of the valeric acid derivative benzoxazine monomer (PEG-DPA-mea) and Figure 3b) is a NMR spectrum of Pent-PA-mea;

- Figure 4a) displays the DSC curves of the PEG-PA-mea, PEG-DPA-mea, PEG-PA-fu, PEG-DPA-fu, Figure 4b) of the Pent-PA-mea and Pent-PA-fu;

- Figure 5 is an DSC of different mixtures of commercial benzoxazine (ARALDITE® MT 35710): a) monofunctional ester benzoxazine (Pent-PA-mea); b) a cardanol-based benzoxazine (Card-fu, without ester or aliphatic hydroxyl functions) ($10°C.min^{-1}$, $N_2$);

- Figure 6 is an example of a polybenzoxazine obtained through the use of Pent-PA-mea and Araldite® MT 35710;

- Figure 7 shows a synthesis reaction of a monoester with a monofunctional phenolic acid for producing methyl 3-(3-(2-hydroxyethyl)-3,4-dihydro-2Hbenzo[e][1,3]oxazin-6-yl) propanoate (Me-PA-mea);

- Figure 8 shows the NMR spectrum of Me-PA-mea ester-containing benzoxazine monomers;

- Figure 9 shows a synthesis reaction of a monoester with a difunctional phenolic acid for producing methyl 4,4-bis(3-(2-hydroxyethyl)-3,4-dihydro-2H-benzo[e][1,3]oxazin-6-yl)pentanoate (Me-DPA-mea);

- Figure 10 shows the NMR spectrum of Me-DPA-mea ester-containing benzoxazine monomers;

- Figure 11 shows a) the DSC and b) the isothermal rheology monitoring curves of Me-PA/DPA-mea/fu ester-containing benzoxazine monomers

All chemicals are commercially available and starting compounds, when applies, used as purchased.

<u>Example 1: Synthesis of benzoxazine containing free aliphatic hydroxyl groups and monoester (Pent-PA-mea) with pentanol, phloretic acid, mono-ethanol amine and paraformaldehyde</u>

The Pent-PA-mea monoester benzoxazine containing free aliphatic hydroxyl groups was synthesized in two stages (Fig. 1).

The first step, step a), corresponds to a Fischer esterification between pentanol (Pent) (1 eq.) and 3-(4-Hydroxyphenyl)propionic acid (phloretic acid, PA) (1 eq.) in presence of *p*-toluene sulfonic acid (*p*-TSA) introduced in catalytic amount (0,5 wt%). The reactants were put together in melt at 130°C and agitated by mechanical stirring for 24

hours, to provide pentyl 3-(4-hydroxyphenyl)propanoate (Pent-PA) (1 eq.).

The second step, step b), corresponds to a Mannich condensation of Pent-PA (1 eq.) with mono-ethanolamine (mea) (1 eq.) and paraformaldehyde (PFA) (2 eq,). All these reactants were agitated together by mechanical stirring and reacted in melt at 85°C for 2,5 hours to provide the Pent-PA-mea monoester benzoxazine containing free aliphatic hydroxyl groups, pentyl 3-(3-(2-hydroxyethyl)-3,4-dihydro-2H-benzo[e][1,3]oxazin-6-yl)propanoate.

Example 2: Synthesis of benzoxazine containing free aliphatic hydroxyl groups monoester (Cyclo-DPA-mea) from cyclohexanol, 4,4-Bis(4-hydroxyphenyl)valeric acid (DPA), mono-ethanol amine and paraformaldehyde

The Cyclo-DPA-mea benzoxazine monoester containing free aliphatic hydroxyl groups was synthesized in two stages (Fig. 2).

The first step, step a), corresponds to a Fischer esterification between cyclohexanol (1 eq.) and 4,4-Bis(4-hydroxyphenyl)valeric acid (DPA) (1 eq.) in presence of p-toluene sulfonic acid (*p*-TSA) introduced in catalytic amount (0,5 wt%). The reactants were put together in melt at 130°C and agitated by mechanical stirring for 24 hours, to provide cyclohexyl 4,4-bis(4-hydroxyphenyl)pentanoate (Cyclo-DPA) (1 eq.).

The second step, step b), corresponds to a Mannich condensation Cyclo-DPA (1 eq.), mono-ethanolamine (2 eq.) and paraformaldehyde (4 eq.). All these reactants were agitated together by mechanical stirring and reacted in melt at 85°C for 2,5 hours followed by 0,5 hours at 90°C to provide the Cyclo-DPA-mea monoester benzoxazine containing free aliphatic hydroxyl groups, cyclohexyl 4,4-bis(3-(2-hydroxyethyl)-3,4-dihydro-2H-benzo[e][1,3]oxazin-6-yl)pentanoate.

Example 3: Synthesis of an ester-containing benzoxazine monomer from 4,4-Bis(4-hydroxyphenyl)valeric acid (DPA) as a phenolic acid derivative

Ester-containing benzoxazine monomer was synthesized in two stages.

The first step, step a), corresponds to a Fischer esterification between polyethylene glycol (PEG) ($M_n$ = 400 $g.mol^{-1}$, p = 8-9, 1 eq., 10 g) and 4,4-Bis(4-hydroxyphenyl)valeric acid (DPA) (2 eq., 14,32 g) in presence of p-toluene sulfonic acid (p-TSA) introduced in catalytic amount (1 wt%). PEG, DPA and p-TSA were reacted together in melt at 130°C and agitated by mechanical stirring for 24 hours, to provide 4,4-Bis(4-hydroxyphenyl)valeric ester terminated polyethylene glycol (PEG-DPA).

The second step, step b), corresponds to a Mannich condensation between 4,4-Bis(4-hydroxyphenyl)valeric ester terminated polyethylene glycol (PEG-DPA) (1 eq, 22,8 g), mono-ethanol amine (mea) (4 eq., 5,95 g) and paraformaldehyde (PFA) (8 eq., 5,84 g). All these reactants were agitated together by mechanical stirring and reacted in melt at 85°C for 2,5 hours followed by 0,5 hours at 90°C to provide the ester-containing benzoxazine monomer named PEG-DPA-mea, polyethylene glycol terminated 4,4-bis(3-(2-hydroxyethyl)-3,4-dihydro-2H-benzo[e][1,3]oxazin-6-yl)pentanoate.

The Figure 3a) displays the NMR spectrum (AVANCE III HD Bruker spectrometer) of PEG-DPA-mea ester-containing benzoxazine monomer, and Figure 3b) displays the NMR spectrum of Pent-PA-mea.

Example 4

Figure 4a) displays the DSC curves of the PEG-PA-mea, PEG-DPA-mea, PEG-PA-fu, PEG-DPA-fu, "fu" designating furfurylamine, Figure 4b) of the Pent-PA-mea and Pent-PA-fu. Conditions: $10°C.min^{-1}$, $N_2$ atmosphere.

PEG-PA-mea: polyethylene glycol terminated 3-(3-(2-hydroxyethyl)-3,4-dihydro-2H-benzo[e][1,3]oxazin-6-yl)propanoate.

PEG-PA-fu: polyethylene glycol terminated 3-(3-(furan-2-ylmethyl)-3,4-dihydro-2H-benzo[e][1,3]oxazin-6-yl)propanoate.

PED-DPA-fu: polyethylene glycol terminated 4,4-bis(3-(furan-2-ylmethyl)-3,4-dihydro-2H-benzo[e][1,3]oxazin-6-yl)pentanoate.

PEG-PA-mea, PEG-PA-fu and PEG-DPA-fu ester-containing benzoxazine monomer are obtained as in Example 3, where furfurylamine is used instead of mono-ethanolamine and phloretic acid is used instead of diphenolic acid.

The Pent-PA-fu monomer is obtained as in Example 1, where furfurylamine is used instead of mono-ethanolamine.

Accordingly, Figure 4a and 4b are respectively displaying the DSC thermogram of polyfunctional and monofunctional ester containing benzoxazine. The abbreviations "mea" and "fu" correspond respectively to mono-ethanolamine (terminated by free hydroxyl groups) and furfurylamine (terminated by furan groups). For the polyfunctional

benzoxazine containing ester and furan groups (Figure 2a), PEG-PA-fu and PEG-DPA-fu, the DSC thermogram shows a first exothermic peak starting at a temperature of 145°C with a maximum located around 220°C. This peak is associated to the ring opening of the benzoxazine rings upon heating. The ring opening of the benzoxazine rings occured at much lower temperature in the case of polyfunctional benzoxazine containing ester and free alcohol groups (Figure 2a) - PEG-PA-mea and PEG-DPA-mea). The first exothermic peak starts at 105°C for a maximum located at 175°C.

Transesterification reactions between ester bonds and aliphatic hydroxyl groups promote the thermal ring opening polymerization of benzoxazine monomer. The second exothermic peak corresponds to the degradation the aliphatic ester, observed in both case (mea and fu). In the case of monofunctional benzoxazine (Figure 2b)), a similar trend is observed. The onset of thermal polymerization appeared at 110 and 140 °C respectively for benzoxazine containing free hydroxyl and furan groups (Pent-PA-mea and Pent-PA-fu).

Example 5

A commercially available benzoxazine monomer (ARALDITE® MT 35710 - Huntsmann) was mixed with different ratios of Pent-PA-mea from at 5, 10, 15 and 20% wt. All the mixtures were subjected to DSC studies to evaluate the catalytic activity of this monofunctional benzoxazine (Figure 5.a). A significant decrease of the onset of thermal polymerization from 189 to 149 °C is observed when the amount of catalyst in the mixtures increases from 0 to 20%. The maximum of the exothermic peak also decreases from 234 to 219 °C in the same condition. The enthalpy of polymerization is not strongly affected by the incorporation of the monofunctional benzoxazine monomer as catalyst (Pent-PA-mea). Therefore, this commercially available benzoxazine monomer (ARALDITE® MT 35710) was mixed with a cardanol-based benzoxazine which contains furfurylamine (terminated by furan groups) but no ester or aliphatic hydroxyl functions (Card-fu) (Figure 5.b). Card-fu is obtained by reacting cardanol (1 eq), furfurylamine (1 eq) and paraformaldéhyde (2 eq) together during 24h at 80°C without solvent. No catalytic activity is observed under these conditions, since the onset values are identical (ratio of Card-fu vs (ARALDITE® MT 35710).

Figure 6 is an obtained polybenzoxazine through the use of Pent-PA-mea and ARALDITE® MT 35710.The curing of the mixture of Pent-PA-mea and ARALDITE® MT 35710 was 160°C for 2 hours followed by 1h at 180°C.

Example 6: Synthesis of benzoxazine containing free aliphatic hydroxyl groups and monoester (Me-PA-mea) with methanol, phloretic acid, mono-ethanolamine and paraformaldehyde

The Me-PA-mea monoester benzoxazine containing free aliphatic hydroxyl groups was synthesized in two stages (Fig. 7). The first step, step a), corresponds to a Fischer esterification between methanol (Me) (1 eq.) and 3-(4-hydroxyphenyl) propionic acid (phloretic acid, PA) (1 eq.) in presence of concentrated sulfuric acid ($H_2SO_4$) introduced in catalytic amount (0,5 wt%). The reactants were put together in melt at 100°C under reflux and agitated by magnetic stirring for 4 hours, to provide methyl 3-(4-hydroxyphenyl)propanoate (Me-PA) (1 eq.).

The second step, step b), corresponds to a Mannich condensation of Me-PA (1 eq.) with mono-ethanolamine (mea) (1 eq.) and paraformaldehyde (PFA) (2 eq,). All these reactants were agitated together by mechanical stirring and reacted in melt at 85°C for 2,5 hours followed by 0,5 hours at 90°C to provide the Me-PA-mea monoester benzoxazine containing free aliphatic hydroxyl groups, methyl 3-(3-(2-hydroxyethyl)-3,4-dihydro-2Hbenzo[e][1,3]oxazin-6-yl)propanoate.

The Figure 8 is displaying the $^1$H NMR spectrum (AVANCE III HD Bruker spectrometer) of Me-PA-mea ester-containing benzoxazine monomers.

Example 7: Synthesis of benzoxazine containing free aliphatic hydroxyl groups monoester (Me-DPA-mea) from methanol, 4,4-Bis(4-hydroxyphenyl)valeric acid (DPA), mono-ethanolamine and paraformaldehyde

The Me-DPA-mea benzoxazine monoester containing free aliphatic hydroxyl groups was synthesized in two stages (Fig. 9). The first step, step a), corresponds to a Fischer esterification between methanol (1 eq.) and 4,4-Bis(4-hydroxyphenyl) valeric acid (DPA) (1 eq.) in presence of sulfuric acid ($H_2SO_4$) introduced in catalytic amount (0,5 wt%). The reactants were put together in melt at 100°C and agitated by magnetic stirring for 14 hours, to provide methyl 4,4-bis(4-hydroxyphenyl)pentanoate (Me-DPA) (1 eq.).

The second step, step b), corresponds to a Mannich condensation Me-DPA (1 eq.), mono-ethanolamine (2 eq.) and paraformaldehyde (4 eq.). All these reactants were agitated together by mechanical stirring and reacted in melt at 85°C for 2,5 hours followed by 0,5 hours at 90°C to provide the Me-DPA-mea monoester benzoxazine containing free aliphatic hydroxyl groups, methyl 4,4-bis(3-(2- hydroxyethyl)-3,4-dihydro-2H-benzo[e][1,3]oxazin-6-yl)pentanoate.

The Figure 10 is displaying the $^1$H NMR spectrum (AVANCE III HD Bruker spectrometer) of Me-DPA-mea ester-containing benzoxazine monomers.

Example 8

Figure 11.a and Figure 11.b are respectively displaying the DSC and isothermal rheology monitoring (160 °C) curves of the Me-PA-mea, Me-DPA-mea, Me-PA-fu, Me-DPA-fu (Me-PA/DPA-mea/fa) ester-containing benzoxazine monomers, "fu" designating furfurylamine. Conditions: 10°C.min$_{-1}$, $N_2$ atmosphere.

Me-PA-fu: methyl 3-(3-(furan-2-ylmethyl)-3,4-dihydro-2H-benzo[e][1,3]oxazin-6-yl)propanoate;
Me-DPA-fu: methyl 4,4-bis(3-(furan-2-ylmethyl)-3,4-dihydro-2H-benzo[e][1,3]oxazin-6-yl)pentanoate;

Me-PA-fu and Me-DPA-fu ester-containing benzoxazine monomer are obtained as in Example 6 and Example 7 respectively, where furfurylamine is used instead of mono-ethanolamine.

Accordingly, Figure 11.a is displaying the DSC thermogram of monofunctional and polyfunctional ester containing benzoxazine. The abbreviations "mea" and "fu" correspond respectively to mono-ethanolamine (terminated by free hydroxyl groups) and furfurylamine (terminated by furan groups). The DSC thermogram of furfurylamine containing benzoxazine monomers shows a first exothermic peak starting at a temperature of 150°C. This peak is associated to the ring opening of the benzoxazine rings upon heating. The ring opening of the benzoxazine rings occured at much lower temperature in the case of I benzoxazine monomers containing ester and free alcohol groups (Figure 11.a- Me-PA-mea and Me-DPA-mea). The first exothermic peak starts at 120°C for a maximum located around 200°C. Transesterification reactions between ester bonds and aliphatic hydroxyl groups promote the thermal ring opening polymerization of benzoxazine monomer. The second exothermic peak corresponds to the degradation the aliphatic ester, observed in both case (mea and fu).

The curing of the Me-PA/DPA-mea/fu ester-containing benzoxazine monomers was monitored by rheological measurement in Figure 11.b. The rheogram is performed under the following conditions: 1 Hz, with linear amplitude from 1 to 0,1%; 25 mm plates. The test is performed following a heating ramp from 80°C to 160 °C at 15°C/ min followed by an isothermal measurement at 160 °C. The complex viscosity is recorded as a function of time. The term "gelation time" is defined as the time when the complex viscosity of the soften monomer increases abruptly to transform into a gel. At 160°C, the gelation time is reached after 60, 450 and 1560s, respectively for Me-DPA-mea, Me-DPA-fu, and Me-PA-mea.

Figure 11.a) displays DSC curves and b) displays Isothermal rheology monitoring of Me-PA/DPA-mea/fu ester-containing benzoxazine monomers.

## Claims

1. A benzoxazine containing free aliphatic hydroxyl groups and monoester of formula (I)

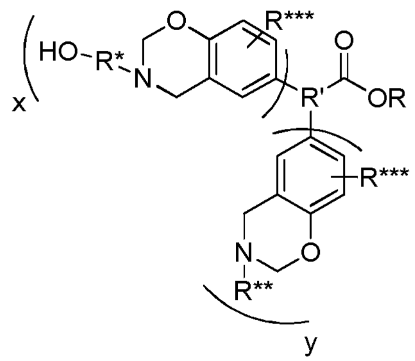

wherein

R is selected from the group consisting of a linear or branched $C_1$-$C_{12}$ alkyl or alkoxy group, a linear or branched $C_2$-$C_6$ alkenyl or alkylenoxy group, a substituted or unsubstituted linear or branched $C_2$-$C_6$ alkynyl group, a cyclo($C_3$-$C_6$ alkyl) group, a heteocyclo($C_3$-$C_6$ alkyl), a linear or branched $C_1$-$C_6$ alkyl or $C_2$-$C_6$ alkenyl substituted or unsubstituted phenyl group and a $(CH_2)_{n3}$-phenyl group, wherein n3 is an integer from 1 to 10;
R' is selected from the group consisting of at least one of -CH, a C-$(CH_2)_{n3}$-$CH_3$ group, a C-$(CH_2)_{n3}$-CH-$(CH_3)_2$ group, a C-$(CH_2)_{n3}$-$(CHZ)_{n4}$-$(CH_3)_2$ group, a C-$(CH_2)_{n3}$-$(CHZ)_{n4}$-$(CH_2)_{n3}$-$CH_3$ group, a C-$(CHZ)_{n4}$-$(CH_2)_{n3}$-$CH_3$ group, a C-$(CHZ)_{n4}$-$[(CH_2)_{n3}$-$CH_3]_2$ group, a C-substituted or unsubstituted $C_2$-$C_6$ linear or branched alkenyl

group, a linear or branched $C_1$-$C_6$ alkyl substituted or unsubstituted phenyl or phenyl including at least one hetero atom selected from N, O and S, a C-$(CH_2)_{n3}$-$C_1$-$C_6$ linear or branched alkyl substituted or unsubstituted phenyl or phenyl including at least one heteroatom selected from N, O and S, a C-$(CH_2)_{n3}$-$C_1$-$C_6$ linear or branched alkyl substituted or unsubstituted phenyl or phenyl including at least one heteroatom selected from N, O and S-$(CH_2)_{n3}$-$CH_3$, a C-$(CH_2)_{n3}$-$C_1$-$C_6$ linear or branched alkyl substituted or unsubstituted phenyl or phenyl including at least one heteroatom selected from N, O and S-$(CH_2)_{n3}$-CH-$(CH_3)_2$, a C-$(CH_2)_{n3}$-$C_1$-$C_6$ linear or branched alkyl substituted or unsubstituted phenyl or phenyl including at least one heteroatom selected from N, O and S-$(CH_2)_{n3}$-$(CHZ)_{n4}$-$(CH_3)_2$ group, a C-$(CH_2)_{n3}$-$C_1$-$C_6$ linear or branched alkyl substituted or unsubstituted phenyl or phenyl including at least one heteroatom selected from N, O and S-$(CHZ)_{n4}$-$(CH_2)_{n3}$-$CH_3$ group and a C-$(CH_2)_{n3}$-$(CHZ)_{n4}$-$C_1$-$C_6$ linear or branched alkyl substituted or unsubstituted phenyl or phenyl including at least one heteroatom selected from N, O and S-$(CH_2)_{n3}$-$CH_3$ group, wherein n3 and n4, independently, are an integer from 1 to 10 and Z is selected from the group consisting in a linear or branched $C_1$-$C_6$ alkyl or alkoxy group, linear or branched $C_2$-$C_6$ alkenyl or alkylenoxy group and a linear or branched $C_1$-$C_6$ alkyl or $C_2$-$C_6$ alkenyl substituted or unsubstituted phenyl group, and at least one O atom is present or not between two adjacent C, or R' is omitted..

R* is selected from the group consisting of a linear or branched $C_1$-$C_{20}$ alkyl or alkoxy group, a cyclo($C_3$-$C_6$ alkyl) group, a heteocyclo($C_3$-$C_6$ alkyl) group, wherein the hetero atom is selected from N, S, and O, linear or branched $C_2$-$C_6$ alkenyl or alkylenoxy group, substituted or unsubstituted linear or branched $C_2$-$C_6$ alkynyl group, a linear or branched $C_1$-$C_6$ alkyl or $C_2$-$C_6$ alkenyl substituted or unsubstituted phenyl group, a $(CH_2)_{n3}$-phenyl group and -$(CH_2)_{n3}$-O-$(CH_2)_{n4}$, wherein n3 and n4, independently, are an integer from 1 to 10;

R** is the same as R* and further includes a member selected from a O-, N- or S-$(CH_2)_{n3}$-CH-$(CH_3)_2$ group, a O-, N- or S-$(CH_2)_{n3}$-$(CHZ)_{n4}$-$(CH_3)_2$ group, a O-, N- or S-$(CH_2)_{n3}$-$(CHZ)_{n4}$-$(CH_2)_{n3}$-$CH_3$ group, a O-, N- or S-$(CHZ)_{n4}$-$(CH_2)_{n3}$-$CH_3$ group, a O-, N- or S-$(CHZ)_{n4}$-$[(CH_2)_{n3}$-$CH_3]_2$ group and a O-substituted or unsubstituted $C_2$-$C_6$ linear or branched alkynyl group, Z being as defined for R', a -$(CH_2)_{n3}$-C≡N group, a polycyclic aromatic or heteroaromatic hydrocarbon, such as naphthalene, anthracene, fluorene, phenanthrene, optionally substituted by a linear or branched $C_1$-$C_6$ alkyl or alkoxy group, a cyclo($C_3$-$C_6$ alkyl) group, a heterocyclo($C_3$-$C_6$alkyl) group, a linear or branched $C_2$-$C_6$ alkenyl or alkylenoxy group, or by a substituted or unsubstituted linear or branched $C_2$-$C_6$ alkynyl group, wherein n3 and n4, independently, are an integer from 1 to 10;

R*** is selected from the group consisting in H, OH and a O-linear or branched $C_1$-$C_6$ alkyl group, and further includes a linear or branched $C_1$-$C_{15}$ alkyl group or a $C_2$-$C_{15}$ alkenyl group or

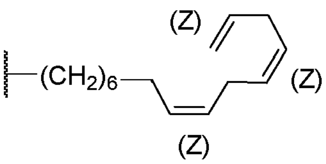 , 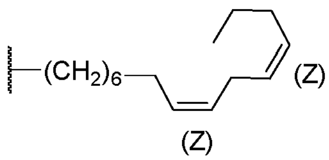 , 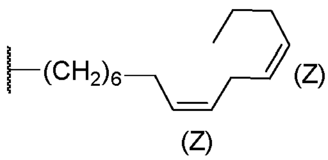

or

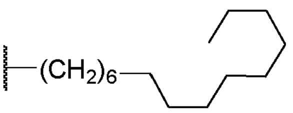 ;

and x value is of from 0 to 1 and y value is 1-x.

**2.** The benzoxazine according to claim 1, wherein

R is selected from the group consisting of a linear or branched $C_1$-$C_4$ alkyl or alkoxy group, a linear or branched $C_2$-$C_4$ alkenyl or alkylenoxy group, an unsubstituted linear or branched $C_2$-$C_4$ alkynyl group, an unsubstituted phenyl group and a $(CH_2)_{n3}$-phenyl group, wherein n3 is an integer from 1 to 6;

R' is selected from the group consisting of at least one of -CH, a C-$(CH_2)_{n3}$-$CH_3$ group, a C-$(CH_2)_{n3}$-CH-$(CH_3)_2$ group, a C-$(CH_2)_{n3}$-$(CHZ)_{n4}$-$(CH_3)_2$ group, C-$(CH_2)_{n3}$-$(CHZ)_{n4}$-$(CH_2)_{n3}$-$CH_3$ group, C-$(CHZ)_{n4}$-$(CH_2)_{n3}$-$CH_3$ group, a C-$(CHZ)_{n4}$-$[(CH_2)_{n3}$-$CH_3]_2$ group, a C-substituted or unsubstituted $C_2$-$C_4$ linear or branched alkenyl group, an unsubstituted phenyl or phenyl including at least one heteroatom selected from N, O and S, a C-$(CH_2)_{n3}$-unsubstituted phenyl or phenyl including at least one heteroatom selected from N, O and S, a C-$(CH_2)_{n3}$-unsubstituted phenyl or phenyl including at least one heteroatom selected from N, O and

S-$(CH_2)_{n3}$-$CH_3$, a C-$(CH_2)_{n3}$-unsubstituted phenyl or phenyl including at least one heteroatom selected from N, O and S-$(CH_2)_{n3}$-CH-$(CH_3)_2$, a C-$(CH_2)_{n3}$-unsubstituted phenyl or phenyl including at least one heteroatom selected from N, O and S-$(CH_2)_{n3}$-$(CHZ)_{n4}$-$(CH_3)_2$ group, a C-$(CH_2)_{n3}$-unsubstituted phenyl or phenyl including at least one heteroatom selected from N, O and S-$(CHZ)_{n4}$-$(CH_2)_{n3}$-$CH_3$ group and a C-$(CH_2)_{n3}$-$(CHZ)_{n4}$-unsubstituted phenyl or phenyl including at least one heteroatom selected from N, O and S-$(CH_2)_{n3}$-$CH_3$ group, wherein n3 and n4, independently, are an integer from 1 to 6 and Z is selected from the group consisting in a linear or branched $C_1$-$C_4$ alkyl or alkoxy group, linear or branched $C_2$-$C_4$ alkenyl or alkylenoxy group and an unsubstituted phenyl group, and at least one O atom is present or not between two adjacent C;

R* is selected from the group consisting of a linear or branched $C_1$-$C_6$ alkyl or alkoxy group, a linear or branched $C_2$-$C_4$ alkenyl or alkylenoxy group, an unsubstituted linear or branched $C_2$-$C_4$ alkynyl group, an unsubstituted phenyl group, a $(CH_2)_{n3}$-phenyl group and -$(CH_2)_{n3}$-O-$(CH_2)_{n4}$, wherein n3 and n4, independently, are an integer from 1 to 6;

R** is the same as R* and further includes a member selected from a O-, N- or S-$(CH_2)_{n3}$-CH-$(CH_3)_2$ group, a O-, N- or S-$(CH_2)_{n3}$-$(CHZ)_{n4}$-$(CH_3)_2$ group, a O-, N- or S-$(CH_2)_{n3}$-$(CHZ)_{n4}$-$(CH_2)_{n3}$-$CH_3$ group, a O-, N- or S-$(CHZ)_{n4}$-$(CH_2)_{n3}$-$CH_3$ group, a O-, N- or S-$(CHZ)_{n4}$-$[(CH_2)_{n3}$-$CH_3]_2$ group and O-substituted or unsubstituted $C_2$-$C_4$ linear or branched alkynyl group, Z being as defined above, a -$(CH_2)_{n3}$-C≡N group, a cyclo($C_3$-$C_4$ alkyl), a heteocyclo($C_3$-$C_4$alkyl) group, a polycyclic aromatic or heteroaromatic hydrocarbon (PAH), wherein the hetero atom is selection from N, S, and O, such as naphthalene, anthracene, fluorene, furane, optionally substituted by a linear or branched $C_1$-$C_4$ alkyl or alkoxy group, a linear or branched $C_2$-$C_4$ alkenyl or alkylenoxy group, or by a substituted or unsubstituted linear or branched $C_2$-$C_4$ alkynyl group, wherein n3 and n4, independently, are an integer from 1 to 6;

R*** is selected from the group consisting in H, OH and a O-linear or branched $C_1$-$C_4$ alkyl group, and further includes a linear or branched $C_1$-$C_{10}$ alkyl group or $C_2$-$C_{10}$ alkenyl group or

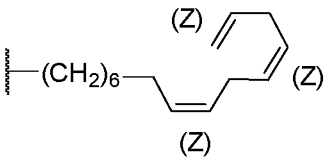 , 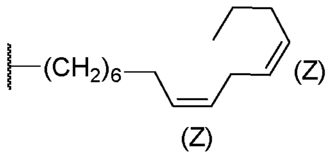 , 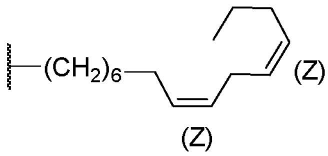

or

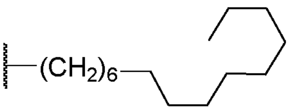 .

3. The benzoxazine according to claim 1, wherein

R is selected from the group consisting of groups -$CH_3$, -$(CH_2)_{n3}$-$CH_3$, -$(CH_2)_{n3}$-CH-$[(CH_2)_{n3}$-$CH_3]_2$, -$C(CH_3)_3$, -$(CH_2)_{n3}$-$(C_6H_5)$, -$(CH_2)_{n3}$-CH=$CH_2$ and -$(CH_2)_{n3}$-C≡CH, wherein n3 is an integer from 1 to 5;

R' is selected form the group consisting of groups -CH, -C-$(CH_2)_2$-$C(CH_3)$, -C-$CH(CH_2CH_3)$, -$C(CH_2CH_2CH_3)$, -C-$CH_2(CH_2)_3CH_3$, -C-$CH_2(CH_2)_4CH_3$, -$C(C_6H_5)$, - $C(CH_3)CH_2$, $C(CH_3)CH_2CH_2$ and -$C(C_6H_5)CH_2$-$CH_3$;

R* is selected from the group consisting of groups -$CH_3$, -$(CH_2)_{n3}$-$CH_3$, -$(CH_2)_{n3}$-CH-$[(CH_2)_{n4}$-$CH_3]_2$, -$C(CH_3)_3$, $(CH_2)_{n3}$-$(C_6H_5)$, -$(CH_2)_{n3}$-CH=$CH_2$, -$(CH_2)_{n3}$-C≡CH, - $(CH_2)_{n3}$-O-$(CH_2)_{n4}$ wherein n3 and n4 independently are integer from 1 to 4, phenyl, and -$(CH_2)_3$-phenyl;

R** is selected from the group consisting of groups $CH_3$, -$(CH_2)_{n3}$-$CH_3$, -$(CH_2)_{n3}$-CH-$[(CH_2)_{n4}$-$CH_3]_2$, -$C(CH_3)_3$, $(CH_2)_{n3}$-$(C_6H_5)$, -$(CH_2)_{n3}$-CH=$CH_2$, -$(CH_2)_{n3}$-C≡CH, O-$(CH_2)_{n3}$-C≡CH, O-$(CH_2)_{n3}$-C≡N, $(CH_2)_{n3}$-C≡N, and -$(CH_2)_{n3}$-substituted or unsubstituted furan, phenyl, and wherein n3 and n4 independently are integer from 1 to 4;

R*** is selected from the group consisting in H, OH and O-linear or branched $C_1$-$C_3$ alkyl group, and further includes linear or branched $C_1$-$C_6$ alkyl group or $C_2$-$C_6$ alkenyl group or

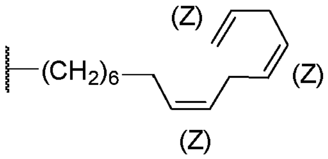,
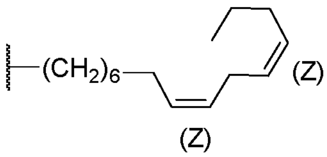,
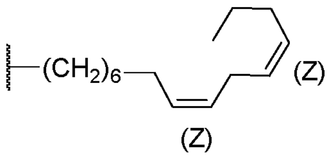
or

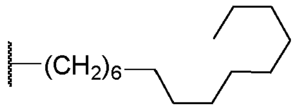,

H being the most preferred.

**4.** Process (1) for producing a benzoxazine containing free aliphatic hydroxyl groups and monoester of formula (I) comprising the following steps of:

a) reaction of a phenolic acid derivative of formula (II), comprising at least one R*** group,

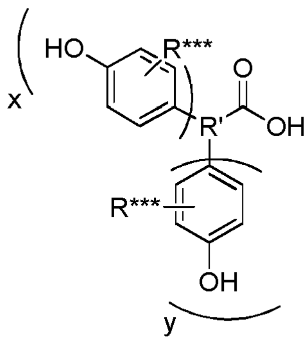

(II)

with a monofunctional oligomer or molecule of formula (III)

R-OH (III)

at a temperature of from 80°C to 200°C, during 12h-48h, in a presence of a Bronsted type acid catalyst, resulting in a phenol terminated oligomer or molecule of formula (IV)

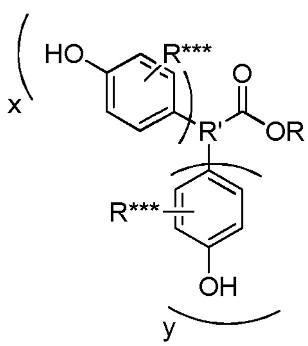

(IV)

and
b) reaction of the phenol terminated oligomer or molecule of formula (IV) with a mixture of

- an amino-alcohol of formula (V):

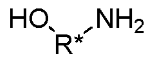

- a primary amine derivative of formula (VI)

R**-$NH_2$ (VI),

and
- paraformaldehyde of formula (VII)

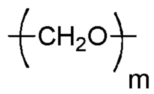

m=8-100

at a temperature range of from 80°C to 100°C, from 1h to 48h, under stirring, wherein R, R', R*, R**, R***, x and y are, independently, as defined in any of claims 1-3, with the proviso that when the at least one R*** of the phenolic acid derivative is in *ortho* position with regard to -OH group, then R*** is H.

**5.** Process according to claim 4, wherein the phenolic acid derivative (formula (II)) includes from 1 to 4 R*** group(s), related to the substitution of the phenolic ring.

**6.** Process according to claim 4 or 5, wherein the phenolic acid derivative is selected from the group consisting of mono-, di-, tri-hydroxybenzoic acid derivatives, anacardic acid derivatives, hydroxycinnamic acid derivatives, aliphatic X-hydroxyphenyl acid derivatives, wherein X is 2-4, and aliphatic diphenolic acid derivatives, or mixtures thereof.

**7.** Process according to any of claims 4 to 6, wherein the respective stoichiometry of starting reactants on step a), phenolic acid derivative:monofunctional molecule or oligomer is 1,0-3,0 eq.:1,0 eq, resulting in an 1,0 eq. of phenol terminated oligomer or molecule.

**8.** Process according to any of claims 4 to 7, wherein the primary amine derivatives bear R** having the definition of R* and are further selected from the group consisting in allylamine, methylamine, ethylamine, propylamine, butylamine, isopropylamine, hexylamine, cyclohexylamine, stearylamine, 2-aminofluorene, aminophenyl acetylene, propargyl ether aniline, 4-aminobenzonitrile, furfurylamine and aniline, or mixtures thereof, and wherein the amino-alcohol of formula (V) is selected from the group consisting of 2-aminoethanol, 2-amino-2-methylpropanol, 5-aminopentan-1-ol, heptaminol and diglycolamine, or mixtures thereof.

**9.** Process according to any of claims 4 to 8, wherein the respective stoichiometry of starting reactants on step b), phenol terminated oligomer or molecule:amino-alcohol:primary amine derivative:paraformaldehyde is 1,0 eq.:x(1,0 eq-18,0 eq): y(1,0 eq-18,0 eq):2,0-36,0 eq, resulting in an 1,0 eq. of the benzoxazine containing free aliphatic hydroxyl groups and monoester, wherein x = 0-1, and y =1-x.

**10.** Benzoxazine ring-opening polymerisation (ROP) catalyst comprising a benzoxazine containing free aliphatic hydroxyl groups and monoester of formula (I) according to any of claims 1 to 3 or as obtainable according to the process (1) of any of claims 4-9.

**11.** A use of a benzoxazine containing free aliphatic hydroxyl groups and monoester of formula (I) according to any of claims 1 to 3 or as obtainable according to the process (1) of any of claims 4-9 or of an ester containing benzoxazine monomer of formula (XX)

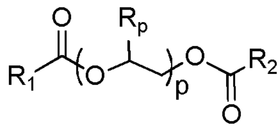

(XX)

wherein

$R_1$ is

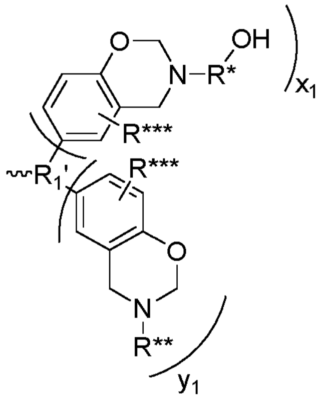

; $R_2$ is

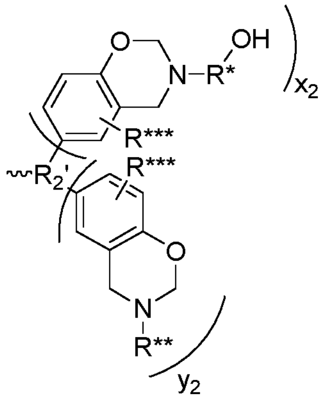

;

and

$R_p$ is selected from the group consisting of H, a linear or branched $C_1$-$C_6$, preferably $C_1$-$C_4$, alkyl or alkoxy group, a linear or branched $C_2$-$C_6$, preferably $C_2$-$C_4$, alkenyl or alkylenoxy group, a substituted or unsubstituted linear or branched $C_2$-$C_6$, preferably $C_2$-$C_4$, alkynyl group, a linear or branched $C_1$-$C_6$, preferably $C_1$-$C_4$, alkyl or $C_2$-$C_6$, preferably $C_2$-$C_4$, alkenyl substituted or unsubstituted phenyl group and

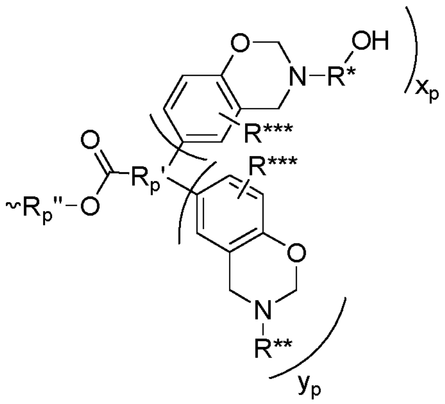

wherein

$R_1$ and $R_2$ of formula XX are identical or different;

$x_1$, $x_2$ and $x_p$, independently, are of from 0 to 1 and are not together 0;

$y_1$ = 1-$x_1$; $y_2$ = 1-$x_2$; $y_p$ = 1-$x_p$;

p is 1-100;

$R_1$', $R_2$', and $R_p$', independently, are selected from the group consisting of a -C-linear or branched $C_1$-$C_6$ alkyl or alkoxy group, a -C-linear or branched $C_2$-$C_6$ alkenyl or alkylenoxy group, a -C-substituted or unsubstituted linear or branched $C_2$-$C_6$ alkynyl group, and a -C-linear or branched $C_1$-$C_6$ alkyl or $C_2$-$C_6$ alkenyl substituted or unsubstituted phenyl group;

$R_p$'' is selected from the group consisting of a linear or branched $C_1$-$C_6$ alkyl or alkoxy group, a linear or branched $C_2$-$C_6$ alkenyl or alkylenoxy group, a substituted or unsubstituted linear or branched $C_2$-$C_6$ alkynyl group and a linear or branched $C_1$-$C_6$ alkyl or $C_2$-$C_6$ alkenyl substituted or unsubstituted phenyl group;

R*, R** and R*** are, independently, as defined in any of claims 1-3,
wherein
$x_1$, $x_2$, $x_p$ and $y_1$, $y_2$, $y_p$ are defined as:

$$x_1 = \frac{n_{aminoalcohol(R1)}}{n^{total}_{amines(R1)}}$$

$$x_2 = \frac{n_{aminoalcohol(R2)}}{n^{total}_{amines(R2)}}$$

$$x_p = \frac{n_{aminoalcohol(Rp)}}{n^{total}_{amines(Rp)}}$$

$$y_1 = \frac{n_{amines\ (R1)}}{n^{total}_{amines\ (R1)}}$$

$$y_2 = \frac{n_{amines\ (R2)}}{n^{total}_{amines\ (R2)}}$$

$$y_p = \frac{n_{amines\ (Rp)}}{n^{total}_{amines\ (Rp)}}$$

wherein $n^{total}_{amine(R1)} = n_{amines(R1)} + n_{aminoalcohol(R1)}$, and $n_{aminoalcohol(R1)}$ being the number of aminoalcohol per $R_1$ group, $n_{amines(R1)}$ represent the number of amines (excepting the number of aminoalcohol) per group $R_1$ and $n^{total}_{amine(R1)} = n_{amines(R1)} + n_{aminoalcohol(R1)}$ is the total number of amino groups per group $R_1$;
wherein $n^{total}_{amine(R2)} = n_{amines(R2)} + n_{aminoalcohol(R2)}$, and $n_{aminoalcohol(R2)}$ being the number of aminoalcohol per $R_2$ group, $n_{amines(R2)}$ represents the number of amines (excepting the number of aminoalcohol) per group $R_2$ and $n^{total}_{amine(R2)} = n_{amines(R2)} + n_{aminoalcohol(R2)}$ is the total number of amino groups per group $R_2$;
wherein $n^{total}_{amine(Rp)} = n_{amines(Rp)} + n_{aminoalcohol(Rp)}$, and $n_{aminoalcohol(Rp)}$ being the number of aminoalcohol per Rp group, $n_{amines(Rp)}$ represents the number of amines (excepting the number of aminoalcohol) per group $R_p$ and $n^{total}_{amine(Rp)} = n_{amines(Rp)} + n_{aminoalcohol(Rp)}$ is the total number of amino groups per group $R_p$, as a catalyst for benzoxazine polymerization.

**12.** A process (2) for synthesizing an ester-containing benzoxazine monomer of formula (XX) comprising the following steps consisting of:

a) reacting a phenolic acid derivative of formula (XXI), comprising at least one R*** group on the phenolic ring,

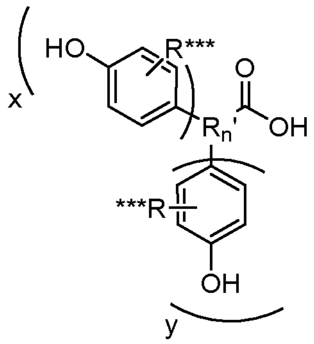

(XXI)

wherein x is of from 0 to 1, and y=1-x,
with a polyfunctional molecule or oligomer of formula (XXII)

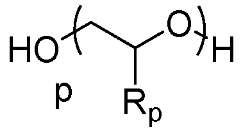

(XXII)

at a temperature of from 25°C to 200°C, during 1h-72h, in the presence of a catalyst of Bronsted acid type, resulting in a phenol terminated oligomer or molecule (compound (XXIII)) and
b) reacting the compound (XXIII) with a mixture of:

- an amino-alcohol of formula (V):

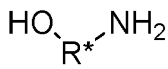

(V)

- a primary amine derivative of formula (VI), and

$R^{**}-NH_2$ (VI)

- paraformaldehyde of formula (VII)

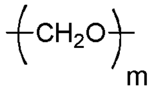

m=8-100

at a temperature range of from 80°C to 100°C, from 1h to 10h, under stirring, for obtaining the compound of formula (XX),
wherein $R_p$, R*, R**, R***, $R_n$' is $R_1$' and $R_2$', and p are, independently, as defined in any of claims 1-3 and 11, with the proviso that when the at least one R*** of the phenolic acid derivative is in *ortho* position with regard to -OH group, then R*** is H.

**13.** A process (3) for preparing a polybenzoxazine derivative comprising the step of polymerizing a composition comprising a benzoxazine containing free aliphatic hydroxyl groups and monoester of formula (I) or as obtainable by the process (1) according to any of claims 4-9 or an ester-containing benzoxazine monomer of formula (XX) according to claim 3 or as obtainable by the process (2) of claim 12, or a mixture thereof, as a benzoxazine catalyst, and comprising of from 0 weight% to 99 weight% of a benzoxazine derivative, different of said benzoxazine catalyst, at temperatures within the range of from 100°C to 250°C for 1h to 24h, for obtaining polybenzoxazine derivatives.

**14.** The process (3) according to claim 13, wherein the proportion of each of the benzoxazine containing free aliphatic hydroxyl groups and monoester of formula (I) or the ester-containing benzoxazine monomer of formula (XX) in the composition thereof is is within the range of 0,5 weight% to 95 weight%, better of from 1 to 50 wt%, most preferably of from 5 to 10 wt%.

**15.** The process (3) according to claim 13 or 14, wherein the benzoxazine derivative different from the catalyst is the class of compounds selected from the group consisting of:

a 3,4-dihydro-2H-1,3-benzoxazine monomer having the formula

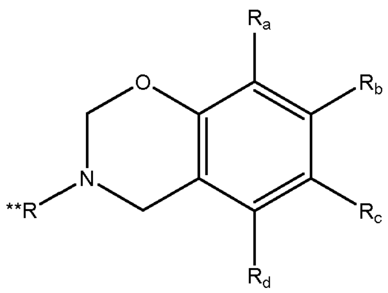

either wherein

$R_a = CH_3$
$R_b$ and $R_c$ together represent

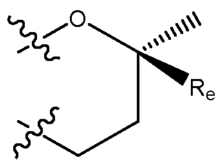

with

$R_e$ = 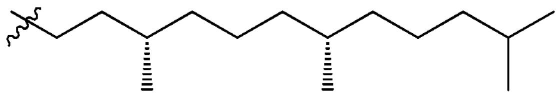

or

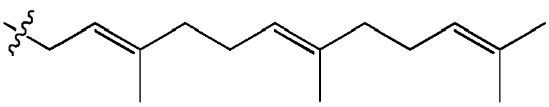,

and
$R_d$ = H, or $CH_3$,
or wherein
$R_d$ and $R_c$ together represent

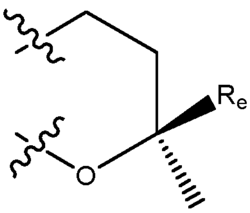

with

$R_e$ = 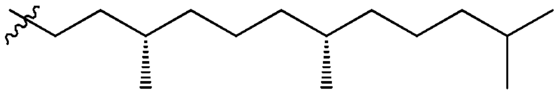

or

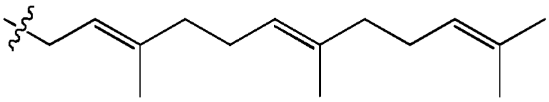,

and

$R_b = R_a = CH_3$,

or wherein

$R_c = R_d = H$,

$R_b$ = 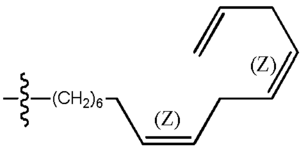, 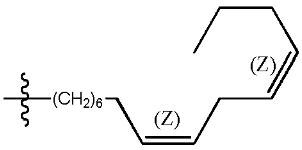, 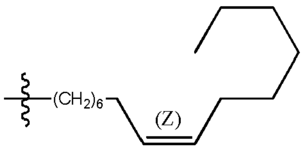

and/or

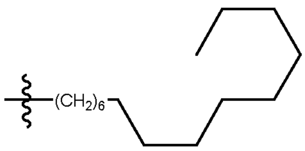

and $R_a$ = COOH,

or wherein

$R_a$ = H, $R_b$ = OH, $R_c$=H and

$R_d$ = 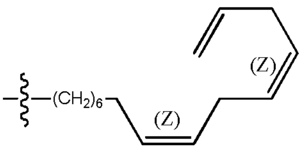, 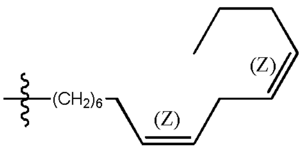, 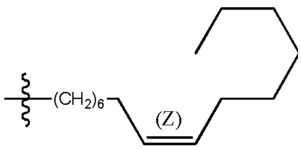

and/or

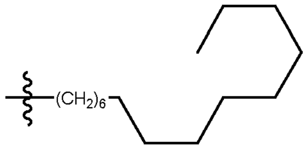

or wherein
$R_a$= H, and

$R_b$ = 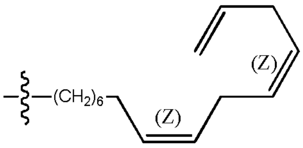, 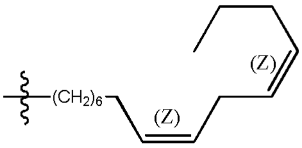, 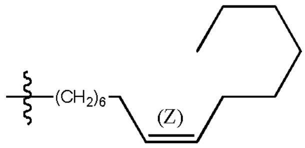

and/or

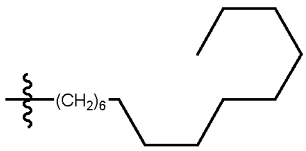

and $R_c$= H, $R_d$= OH,
or wherein
$R_a = CH_3$, $R_b$ = OH, $R_c$ = H

$R_d$ = 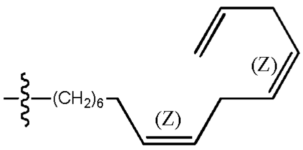, 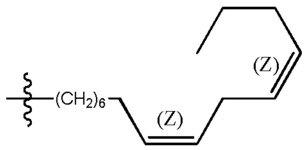, 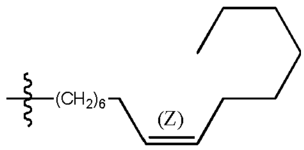

and/or

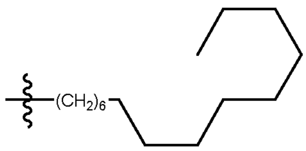

or wherein
$R_a = R_c = R_d$ = H, and
$R_b$ =

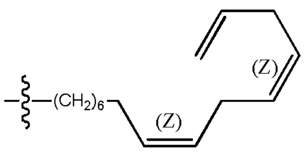, 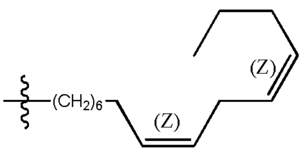, 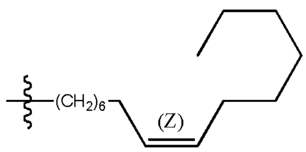

and/or

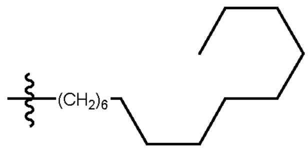

or wherein

$R_b = R_d = H$
$R_c = -CH_2-HC=CH_2$ and
$R_a = OCH_3$;
or
$R_b = R_d = H$
$R_c = -CH=HC-CH_3$ and
$R_a = OCH_3$,
or a mixture thereof, R** being defined in any of claims 1-3;

a compound of formula A, B or C, or mixture thereof, as follows Compound of formula A:

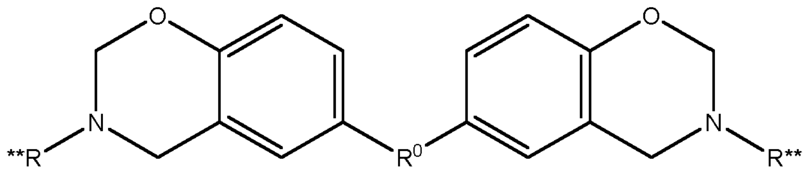

A

wherein

$R^0$ is a $-CH_2-$, $-C(CH_3)_2-$, $SO_2$, $-C(CF_3)_2-$, $-C(CH_3)(C_6H_5)-$, $-C(CH_3)(C_2H_5)-$, $-C(C_6H_5)_2-$ , $-CHCH_3-$, $-C_6H_{10}-$ or $-C(CH_3)CH_2CH_2COOH-$ group ;
R** is a $-CH_2CH_2OH$, vinyl, methyl, ethyl, propyl, isopropyl, butyl, hexyl, cyclohexyl, fluorene, phenylacetylene, phenyl propargyl ether, benzonitrile, furfuryl, phenylene or $-(CH_2)_{17}CH_3$ group;
compound of formula B

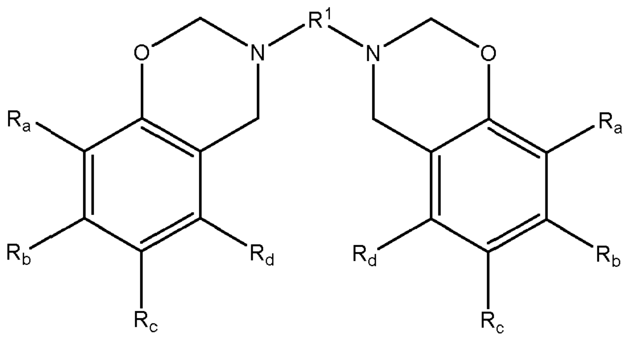

B

either wherein
$R^1$ is a $-(CH_2)_n-$ group, with n=1-10 or

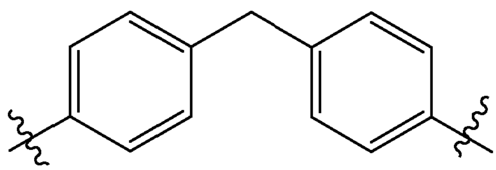

or

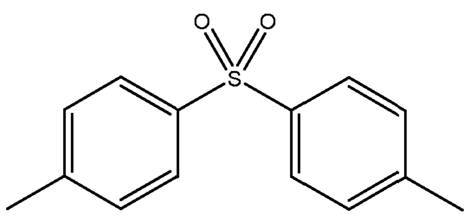

or

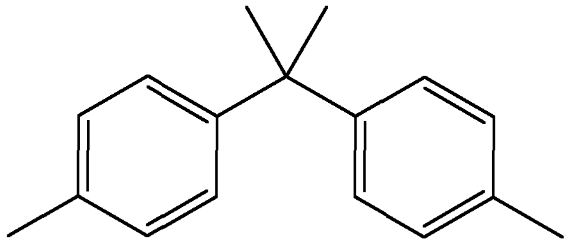

or

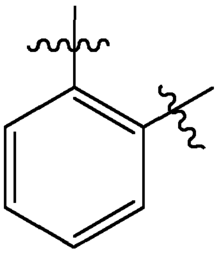 or 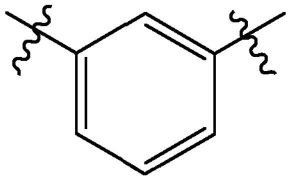 or 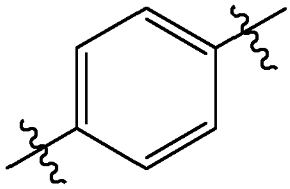

or

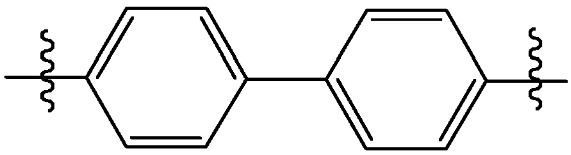

or

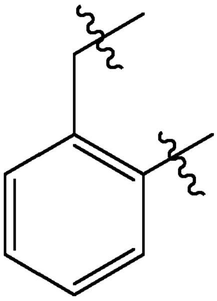 or 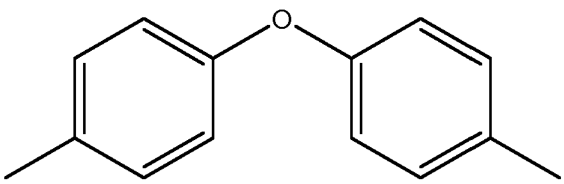

or

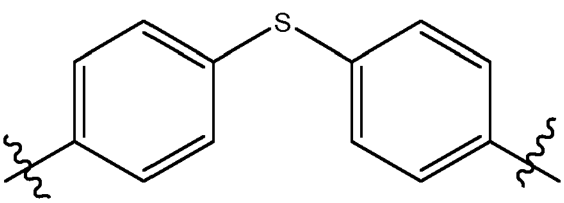 or 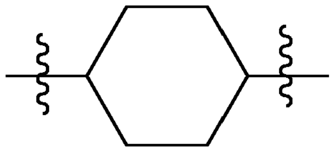 ,

and wherein $R_a$,
$R_b$, $R_c$, $R_d$ are selected from the group consisting of
$R_a = R_b = R_c = R_d = H$,

$R_a = OCH_3$, $R_b = R_d = H$, $R_c = CH_2\text{-}CH{=}CH_2$,
$R_a = OCH_3$, $R_b = R_d = H$, $R_c = CH{=}CH\text{-}CH_3$,
$R_a = OCH_3$, $R_b = R_d = H$, $R_c = CHO$,
$R_a = OCH_3$, $R_b = R_c = R_d = H$,
$R_a = OCH_3$, $R_b = R_d = H$, $R_c = CHO$,
$R_a = OCH_3$, $R_b = R_d = H$, $R_c = CH_2CH_2COOH$,
$R_a = R_b = R_d = H$, $R_c = CH_2CH_2COOH$,
$R_a = R_b = R_d = H$, $R_c = CH{=}CHCOOH$, and
$R_a = OCH_3$, $R_b = R_d = H$, $R_c = CH{=}CHCOOH$,
or wherein
$R_c = R_d = H$,

$R_b$ = 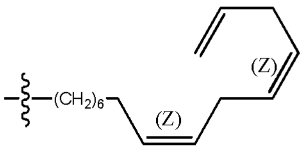 , 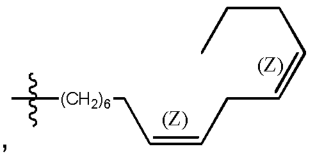 , 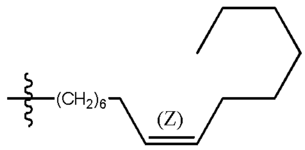

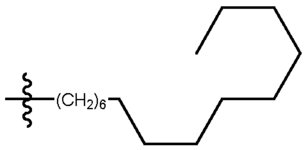 ,

or
and $R_a = COOH$,
or wherein
$R_a = H$, $R_b = OH$, $R_c$=H and

$R_d$ = 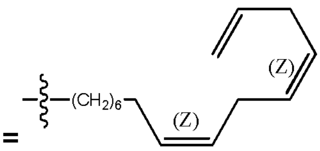 , 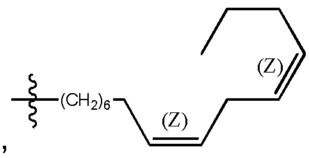 , 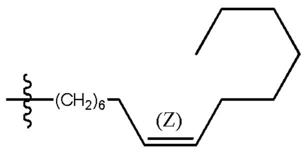

or

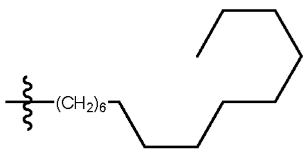 ,

or wherein
$R_a$= H, and

$R_b$ = 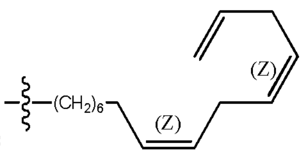 , 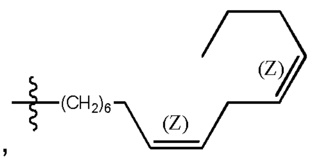 , 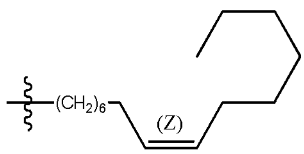

or

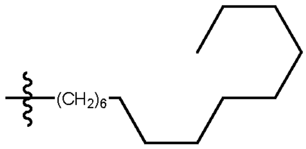,

and $R_c$= H, $R_d$= OH,
or wherein
$R_a$ = $CH_3$, $R_b$ = OH, $R_c$ = H

$R_d$ = 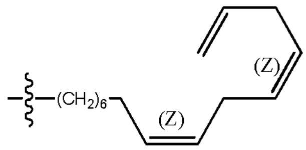, 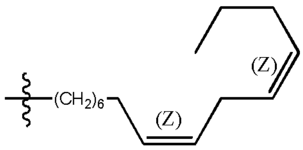, 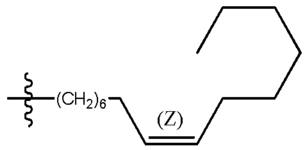

or

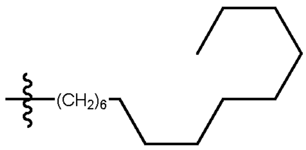,

or wherein
$R_a$ = $R_c$ = $R_d$ = H, and

$R_b$ = 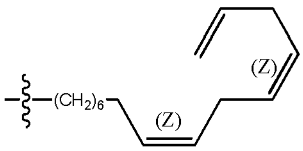, 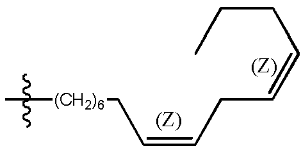, 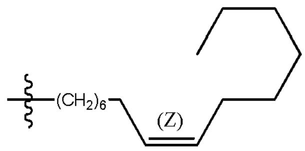

or

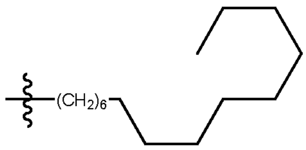,

or wherein
$R_a$ = $R_c$ = H
$R_b$ = $-CH_2HC=CH_2$, and $R_d$ = $OCH_3$;
or
$R_a$ = $R_c$ = H
$R_b$ = $-CH=HC-CH_3$ and
$R_d$ = $OCH_3$ ;
or $R_a$ = $R_b$ = $R_d$ = Hand

$R_c$ = 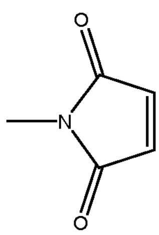,

or $R_a = R_b = R_c = H$ and $R_d = CH_2CH{=}CH_2$
or $R_a = R_b = R_d = H$ and $R_c = CH_2CH{=}CH_2$
or $R_a = R_c = R_d = H$ and $R_b = CH_2CH{=}CH_2$; and

a compound of formula C

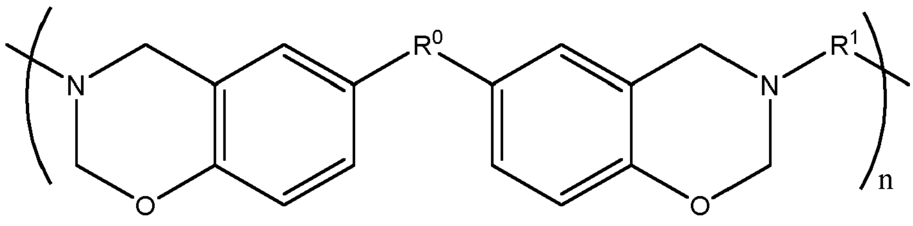

C

wherein $R^0$ is a $-CH_2-$, $-C(CH_3)_2-$, $SO_2$, $-C(CF_3)_2-$, $-C(CH_3)(C_6H_5)-$, $-C(CH_3)(C_2H_5)-$, - $C(C_6H_5)_2-$, $-CHCH_3-$, $-C_6H_{10}-$ or $-C(CH_3)CH_2CH_2COOH-$ group ;
and either within
n is an integer from 1 to 10;
$R^1$ is a $-(CH_2)_n-$ group, with n=1-10;
or

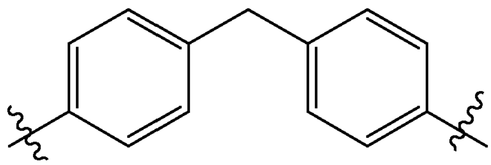

or

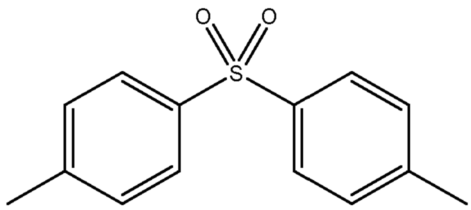

or

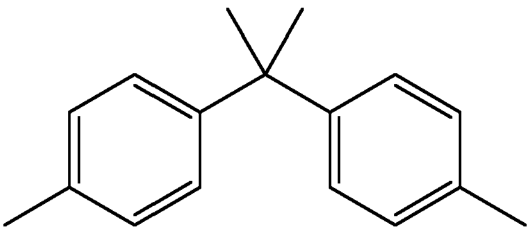

or

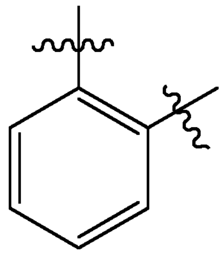

.

**Patentansprüche**

1. Ein Benzoxazin, das freie aliphatische Hydroxylgruppen und einen Monoester der Formel (I)

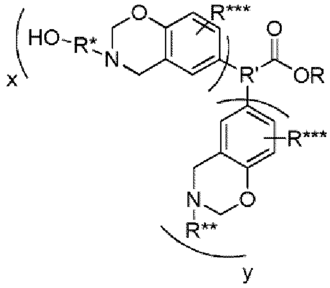

enthält, worin R aus der Gruppe ausgewählt ist, die aus einer linearen oder verzweigten $C_1$-$C_{12}$-Alkyl- oder Alkoxygruppe, einer linearen oder verzweigten $C_2$-$C_6$-Alkenyl- oder Alkylenoxygruppe, einer substituierten oder unsubstituierten linearen oder verzweigten $C_2$-$C_6$-Alkinylgruppe, eine Cyclo($C_3$-$C_6$-Alkyl)-Gruppe, eine Heteocyclo($C_3$-$C_6$-Alkyl)-Gruppe, eine lineare oder verzweigte $C_1$-$C_6$-Alkyl- oder $C_2$-$C_6$-Alkenylgruppe, die substituiert oder unsubstituiert ist, eine Phenylgruppe und eine $(CH_2)_{n3}$-Henylgruppe, wobei n3 eine ganze Zahl von 1 bis 10 ist;

R' ausgewählt ist aus der Gruppe bestehend aus mindestens einem von -CH, einer C-$(CH_2)_{n3}$-$CH_3$-Gruppe, einer C-$(CH_2)n_3$-CH-$(CH_3)_2$-Gruppe, einer C-$(CH_2)_{n3}$-$(CHZ)_{n4}$-$(CH_3)_2$-Gruppe, eine C-$(CH_2)n_3$-$(CHZ)n_4$-$(CH_2)n_3$-$CH_3$-Gruppe, eine C-$(CHZ)n_4$-$(CH_2)_{n3}$-$CH_3$-Gruppe, eine C-$(CHZ)n_4$-$[(CH_2)n_3$-$CH_3]_2$-Gruppe, eine C-substituierte oder unsubstituierte lineare oder verzweigte $C_2$-$C_6$-Alkenylgruppe, eine lineare oder verzweigte $C_1$-$C_6$-Alkylgruppe, die substituiert oder unsubstituiert ist, Phenyl oder Phenyl mit mindestens einem Heteroatom, ausgewählt aus N, O und S, eine lineare oder verzweigte C-$(CH_2)n_3$-$C_1$-$C_6$-Alkylgruppe, die substituiert oder unsubstituiert ist, Phenyl oder Phenyl mit mindestens einem Heteroatom, ausgewählt aus N, O und S, einem linearen oder verzweigten C-$(CH_2)n_3$-$C_1$-$C_6$-Alkyl, das substituiert oder unsubstituiert ist, Phenyl oder Phenyl, das mindestens ein Heteroatom, ausgewählt aus N, O und S-$(CH_2)n_3$-$CH_3$, enthält, einem linearen oder verzweigten C-$(CH_2)n_3$-$C_1$-$C_6$-Alkyl, das substituiert oder unsubstituiert ist, Phenyl oder Phenyl, das mindestens ein Heteroatom, ausgewählt aus N, O und S-$(CH_2)n_3$-CH-$(CH_3)_2$, einem linearen oder verzweigten C-$(CH_2)n_3$-$C_1$-$C_6$-Alkyl, das substituiert oder unsubstituiert ist, Phenyl oder Phenyl, das mindestens ein Heteroatom, ausgewählt aus N, O und S-$(CH_2)_{n3}$-$(CHZ)_{n4}$-$(CH3)_2$, enthält, einem linearen oder verzweigten C-$(CH_2)n_3$-$C_1$-$C_6$-Alkyl, das substituiert oder unsubstituiert ist, Phenyl oder Phenyl, das mindestens ein Heteroatom enthält, das aus N, O und der Gruppe S-$(CHZ)_{n4}$-(CH2)n3-CH3 ausgewählt ist, und eine lineare oder verzweigte C-(CHa2)$_{n3}$-$(CHZ)_{n4}$-$C_1$-$C_6$-Alkylgruppe, die substituiert oder unsubstituiert ist, oder Phenyl, das mindestens ein Heteroatom enthält, das aus N, O und der Gruppe S-$(CH_2)_{n3}$-$CH_3$ ausgewählt ist, worin n3 und n4 unabhängig voneinander, eine ganze Zahl von 1 bis 10 sind und Z ausgewählt ist aus der Gruppe bestehend aus einer linearen oder verzweigten $C_1$-$C_6$-Alkyl- oder -Alkoxygruppe, einer linearen oder verzweigten $C_2$-$C_6$-Alkenyl- oder - Alkylenoxygruppe und einer linearen oder verzweigten $C_1$-$C_6$-Alkyl- oder $C_2$-$C_6$-Alkenylgruppe, die substituiert oder unsubstituiert ist, und mindestens ein O-Atom zwischen zwei benachbarten C vorhanden ist oder nicht, oder R' ausgelassen ist.

R* ist ausgewählt aus der Gruppe bestehend aus einer linearen oder verzweigten $C_1$-$C_{20}$-Alkyl- oder -Alkoxygruppe, einer Cyclo($C_3$-$C_6$-Alkyl)-Gruppe, einer Heteocyclo($C_3$-$C_6$-Alkyl)-Gruppe, wobei das Heteroatom aus N, S und O ausgewählt ist, einer linearen oder verzweigten $C_2$-$C_6$-Alkenyl- oder -Alkylenoxygruppe, einer substituierten oder unsubstituierten linearen oder verzweigten $C_2$-$C_6$-Alkinylgruppe, einer linearen oder verzweigten $C_1$-$C_6$-Alkyl- oder $C_2$-$C_6$-Alkenyl substituierte oder unsubstituierte Phenylgruppe, eine $(CH_2)n_3$-Phenylgruppe und -$(CH_2)_{n3}$-O-$(CH_2)_{n4}$, worin $n_3$ und $n_4$, unabhängig voneinander eine ganze Zahl von 1 bis 10 sind;

R** dasselbe wie R* ist und ferner ein Mitglied einschließt, das aus einer O-, N- oder S-$(CH_2)n_3$-CH-$(CH_3)_2$-Gruppe, einer O-, N- oder S-$(CH_2)_{n3}$-$(CHZ)_{n4}$-$(CH_3)_2$-Gruppe, einer O-, N- oder S-$(CH_2)n_3$-$(CHZ)n_4$-(CH2)$_{n3}$-$CH_3$-Gruppe, einer O-, N- oder S-$(CHZ)_{n4}$-$(CH_2)_{n3}$-$CH_3$-Gruppe, eine O-, N- oder S-$(CHZ)_{n4}$-$[(CH_2)_{n3}$-$CH_3]_2$-Gruppe und eine O-substituierte oder unsubstituierte lineare oder verzweigte $C_2$-$C_6$-Alkinylgruppe, wobei Z wie für R' definiert ist, eine -$(CH_2)_{n3}$-C=N-Gruppe, ein polyzyklischer aromatischer oder heteroaromatischer Kohlenwasserstoff, wie Naphthalin, Anthracen, Fluoren, Phenanthren, gegebenenfalls substituiert durch eine lineare oder verzweigte $C_1$-C6-Alkyl- oder -Alkoxygruppe, eine Cyclo($C_3$-$C_6$-Alkyl)gruppe, eine Heterocyclo($C_3$-$C_6$-Alkyl)gruppe, eine lineare oder verzweigte $C_2$-C6-Alkenyl- oder - Alkylenoxygruppe oder durch eine substituierte oder unsubstituierte lineare oder verzweigte $C_2$-$C_6$-Alkinylgruppe, wobei n3 und n4 unabhängig voneinander eine ganze Zahl von 1 bis 10 sind;

R*** ist ausgewählt aus der Gruppe bestehend aus H, OH und einer linearen oder verzweigten $C_1$-$C_6$-Alkylgruppe, und schließt ferner eine lineare oder verzweigte $C_1$-$C_{15}$-Alkylgruppe oder eine $C_2$-$C_{15}$-Alkenylgruppe oder

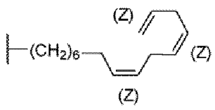 , 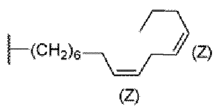 , 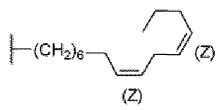

oder

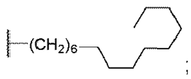

und der x-Wert von 0 bis 1 ist und der y-Wert 1-x ist.

2. Das Benzoxazin nach Anspruch 1, wobei R ausgewählt ist aus der Gruppe bestehend aus einer linearen oder verzweigten $C_1$-$C_4$-Alkyl- oder Alkoxygruppe, einer linearen oder verzweigten $C_2$-$C_4$-Alkenyl- oder Alkylenoxygruppe, einer unsubstituierten linearen oder verzweigten $C_2$-$C_4$-Alkinylgruppe, einer unsubstituierten Phenylgruppe und einer $(CH_2)_{n3}$-Phenylgruppe, wobei n3 eine ganze Zahl von 1 bis 6 ist;

R' ist ausgewählt aus der Gruppe bestehend aus mindestens einem von -CH, einer C-$(CH_2)_{n3}$-$CH_3$-Gruppe, einer C-$(CH_2)_{n3}$-CH-$(CH_3)$2-Gruppe, einer C-(CH2)n3-(CHZ)n4-(CH3)2-Gruppe, C-$(CH_2)_{n3}$-$(CHZ)_{n4}$-$(CH_2)_{n3}$-$CH_3$-Gruppe, C-$(CHZ)_{n4}$-(CH2)$_{n3}$-$CH_3$-Gruppe, einer C-$(CHZ)_{n4}$-[$(CH_2)_{n3}$-$CH_3$]$_2$-Gruppe, eine C-substituierte oder unsubstituierte lineare oder verzweigte $C_2$-$C_4$-Alkenylgruppe, ein unsubstituiertes Phenyl oder Phenyl, das mindestens ein Heteroatom, ausgewählt aus N, O und S, enthält, ein C-$(CH_2)_{n3}$-unsubstituiertes Phenyl oder Phenyl, das mindestens ein Heteroatom, ausgewählt aus N, O und S, enthält, ein C-$(CH_2)_{n3}$-unsubstituiertes Phenyl oder Phenyl, das mindestens ein Heteroatom, ausgewählt aus N, O und S-$(CH_2)_{n3}$-$CH_3$, enthält, ein C-$(CH_2)_{n3}$-unsubstituiertes Phenyl oder Phenyl, das mindestens ein Heteroatom, ausgewählt aus N, O und S-$(CH_2)_{n3}$-CH-$(CH_3)_2$, enthält ein C-$(CH_2)_{n3}$-unsubstituiertes Phenyl oder Phenyl, das mindestens ein Heteroatom enthält, das aus N, O und der Gruppe S-$(CH_2)_{n3}$-$(CHZ)_{n4}$-$(CH_3)_2$ ausgewählt ist, ein C-$(CH_2)_{n3}$-unsubstituiertes Phenyl oder Phenyl, das mindestens ein Heteroatom enthält, das aus N, O und der Gruppe S-$(CHZ)_{n4}$-$(CH_2)_{n3}$-$CH_3$ ausgewählt ist, und ein C-$(CH_2)_{n3}$-$(CHZ)_{n4}$-unsubstituiertem Phenyl oder Phenyl, das mindestens ein Heteroatom enthält, ausgewählt aus N, O und einer S-$(CH_2)_{n3}$-$CH_2$-Gruppe, worin n3 und n4 unabhängig voneinander eine ganze Zahl von 1 bis 6 sind und Z ausgewählt ist aus der Gruppe, bestehend aus einer linearen oder verzweigten $C_1$-$C_4$-Alkyl- oder Alkoxygruppe, einer linearen oder verzweigten $C_2$-$C_4$-Alkenyl- oder Alkylenoxygruppe und einer unsubstituierten Phenylgruppe, und mindestens ein O-Atom zwischen zwei benachbarten C vorhanden ist oder nicht;
R* ausgewählt ist aus der Gruppe bestehend aus einer linearen oder verzweigten $C_1$-$C_6$-Alkyl- oder Alkoxygruppe, einer linearen oder verzweigten $C_2$-$C_4$-Alkenyl- oder Alkylenoxygruppe, einer unsubstituierten linearen oder verzweigten $C_2$-$C_4$-Alkinylgruppe, einer unsubstituierten Phenylgruppe, einer $(CH_2)_{n3}$-Phenylgruppe und -$(CH_2)_{n3}$-O-$(CHZ)_{n4}$, worin n3 und n4 unabhängig voneinander eine ganze Zahl von 1 bis 6 sind;
R** das gleiche wie R* ist und ferner ein Mitglied einschließt, das aus einer O-, N- oder S-$(CH_2)_{n3}$-CH-$(CH_3)_2$-Gruppe, einer O-, N- oder S-$(CH_2)_{n3}$-$(CHZ)_{n4}$-$(CH_3)_2$-Gruppe, einer O-, N- oder S-(CH2)n3-(CHZ)n4-(CH2)n3-CH3-Gruppe, einer O-, N- oder S-$(CHZ)_{n4}$-$(CH_2)_{n3}$-$CH_3$-Gruppe, eine O-, N- oder S-$(CHZ)_{n4}$-[$(CH_2)_{n3}$-$CH_3$]$_2$-Gruppe und eine O-substituierte oder unsubstituierte lineare oder verzweigte $C_2$-$C_4$-Alkinylgruppe, wobei Z wie oben definiert ist, eine -$(CH_2)_{n3}$-C≡N-Gruppe, eine Cyclo($C_3$-$C_4$-Alkyl)-, eine Heteocyclo($C_3$-$C_4$-Alkyl)-Gruppe, ein polyzyklischer aromatischer oder heteroaromatischer Kohlenwasserstoff (PAH), wobei das Heteroatom eine Auswahl aus N, S und O ist, wie Naphthalin, Anthracen, Fluoren, Furan, gegebenenfalls substituiert durch eine lineare oder verzweigte $C_1$-$C_4$-Alkyl- oder Alkoxygruppe, eine lineare oder verzweigte $C_2$-$C_4$-Alkenyl- oder Alkylenoxygruppe oder durch eine substituierte oder unsubstituierte lineare oder verzweigte $C_2$-$C_4$-Alkinylgruppe, wobei n3 und n4 unabhängig voneinander eine ganze Zahl von 1 bis 6 sind;
R*** ist ausgewählt aus der Gruppe bestehend aus H, OH und einer O-linearen oder verzweigten $C_1$-$C_4$-Alkylgruppe, und umfasst ferner eine lineare oder verzweigte $C_1$-$C_{10}$-Alkylgruppe oder $C_2$-$C_{10}$-Alkenylgruppe oder

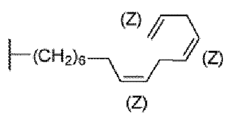,
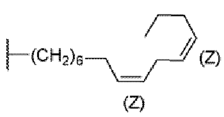,
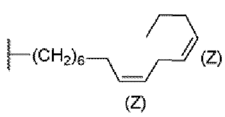

oder

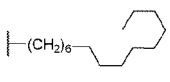

3. Das Benzoxazin nach Anspruch 1, worin R ausgewählt ist aus der Gruppe bestehend aus Gruppen -$CH_3$, -$(CH_2)_{n3}$-$CH_3$, -$(CH_2)_{n3}$-CH-[$(CH_2)_{n3}$-$CH_3$]$_2$, -C$(CH_3)_3$, - $(CH_2)_{n3}$-$(C_6H_5)$, -$(CH_2)_{n3}$-CH=$CH_2$ und -$(CH_2)_{n3}$-C≡CH,

worin n3 eine ganze Zahl von 1 bis 5 ist;

R' aus der Gruppe ausgewählt ist, die aus den Gruppen -CH, -C-(CH2)$_2$-C($CH_3$), -C-CH($CH_2CH_3$), -C($CH_2CH_2CH_3$), -C-CH2($CH_2$)$_3$$CH_3$, -C-$CH_2$($CH_2$)$_4$$CH_3$, -C($C_6H_5$), - C($CH_3$)$CH_2$, C($CH_3$)$CH_2$CH2 und -C($C_6H_5$)$CH_2$-CH3 besteht;
R* ist ausgewählt aus der Gruppe bestehend aus den Gruppen -$CH_3$, -($CH_2$)$_{n3}$-$CH_3$, - ($CH_2$)$_{n3}$-CH-[($CH_2$)$_{n4}$-$CH_3$]$_2$, -C($CH_3$)$_3$, ($CH_2$)$_{n3}$-($C_6H_5$), -($CH_2$)$_{n3}$-CH=$CH_2$, -($CH_2$)$_{n3}$-C=CH, -($CH_2$)$_{n3}$-O-($CH_2$)$_{n4}$, worin n3 und n4 unabhängig voneinander eine ganze Zahl von 1 bis 4 sind, Phenyl und -($CH_2$)$_3$-Phenyl;
R** ist ausgewählt aus der Gruppe bestehend aus den Gruppen $CH_3$, -($CH_2$)$_{n3}$-$CH_3$, - ($CH_2$)$_{n3}$-CH- [($CH_2$)$_{n4}$-$CH_3$]$_2$, -C(CH3)3, ($CH_2$)n3-($C_6H_5$), -($CH_2$)$_{n3}$-CH=$CH_2$, -($CH_2$)$_{n3}$-C=CH, O-($CH_2$)$_{n3}$-C=CH, O-($CH_2$)$_{n3}$-C=N, ($CH_2$)$_{n3}$-C=N, und -($CH_2$)$_{n3}$-substituiertes oder unsubstituiertes Furan, Phenyl, und worin n3 und n4 unabhängig voneinander eine ganze Zahl von 1 bis 4 sind:
R*** ist ausgewählt aus der Gruppe bestehend aus H, OH und O-linearer oder verzweigter C1-C3-Alkylgruppe, und schließt ferner lineare oder verzweigte $C_1$-$C_6$-Alkylgruppe oder $C_2$-$C_6$-Alkenylgruppe oder

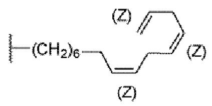 , 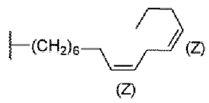 , 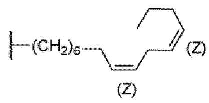

oder

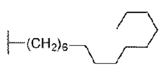

wobei H die bevorzugte Variante ist.

**4.** Verfahren (1) zur Herstellung eines Benzoxazins, das freie aliphatische Hydroxylgruppen und einen Monoester der Formel (I) enthält, umfassend die folgenden Schritte

a) Umsetzung eines Phenolsäurederivats der Formel (II), das mindestens eine R***-Gruppe,

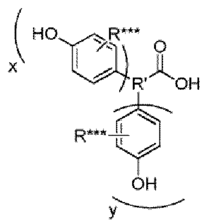

(II)

mit einem monofunktionellen Oligomer oder Molekül der Formel (III)

R-OH (III)

bei einer Temperatur von 80°C bis 200°C während 12 bis 48 Stunden in Gegenwart eines Säurekatalysators vom Bronsted-Typ, was zu einem Oligomer oder Molekül mit Phenol-Endgruppen der Formel (IV) führt

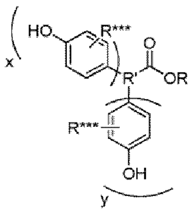

(IV)

und

b) Reaktion des phenolterminierten Oligomers oder Moleküls der Formel (IV) mit einer Mischung aus

- eines Aminoalkohols der Formel (V):

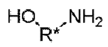

(V)

- ein primäres Aminderivat der Formel (VI)

R**-NH2 (VI),

und

- Paraformaldehyd der Formel (VII)

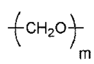

m=8-100

bei einer Temperatur im Bereich von 80°C bis 100°C, von 1h bis 48h, unter Rühren, wobei R, R', R*, R**, R***, x und y unabhängig voneinander wie in einem der Ansprüche 1-3 definiert sind, mit der Maßgabe, dass, wenn das mindestens eine R*** des Phenolsäurederivats in *ortho*-Position in Bezug auf die -OH-Gruppe ist, R*** H ist.

**5.** Verfahren nach Anspruch 4, wobei das Phenolsäurederivat (Formel (II)) 1 bis 4 R***-Gruppe(n), bezogen auf die Substitution des Phenolrings, enthält.

**6.** Verfahren nach Anspruch 4 oder 5, wobei das Phenolsäurederivat ausgewählt ist aus der Gruppe bestehend aus Mono-, Di-, Tri-Hydroxybenzoesäure-Derivaten, Anacardsäure-Derivaten, Hydroxyzimtsäure-Derivaten, aliphatischen X-Hydroxyphenylsäure-Derivaten, wobei X 2-4 ist, und aliphatischen Diphenolsäure-Derivaten oder Mischungen davon.

**7.** Verfahren nach einem der Ansprüche 4 bis 6, wobei die jeweilige Stöchiometrie der Ausgangsreaktanten in Schritt a), Phenolsäurederivat:monofunktionelles Molekül oder Oligomer, 1,0-3,0 Äquivalente: 1,0 Äquivalente beträgt, was zu einem 1,0 Äquivalente Phenol-terminierten Oligomer oder Molekül führt.

**8.** Verfahren nach einem der Ansprüche 4 bis 7, wobei die primären Aminderivate R** mit der Definition von R* tragen und ferner ausgewählt sind aus der Gruppe bestehend aus Allylamin, Methylamin, Ethylamin, Propylamin, Butylamin, Isopropylamin, Hexylamin, Cyclohexylamin, Stearylamin, 2-Aminofluoren, Aminophenylacetylen, Propargyletheranilin, 4-Aminobenzonitril, Furfurylamin und Anilin oder Mischungen davon, und wobei der Aminoalkohol der Formel (V) ausgewählt ist aus der Gruppe bestehend aus 2-Aminoethanol, 2-Amino-2-methylpropanol, 5-Aminopentan-1-ol, Heptaminol und Diglycolamin oder Mischungen davon.

**9.** Verfahren nach einem der Ansprüche 4 bis 8, wobei die jeweilige Stöchiometrie der Ausgangsreaktanten in Schritt b), Phenol-terminiertes Oligomer oder Molekül:Aminoalkohol:primäres Aminderivat:Paraformaldehyd 1,0 eq.: x(1,0 eq-18,0 eq):y(1,0 eq-18,0 eq):2,0-36,0 eq, was zu einem 1,0 eq des Benzoxazins führt, das freie aliphatische Hydroxylgruppen und Monoester enthält, wobei x = 0-1 und y =1-x.

10. Benzoxazin-Katalysator für die ringöffnende Polymerisation (ROP), umfassend ein Benzoxazin, das freie aliphatische Hydroxylgruppen und einen Monoester der Formel (I) nach einem der Ansprüche 1 bis 3 enthält, oder wie er nach dem Verfahren (1) nach einem der Ansprüche 4-9 erhältlich ist.

11. Verwendung eines Benzoxazins, das freie aliphatische Hydroxylgruppen enthält, und eines Monoesters der Formel (I) nach einem der Ansprüche 1 bis 3 oder wie nach dem Verfahren (1) nach einem der Ansprüche 4-9 erhältlich, oder eines Esters, der ein Benzoxazinmonomer der Formel (XX) enthält

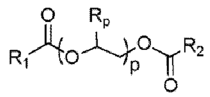
(XX)

Wobei

R1 ist

R2 ist

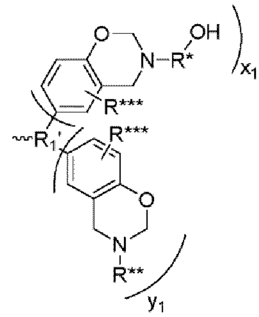

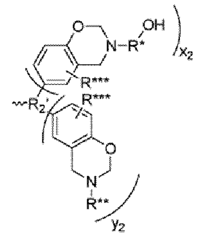

und

$R_p$ ist ausgewählt aus der Gruppe bestehend aus H, einer linearen oder verzweigten C1-C6-, vorzugsweise C1-C4-Alkyl- oder Alkoxygruppe, einer linearen oder verzweigten C2-C6-, vorzugsweise C2-C4-Alkenyl- oder Alkylenoxygruppe, einer substituierten oder unsubstituierten linearen oder verzweigten C2-C6-, vorzugsweise C2-C4-Alkinylgruppe, einer linearen oder verzweigten C1-C6-, vorzugsweise C1-C4-Alkyl- oder C2-C6-, vorzugsweise C2-C4-Alkenylgruppe, die substituiert oder unsubstituiert ist, einer Phenylgruppe und

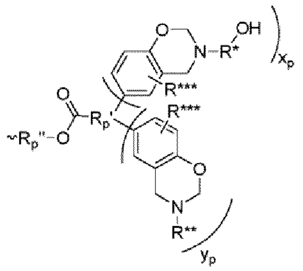

worin

R1 und R2 der Formel XX gleich oder verschieden sind;
X1, X2 und Xp, unabhängig voneinander, von 0 bis 1 sind und nicht zusammen 0 sind;

$$y_1 = 1\text{-}X_1;\ y_2 = 1\text{-}X_2\text{: } Y_p = 1\text{-}x_p;$$

p ist 1-100;
Ri', R2' und Rp' unabhängig voneinander ausgewählt sind aus der Gruppe bestehend aus: einer -C linearen oder verzweigten C1-C5 Alkyl- oder Alkoxygruppe, einer -C-linearen oder verzweigten C2-C6-Alkenyl- oder Alkylenoxygruppe, einer -C-substituierten oder unsubstituierten linearen oder verzweigten C2-C5-Alkinylgruppe, und eine -C-lineare oder verzweigte substituierte oder unsubstituierte C1-C6-Alkyl- oder C2-C6-Alkenyl Phenylgruppe;

$R_p$" ist ausgewählt aus der Gruppe bestehend aus einer linearen oder verzweigten C1-C6-Alkyl- oder Alkoxygruppe, einer linearen oder verzweigten C2-C6-Alkenyl- oder Alkylenoxygruppe, einer substituierten oder unsubstituierten linearen oder verzweigten C2-C6-Alkinylgruppe und einer linearen oder verzweigten substituierte oder unsubstituierte C1-C6-Alkyl- oder C2-C6-Alkenyl Phenylgruppe;

R*, R** und R*** unabhängig voneinander wie in einem der Ansprüche 1-3 definiert sind,

worin

$X_1$, $X_2$, $X_p$ und $y_1$, $y_2$, $y_p$ sind definiert als:

$$x1 = \frac{n\text{Aminoalkohol (R1)}}{n\text{ Amine gesamt(R1)}}$$

$$x2 = \frac{n\text{Aminoalkohol (R2)}}{n\text{ Amine gesamt(R2)}}$$

$$xp = \frac{n\text{Aminoalkohol (Rp)}}{n\text{ Amine gesamt(Rp)}}$$

$$y1 = \frac{n\text{Amine (R1)}}{n\text{ Amine gesamt(R1)}}$$

$$y2 = \frac{n\text{Aminoalkohol (R2)}}{n\text{ Amine gesamt(R2)}}$$

$$yp = \frac{n\text{Amine (Rp)}}{n\text{ Amine gesamt(Rp)}}$$

worin $n_{Amine\ gesamt\ (R1)} = n_{Amine(R1)} + n_{Aminoalkohol\ (R1)}$ *und* $n_{Aminoalkohol\ (R1)}$ die Anzahl der Aminoalkohole pro Gruppe R1 sind, $n_{Amine(R1)}$ die Anzahl der Amine (mit Ausnahme der Anzahl der Aminoalkohole) pro Gruppe R1 darstellt und $n_{Amine\ gesamt\ (R1)} = n_{Amine(R1)} + n_{Aminoalkohol\ (R1)}$ *und* $n_{Aminoalkohol\ (R1)}$ die Gesamtzahl der Aminogruppen pro Gruppe $R_1$ ist,

worin $n_{Amine\ gesamt\ (R2)} = n_{Amine(R2)} + n_{Aminoalkohol\ (R2)}$ *und* $n_{Aminoalkohol\ (R2)}$ und $n_{Aminoalkohol\ (R2)}$ die Anzahl der Aminoalkohole pro R2-Gruppe sind, $n_{Amine(R2)}$ die Anzahl der Amine (mit Ausnahme der Anzahl der Aminoalkohole) pro Gruppe R2 darstellt und $n_{Amine\ gesamt\ (R2)} = n_{Amine(R2)} + n_{Aminoalkohol\ (R2)}$ *und* $n_{Aminoalkohol\ (R2)}$ und $n_{Aminoalkohol\ (R2)}$ die Gesamtzahl der Aminogruppen pro Gruppe R2 ist;

worin $n_{Aminegesamt\ (Rp)} = n_{Amine(Rp)} + n_{Aminoalkohol\ (Rp)}$ *und* $n_{Aminoalkohol\ (Rp)}$ und $n_{Aminoalkohol\ (Rp)}$ die Anzahl der Aminoalkohole pro Rp-Gruppe sind, $n_{Amine(Rp)}$ die Anzahl der Amine (außer der Anzahl der Aminoalkohole) pro Rp-Gruppe darstellt und $n_{Amine\ gesamt\ (Rp)} = n_{Amine(Rp)} + n_{Aminoalkohol(Rp)}$ *und* $n_{Aminoalkohol\ (Rp)}$ die Gesamtzahl der Aminogruppen pro Rp-Gruppe ist, als Katalysator für die Benzoxazin-Polymerisation.

**12.** Ein Verfahren (2) zur Synthese eines esterhaltigen Benzoxazin-Monomers der Formel (XX), das die folgenden Schritte umfasst, bestehend aus:

a) Umsetzen eines Phenolsäurederivats der Formel (XXI), das mindestens eine R***-Gruppe am Phenolring enthält,

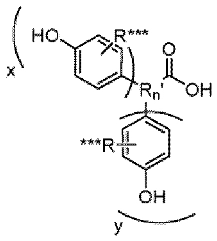

(XXI)

worin x von 0 bis 1 ist, und y=1-x,

mit einem polyfunktionellen Molekül oder Oligomer der Formel (XXII)

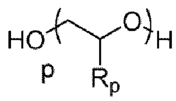

(XXII)

bei einer Temperatur von 25°C bis 200°C, während 1h-72h, in Gegenwart eines Katalysators vom Brönsted-Säure-Typ, was zu einem Oligomer oder Molekül mit Phenol-Endgruppen (Verbindung (XXIII)) führt und

b) Umsetzen der Verbindung (XXIII) mit einer Mischung aus:

- einen Amino-Alkohol der Formel (V):

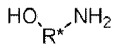

(V)

- ein primäres Aminderivat der Formel (VI), und

R**-NH2 (VI)

- Paraformaldehyd der Formel (VII)

bei einer Temperatur im Bereich von 80°C bis 100°C, von 1h bis 10h, unter Rühren, um die Verbindung der Formel (XX) zu erhalten,
worin Rp, R*, R**, R***, Rn', $R_1$' und $R_2$' und p unabhängig voneinander wie in einem der Ansprüche 1-3 und 11 definiert sind, mit der Maßgabe, dass, wenn das mindestens eine R*** des Phenolsäurederivats in ortho-Position in Bezug auf die -OH-Gruppe ist, R*** H ist.

**13.** Ein Verfahren (3) zur Herstellung eines Polybenzoxazinderivats, umfassend den Schritt des Polymerisierens einer Zusammensetzung, die ein Benzoxazin, das freie aliphatische Hydroxylgruppen und einen Monoester der Formel (I) enthält oder wie durch das Verfahren (1) gemäß einem der Ansprüche 4-9 erhältlich ist, oder ein esterhaltiges Benzoxazinmonomer der Formel (XX) gemäß Anspruch 3 oder wie durch das Verfahren (2) des Anspruchs 12 erhältlich ist, oder ein Gemisch davon, als Benzoxazin-Katalysator, und umfassend von 0 Gew.-% bis 99 Gew.-% eines Benzoxazin-Derivats, verschieden von dem Benzoxazin-Katalysator, bei Temperaturen im Bereich von 100°C bis 250°C für 1h bis 24h, um Polybenzoxazin-Derivate zu erhalten.

**14.** Ein Verfahren (3) nach Anspruch 13, wobei der Anteil des Benzoxazins, das freie aliphatische Hydroxylgruppen enthält, und des Monoesters der Formel (I) oder des esterhaltigen Benzoxazinmonomers der Formel (XX) in der Zusammensetzung davon im Bereich von 0,5 Gew.-% bis 95 Gew.-%, besser von 1 bis 50 Gew.-%, am meisten bevorzugt von 5 bis 10 Gew.-% liegt.

**15.** Ein Verfahren (3) nach Anspruch 13 oder 14, wobei es sich bei dem vom Katalysator verschiedenen Benzoxazinderivat um die Klasse von Verbindungen handelt, die ausgewählt ist aus der Gruppe bestehend aus:

einem 3,4-Dihydro-2H-1,3-benzoxazin-Monomer mit der Formel

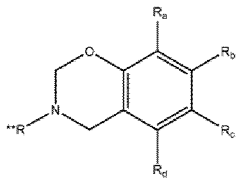

Entweder wobei

$R_a = CH_3$
$R_b$ und $R_c$ zusammen darstellen

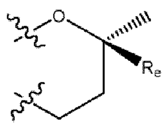

Mit $R_e$ =

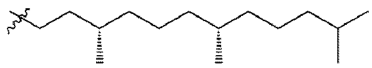

oder

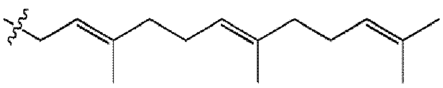

und
$R_d$ = H oder $CH_3$
entweder wobei
$R_d$ und $R_c$ darstellen zusammen

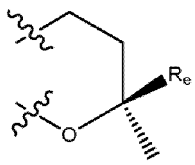

Mit

$R_e$ = 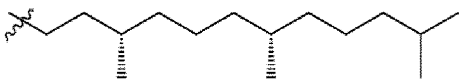

oder

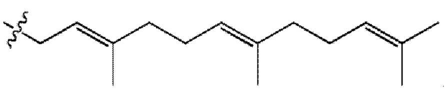

und
$R_b = R_a = CH_3$,

Oder wobei
$R_c = R_d = H$,

$R_b$ = 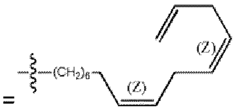 , 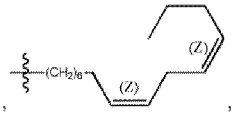 , 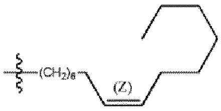

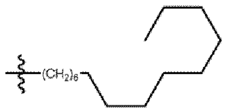

und/oder
Und Ra= CCOH
Oder wobei
Ra= H, Rb = OH, Rc = H
Und
and

$R_d$ = 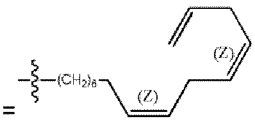 , 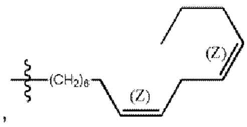 , 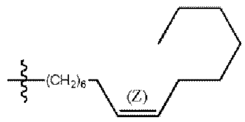

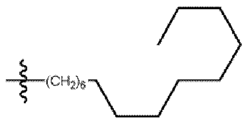

und/oder
Oder wobei
Ra = H
Und

$R_b$ = 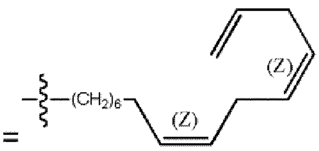 , 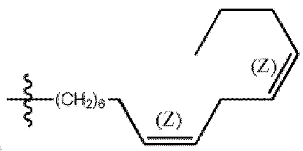 , 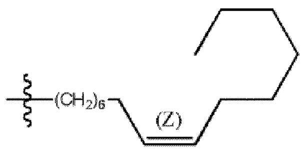

und/oder

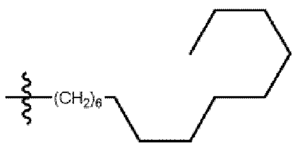

Und Rc = H, Rd = OH,
oder wobei
Ra= CH3, Rb = OH, Rc =H

$R_d$ = 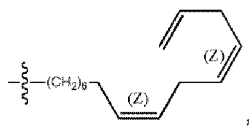 , 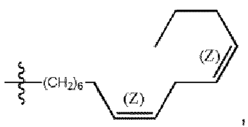 , 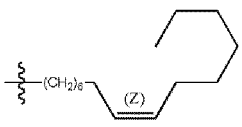

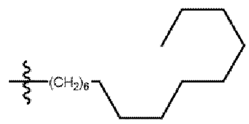

und/oder

Oder wobei

Ra = Rc = Rd = H, und

$R_b$ = 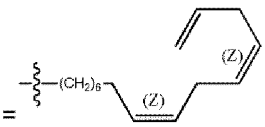 , 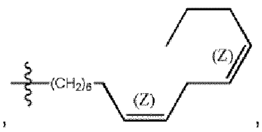 , 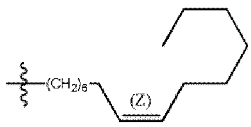

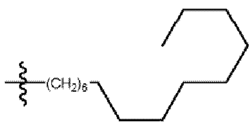

und/oder

Oder wobei

$R_b = R_d = H$

$R_c = -CH_2-HC=CH_2$ und

$R_a = OCH_3$ ;

Oder

$R_b = R_d = H$

$R_c = -CH=HC-CH_3$ und

$R_a = OCH_3$,

oder eine Mischung davon, wobei R** in einem der Ansprüche 1-3 definiert ist: eine Verbindung der Formel A, B oder C oder eine Mischung davon, wie folgt Verbindung der Formel A:

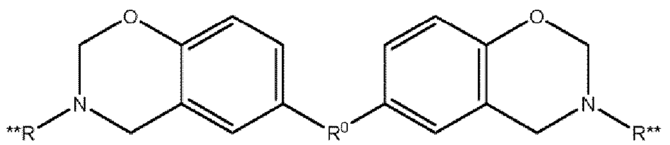

A

Wobei

$R^0$ eine $-CH_2-$, $-C(CH_3)_2-$, $SO_2$, $-C(CF_3)_2-$, $-C(CH_3)(C_6H_5)-$, $-C(CH_3)(C_2H_5)-$, $-C(C_6H_5)_2-$, $-CHCH_3-$, $-C_6H_{10}-$ or $-C(CH_3)CH_2CH_2COOH-$ Gruppe ist,

R** ist eine $-CH_2CH_2OH-$, Vinyl-, Methyl-, Ethyl-, Propyl-, Isopropyl-, Butyl-, Hexyl-, Cyclohexyl-, Fluoren-, Phenylacetylen-, Phenylpropargy!ether-, Benzonitril-, Furfuryl-, Phenylen- oder $-(CH_2)_{17}CH_3$-Gruppe;

Verbindung der Formel B:

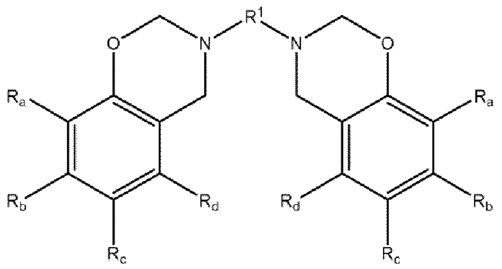

B

Wobei entweder

$R^1$ eine $-(CH_2)_n$-Gruppe ist, mit n= 1-10 Oder

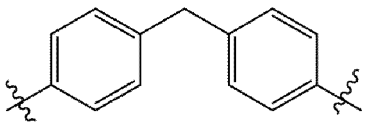

oder

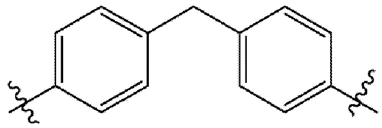

Oder

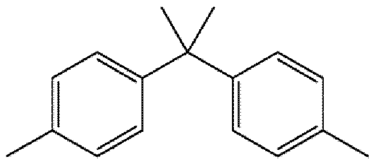

Oder

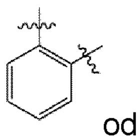

oder

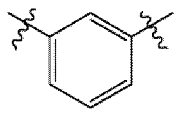

oder

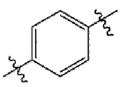

Oder

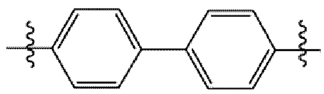

Oder

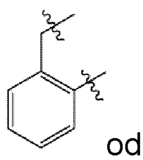

oder

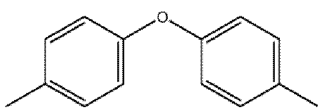

Oder

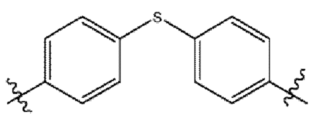

oder

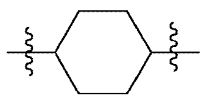

oder wobei Ra,
Rb, Re, Ra aus der Gruppe ausgewählt sind, bestehend aus
$R_a = R_b = R_c = R_d = H$,
$R_a = OCH_3$, $R_b = R_d = H$, $R_c = CH_2\text{-}CH{=}CH_2$,
$R_a = OCH_3$, $R_b = R_d = H$, $R_c = CH{=}CH\text{-}CH_3$,
$R_a = OCH_3$, $R_b = R_d = H$, $R_c = CHO$,
$R_a = OCH_3$, $R_b = R_c = R_d = H$,
$R_a = OCH_3$, $R_b = R_d = H$, $R_c = CHO$,
$R_a = OCH_3$, $R_b = R_d = H$, $R_c = CH_2CH_2COOH$,
$R_a = R_b = R_d = H$, $R_c = CH_2CH_2COOH$,
$R_a = R_b = R_d = H$, $R_c = CH{=}CHCOOH$, und
$R_a = OCH_3$, $R_b = R_d = H$, $R_c = CH{=}CHCOOH$,
Oder wobei
$R_c = R_d = H$, oder

$R_b$ = 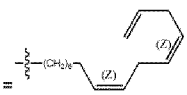, 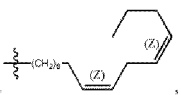, 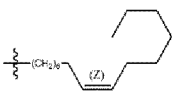

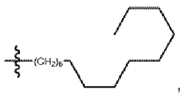,

und Ra=COOH
oder wobei
$R_a = H$, $R_b = OH$, $R_c=H$
Und

$R_d$ = 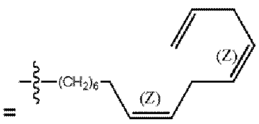, 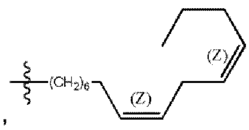, 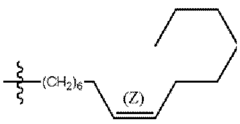

oder

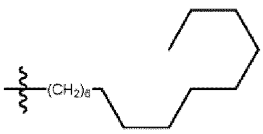,

Oder wobei
Ra= H

$R_b$ = 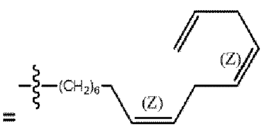, 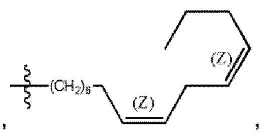, 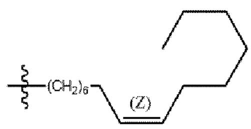.

Und oder

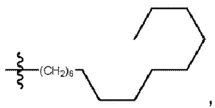,

Und Rc = H, Rd = OH,
oder wobei
$R_a = CH_3$, $R_b = OH$, $R_c = H$

$R_d$ = 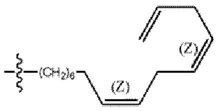, 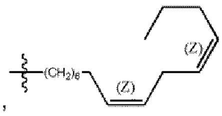, 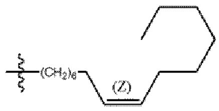

oder

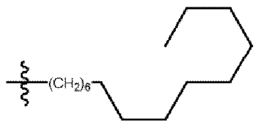,

Oder wobei
Ra = Rc = Rd = H, und

$R_b$ = 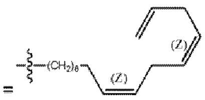, 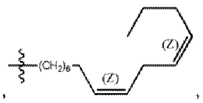, 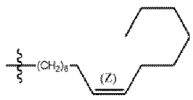

oder

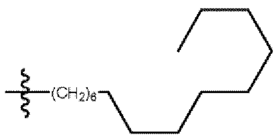,

Oder wobei
$R_a = R_c = H$
$R_b = -CH_2HC=CH_2$, und Rd = $OCH_3$, oder
$R_a = R_c = H$
$R_b = -CH=HC-CH_3$ und
$R_d = OCH_3$ ;
Oder Ra =Rb =Rd = H und

Rc = 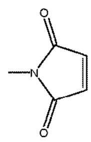,

Oder $R_a = R_b = R_c = H$ und $R_d = CH_2CH=CH_2$ $R_a = R_b = R_d = H$ $R_c = CH_2CH=CH_2$ Oder $R_a = R_c = R_d = H$ und $R_b = CH_2CH=CH_2$;
Oder und und
eine Verbindung der Formel C

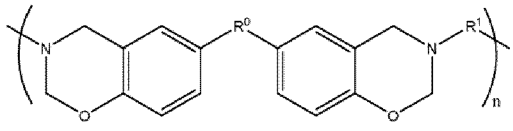

C

Wobei $R^0$ eine $-CH_2-$, $-C(CH_3)_2-$, $SO_2$, $-C(CF_3)_2-$, $-C(CH_3)(C_6H_5)-$, $-C(CH_3)(C_2H_5)-$, $-C(C_6H_5)_2-$, $-CHCH_3-$, $-C_6H_{10}-$ or $-C(CH_3)CH_2CH_2COOH-$ Gruppe ist.
und entweder innerhalb von
n eine ganze Zahl von 1 bis 10 ist;
$R^1$ ist eine $-(CH_2)_n-$Gruppe, mit n=1-10;
Oder

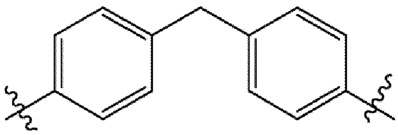

oder

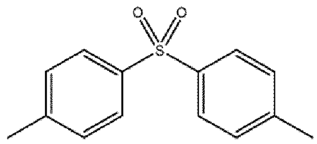

Oder

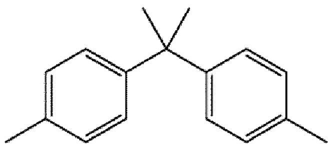

oder

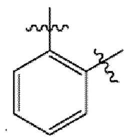

.

## Revendications

1. Benzoxazine contant des groupes hydroxyles aliphatiques libres et un monoester de formule (I)

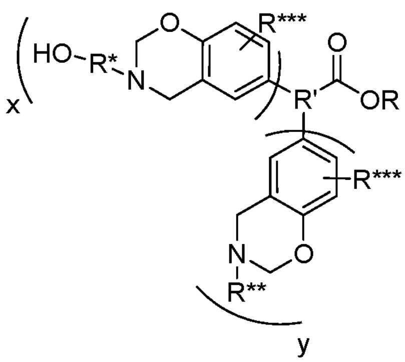

dans laquelle

R est choisi dans le groupe constitué par un groupe alkyle ou alcoxy en $C_1$-$C_{12}$ linéaire ou ramifié, un groupe alcényle ou alkylènoxy en $C_2$-$C_6$ linéaire ou ramifié, un groupe alcynyle en $C_2$-$C_6$, linéaire ou ramifié, substitué ou non substitué, un groupe cyclo(alkyle en $C_3$-$C_6$), un hétérocyclo(alkyle en $C_3$-$C_6$), un groupe phényle non substitué ou substitué par un alkyle en $C_1$-$C_6$ ou un alcényle en $C_2$-$C_6$ linéaire ou ramifié, et un groupe $(CH_2)_{n3}$-phényle, où n3 est un entier de 1 à 10;
R' est choisi dans le groupe constitué par au moins l'un parmi -CH, un groupe $C-(CH_2)_{n3}-CH_3$, un groupe $C-(CH_2)_{n3}-CH-(CH_3)_2$, un groupe $C-(CH_2)_{n3}-(CHZ)_{n4}-(CH_3)_2$, un groupe $C-(CH_2)_{n3}-(CHZ)_{n4}-(CH_2)_{n3}-CH_3$, un groupe $C-(CHZ)_{n4}-(CH_2)_{n3}-CH_3$, un groupe $C-(CHZ)_{n4}-[(CH_2)_{n3}-CH_3]_2$, un groupe C-alcényle en $C_2$-$C_6$ linéaire ou ramifié substitué ou non substitué, un phényle non substitué ou substitué par un alkyle en $C_1$-$C_6$ linéaire ou ramifié ou un phényle comprenant au moins un hétéroatome choisi parmi N, O et S, un groupe phényle $C-(CH_2)_{n3}$

non substitué ou substitué par alkyle en $C_1$-$C_6$ linéaire ou ramifié ou un phényle comprenant au moins un hétéroatome choisi parmi N, O et S, un groupe C-$(CH_2)_{n3}$-phényle non substitué ou substitué par alkyle en $C_1$-$C_6$ linéaire ou ramifié ou un phényle comprenant au moins un hétéroatome choisi parmi N, O et S-$(CH_2)n_3$-$CH_3$, un groupe C-$(CH_2)_{n3}$-phényle non substitué ou substitué par un alkyle linéaire ou ramifié en $C_1$-$C_6$ ou un phényle comprenant au moins un hétéroatome choisi parmi N, O et S-$(CH_2)n_3$-CH-$(CH_3)_2$, un groupe C-$(CH_2)_{n3}$-phényle non substitué ou substitué par alkyle linéaire ou ramifié en $C_1$-$C_6$ ou un phényle comprenant au moins un hétéroatome choisi parmi N, O et S-$(CH_2)_{n3}$-$(CHZ)_{n4}$-$(CH_3)_2$, un groupe C-$(CH_2)_{n3}$-phényle non substitué ou substitué par alkyle linéaire ou ramifié en $C_1$-$C_6$ ou un phényle comprenant au moins un hétéroatome choisi parmi N, O et S-$(CHZ)_{n4}$-$(CH_2)_{n3}$-$CH_3$ et un groupe C-$(CH_2)n_3$-(CHZ)-phényle non substitué ou substitué par alkyle linéaire ou ramifié en $C_1$-$C_6$ ou un phényle comprenant au moins un hétéroatome choisi parmi N, O et S-$(CH_2)_{n3}$-$CH_3$, où n3 et n4, indépendamment, sont un entier de 1 à 10 et Z est choisi dans le groupe consistant en un groupe alkyle ou alcoxy en $C_1$-$C_6$ linéaire ou ramifié, un groupe alcényle ou alkylénoxy en $C_2$-$C_6$ linéaire ou ramifié et un groupe phényle non substitué ou substitué par un alkyle en $C_1$-$C_6$ ou alcényle en $C_2$-$C_6$ linéaire ou ramifié, et au moins un atome d'oxygène est présent entre deux C adjacents ou pas, ou R' est omis ;

R* est choisi dans le groupe constitué par un groupe alkyle ou alcoxy en $C_1$-$C_{20}$ linéaire ou ramifié, un groupe cyclo(alkyle en $C_3$-$C_6$), un hétéocyclo(alkyle en $C_3$-$C_6$), où l'hétéroatome est choisi parmi N, S et O, un groupe alcényle ou alkylénoxy en $C_2$-$C_6$ linéaire ou ramifié, un groupe alcynyle en $C_2$-$C_6$ substitué ou non substitué linéaire ou ramifié, un groupe phényle non substitué ou substitué par un alkyle en $C_1$-$C_6$ ou un alcényle en $C_2$-$C_6$ linéaire ou ramifié, un groupe $(CH_2)_{n3}$-phényle et $(CH_2)_{n3}$-O-$(CH_2)_{n4}$, où n3 et n4, indépendamment, sont un entier de 1 à 10 ;

R** est identique à R* et comprend en outre un membre choisi dans le groupe constitué par un groupe O-, N- ou S-$(CH_2)n_3$-CH-$(CH_3)_2$, un groupe O-, N- ou S-$(CH_2)_{n3}$-$(CHZ)_{n4}$-$(CH_3)_2$, un groupe O-, N- ou S-$(CH_2)_{n3}$-$(CHZ)_{n4}$-$(CH_2)_{n3}$-$CH_3$, un groupe O-, N- ou S-$(CHZ)_{n4}$-$(CH_2)_{n3}$-$CH_3$, un groupe O-, N- ou S-$(CHZ)_{n4}$-$[(CH_2)_{n3}$-$CH_3]_2$, un groupe O-alcynyle en $C_2$-$C_6$ non substitué ou substitué, linéaire ou ramifié, Z étant défini comme pour R', -$(CH_2)_{n3}$-C≡N, un aromatique polycyclique ou un hydrocarbure aromatique hétérocyclique, tel que le naphtalène, anthracène, fluorène ou phénantrène, optionnellement substitué par un groupe alkyle ou alcoxy en $C_1$-$C_6$, un cyclo(alkyle en $C_3$-$C_6$), un hétéocyclo(alkyle en $C_3$-$C_6$), un groupe alcényle ou alkylénoxy en $C_2$-$C_6$ linéaire ou ramifié, ou par un groupe alcynyle en $C_2$-$C_6$ non substitué ou substitué, linéaire ou ramifié, dans lequel n3 et n4, indépendamment, sont un entier de 1 à 10 ;

R*** est choisi dans le groupe constitué par H, OH et O- groupe alkyle en $C_1$-$C_6$ linéaire ou ramifié, et comprend en outre un groupe alkyle en $C_1$-$C_{15}$ linéaire ou ramifié ou un groupe alcényle en $C_2$-$C_{15}$ ou

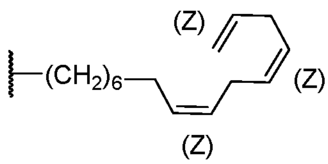 , 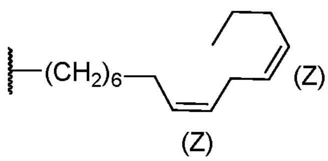 , 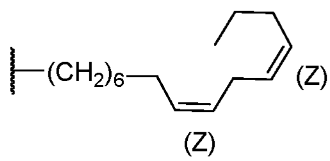 ou

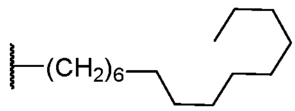 ;

et la valeur de x est de 0 à 1 et la valeur de y est 1 -x.

**2.** Le benzoxazine selon la revendication 1, dans laquelle

R est choisi dans le groupe constitué par un groupe alkyle ou alcoxy en $C_1$-$C_4$ linéaire ou ramifié, un groupe alcényle ou alkylènoxy en $C_2$-$C_4$ linéaire ou ramifié, un groupe alcynyle linéaire ou ramifié en $C_2$-$C_4$, un groupe phényle non substitué et un groupe $(CH_2)_{n3}$-phényle, où n3 est un entier de 1 à 6;

R' est choisi dans le groupe constitué par au moins l'un parmi -CH, un groupe C-$(CH_2)_{n3}$-$CH_3$, un groupe C-$(CH_2)_{n3}$-CH-$(CH_3)_2$, un groupe C-$(CH_2)_{n3}$-$(CHZ)_{n4}$-$(CH_3)_2$, un groupe C-$(CH_2)_{n3}$-$(CHZ)_{n4}$-$(CH_2)_{n3}$-$CH_3$, un groupe C-$(CHZ)_{n4}$-$(CH_2)_{n3}$-$CH_3$, un groupe C-$(CHZ)_{n4}$-$[(CH_2)_{n3}$-$CH_3]_2$, un groupe C-alcényle en $C_2$-$C_4$ linéaire ou ramifié substitué ou non substitué, un phényle substitué ou non comprenant au moins un hétéroatome choisi parmi N, O et S, un groupe C-$(CH_2)_{n3}$-phényle non substitué ou un phényle comprenant au moins un hétéroatome choisi parmi N, O et S, un groupe C-$(CH_2)n_3$-phényle non substitué ou un phényle comprenant au moins un

hétéroatome choisi parmi N, O et S-$(CH_2)n_3$-$CH_3$, un groupe C-$(CH_2)_{n3}$-phényle non substitué ou un phényle comprenant au moins un hétéroatome choisi parmi N, O et S-$(CH_2)_{n3}$-CH-$(CH_3)_2$, un groupe C-$(CH_2)_{n3}$-phényle non substitué ou un phényle comprenant au moins un hétéroatome choisi parmi N, O et S-$(CH_2)_{n3}$-$(CHZ)_{n4}$-$(CH_3)_2$, et un groupe C-$(CH_2)_{n3}$-phényle non substitué ou un phényle comprenant au moins un hétéroatome choisi parmi N, O et S-$(CHZ)_{n4}$-$(CH_2)_{n3}$-$CH_3$ et un groupe C-$(CH_2)_{n3}$-$(CHZ)_{n4}$-phényle non substitué ou un phényle comprenant au moins un hétéroatome choisi parmi N, O et S-$(CH_2)n_3$-$CH_3$, où n3 et n4, indépendamment, sont un entier de 1 à 6 et Z est choisi dans le groupe consistant en un groupe alkyle ou alcoxy en $C_1$-$C_4$ linéaire ou ramifié, un groupe alcényle ou alkylénoxy en $C_2$-$C_4$ linéaire ou ramifié et un groupe phényle non substitué, et au moins un atome d'oxygène est présent entre deux C adjacents ou pas;

R* est choisi dans le groupe constitué par un groupe alkyle ou alcoxy en $C_1$-$C_6$ linéaire ou ramifié, un groupe alcényle ou alkylénoxy en $C_2$-$C_4$ linéaire ou ramifié, un groupe alcynyle en $C_2$-$C_4$ non substitué linéaire ou ramifié, un groupe phényle non substitué, un groupe $(CH_2)_{n3}$-phényle et $(CH_2)_{n3}$-O-$(CH_2)_{n4}$, où n3 et n4, indépendamment, sont un entier de 1 à 6 ;

R** est identique à R* et comprend en outre un membre choisi dans le groupe constitué par un groupe O-, N- ou S-$(CH_2)n_3$-CH-$(CH_3)_2$, un groupe O-, N- ou S-$(CH_2)_{n3}$-$(CHZ)_{n4}$-$(CH_3)_2$, un groupe O-, N- ou S-$(CH_2)_{n3}$-$(CHZ)_{n4}$-$(CH_2)_{n3}$-$CH_3$, un groupe O-, N- ou S-$(CHZ)_{n4}$-$(CH_2)_{n3}$-$CH_3$, un groupe O-, N- ou S-$(CHZ)_{n4}$-$[(CH_2)_{n3}$-$CH_3]_2$, un groupe alcynyle en $C_2$-$C_4$ non substitué ou substitué, linéaire ou ramifié, Z étant défini ci-dessus, un groupe-$(CH_2)_{n3}$-C≡N, un cyclo (alkyle en $C_3$-$C_4$), un groupe hétéorcyclo (alkyle en $C_3$-$C_4$), un aromatique polycyclique ou un hydrocarbure aromatique hétérocyclique, où l'hétéroatome est sélection parmi le groupe comprenant N, S et O, tel que le naphtalène, anthracène, fluorène ou furane, optionnellement substitué par un groupe alkyle ou alcoxy en $C_1$-$C_4$, un groupe alcényle ou alkylénoxy en $C_2$-$C_4$ linéaire ou ramifié, ou par un groupe alcynyle en $C_2$-$C_4$ non substitué ou substitué linéaire ou ramifié, dans lequel n3 et n4, indépendamment, sont un entier de 1 à 6 ;

R*** est choisi dans le groupe constitué par H, OH et O- groupe alkyle en $C_1$-$C_4$ linéaire ou ramifié, et comprend en outre un groupe alkyle en $C_1$-$C_{10}$ linéaire ou ramifié ou un groupe alcényle en $C_2$-$C_{10}$ ou

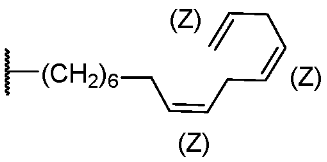
,
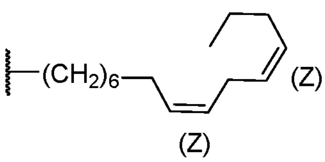
,
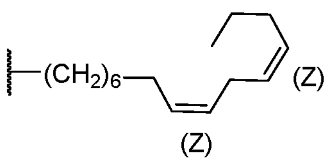

ou

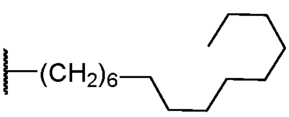
.

3. Le benzoxazine selon la revendication 1, dans laquelle,

R est choisi dans le groupe constitué par -$CH_3$, -$(CH_2)_{n3}$-$CH_3$, -$(CH_2)_{n3}$-CH-$[(CH_2)_{n3}$-$CH_3]_2$, -$C(CH_3)_3$, -$(CH_2)_{n3}$-$(C_6H_5)$, -$(CH_2)_{n3}$-CH=$CH_2$ and -$(CH_2)_{n3}$-C≡CH, où n3 est un nombre entier de 1 à 5 ;

R' est choisi dans le groupe constitué par -CH, -C-$(CH_2)_2$-$C(CH_3)$, -C-$CH(CH_2CH_3)$, - $C(CH_2CH_2CH_3)$, -C-$CH_2(CH_2)_3CH_3$, -C-$CH_2(CH_2)_4CH_3$, -$C(C_6H_5)$, -$C(CH_3)CH_2$, $C(CH_3)CH_2CH_2$, -$C(C_6H_5)CH_2$-$CH_3$ ;

R* est choisi dans le groupe constitué par -$CH_3$, -$(CH_2)_{n3}$-$CH_3$, -$(CH_2)_{n3}$-CH-$[(CH_2)_{n4}$-$CH_3]_2$, -$C(CH_3)_3$, $(CH_2)_{n3}$-$(C_6H_5)$, -$(CH_2)_{n3}$-CH=$CH_2$, -$(CH_2)_{n3}$-C=CH, -$(CH_2)_{n3}$-O-$(CH_2)_{n4}$, où n3 et n4 sont, indépendamment, un nombre entier de 1 à 4, phényle, et $(CH_2)_3$-phényle ;

R** est choisi dans le groupe constitué par $CH_3$, -$(CH_2)_{n3}$-$CH_3$, -$(CH_2)_{n3}$-CH-$[(CH_2)_{n4}$-$CH_3]_2$, -$C(CH_3)_3$, $(CH_2)_{n3}$-$(C_6H_5)$, -$(CH_2)_{n3}$-CH=$CH_2$, -$(CH_2)_{n3}$-C≡CH, O-$(CH_2)_{n3}$-C≡CH, O-$(CH_2)_{n3}$-C≡N, $(CH_2)_{n3}$-C≡N, et -$(CH_2)_{n3}$-furane non substitué ou substitué, phényle, et où n3 et n4 sont, indépendamment, un nombre entier de 1 à 4 ;

R*** est choisi dans le groupe constitué par H, OH et O- groupe alkyle en $C_1$-$C_3$ linéaire ou ramifié, et comprend en outre un groupe alkyle en $C_1$-$C_6$ linéaire ou ramifié ou un groupe alcényle en $C_2$-$C_6$ ou

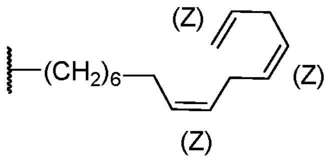,
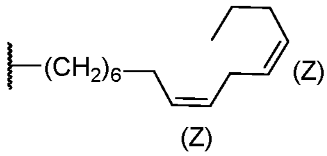,
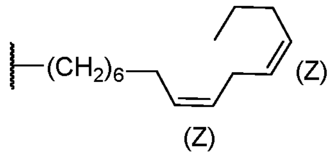

ou

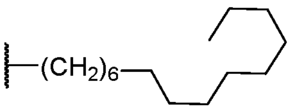,

H étant le plus préféré.

**4.** Procédé (1) de production d'une benzoxazine contenant des groupes hydroxyles aliphatiques libres et un monoester de formule (I) comprenant les étapes suivantes :

a) faire réagir un dérivé d'acide phénolique de formule (II), comprenant au moins un groupe R***,

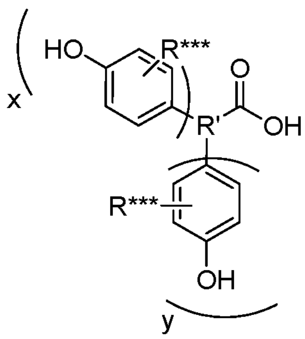

(II)

avec une molécule ou un oligomère monofonctionnel de formule (III)

R-OH (III)

à une température de 80°C à 200°C, pendant 12h-48h, en présence d'un catalyseur de type acide Bronsted, conduisant à une molécule ou un oligomère à terminaison monophénolique de formule (IV)

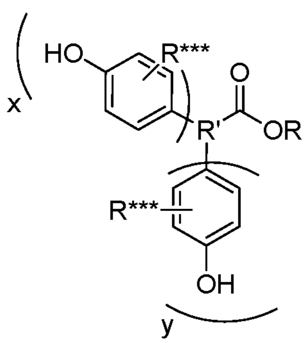

(IV)

et,
b) faire réagir la molécule ou l'oligomère à terminaison phénolique de formule (IV) avec un mélange de

- un aminoalcool de formule (V)

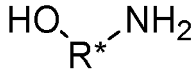

(V)

- un dérivé d'amine primaire de formule (VI)

$R^{**}-NH_2$ (VI),

et
- le paraformaldehyde de formule (VII)

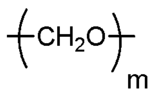

m=8-100

à une plage de températures de 80°C à 100°C, de 1h à 48h, sous agitation, où R, R', R*, R**, R***, x et y sont, indépendamment, tels que définis dans l'une quelconque des revendications 1 à 3, à condition que lorsque l'au moins un R*** du dérivé d'acide phénolique est en position *ortho* par rapport au groupe -OH, alors R *** est H.

**5.** Procédé selon la revendication 4, dans lequel le dérivé d'acide phénolique (formule (II)) comprend de 1 à 4 groupe(s) R***, lié à la substitution du cycle phénolique.

**6.** Procédé selon la revendication 4 ou 5, dans lequel le dérivé d'acide phénolique est choisi dans le groupe constitué par les dérivés d'acide mono-, di-, tri-hydroxybenzoïques, les dérivés d'acide anacardique, les dérivés d'acide hydroxycinnamique, les dérivés d'acides X-hydroxyphényles aliphatiques, dans lesquels X est 2-4, et les dérivés d'acides diphénoliques aliphatiques, ou les mélanges de ceux-ci.

**7.** Procédé selon l'une quelconque des revendications 4 à 6, dans lequel la stoechiométrie respective des réactifs de départ à l'étape a), dérivé d'acide phénolique: molécule ou oligomère monofonctionnel est de 1,0-3,0 eq.:1,0 eq, conduisant à un 1,0 éq. d'une molécule ou d'un oligomère à terminaison phénolique.

**8.** Procédé selon l'une quelconque des revendications 4 à 7, dans lequel les dérivés amines primaires portent R** ayant la définition de R * et sont en outre choisis dans le groupe constitué par l'allylamine, la méthylamine, l'éthylamine, la propylamine, la butylamine, l'isopropylamine, l'hexylamine, la cyclohexylamine, le 2-aminofluorène, l'aminophénylacétylène, l'éther propargylique, l'aniline, le 4-aminobenzonitrile, la furfurylamine et l'aniline, ou leurs mélanges, dans lequel l'amino-alcool de formule (V) est choisi dans le groupe constitué par le 2-aminoéthanol, le 2-amino-2-méthylpropanol, le 5-aminopentan-1-ol, l'heptaminol et la diglycolamine, ou leurs mélanges.

**9.** Procédé selon l'une quelconque des revendications 4 à 9, dans lequel la stoechiométrie respective des réactifs de départ à l'étape b), oligomère ou molécule à terminaison monophénolique:amino-alcool:dérivé d'amine primaire: paraformaldéhyde est de 1,0 éq.:x(1,0 éq-18,0 éq): y(1,0 eq-18,0 éq): 2,0-36,0 éq, conduisant à 1,0 eq. de benzoxazine contenant des groupes hydroxyle aliphatiques libres et un monoester, où x = 0-1 et y = 1-x.

**10.** Catalyseur de polymérisation par ouverture de cycle de benzoxazine (ROP) comprenant une benzoxazine contenant des groupes hydroxyles aliphatiques libres et un monoester de formule (I) selon l'une quelconque des revendications 1 à 3 ou tel que pouvant être obtenu selon le procédé (1) selon l'une quelconque des revendications 4 à 9.

**11.** Utilisation d'une benzoxazine contenant des groupes hydroxyles aliphatiques libres et d'un monoester de formule (I) selon l'une quelconque des revendications 1 à 3 ou telle qu'elle peut être obtenue selon le procédé (1) selon l'une quelconque des revendications 4 à 9 ou d'un monomère de benzoxazine contant un ester de formule (XX)

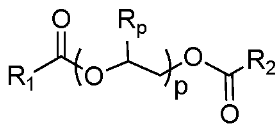

(XX)

où,

$R_1$ est

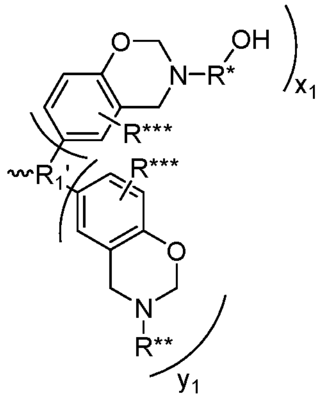

; $R_2$ est

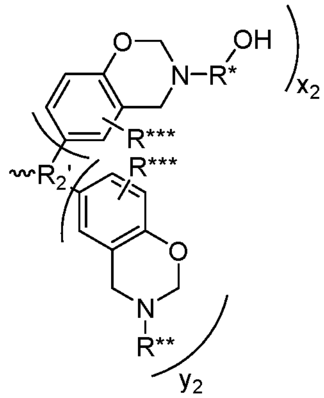

et

$R_p$ est choisi dans le groupe constitué par H, un groupe alkyle ou alcoxy, linéaire ou ramifié, en $C_1$-$C_6$, de préférence en $C_1$-$C_4$, un groupe alcényle ou alkylénoxy en $C_2$-$C_6$, de préférence en $C_2$-$C_4$, linéaire ou ramifié, un groupe alcynyle en $C_2$-$C_6$, de préférence en $C_2$-$C_4$, non substitué ou substitué, linéaire ou ramifié, un groupe phényle non substitué ou substitué par un alkyle en $C_1$-$C_6$, de préférence en $C_1$-$C_4$, ou un alcényle $C_2$-$C_6$, de préférence $C_2$-$C_4$, linéaire ou ramifié et

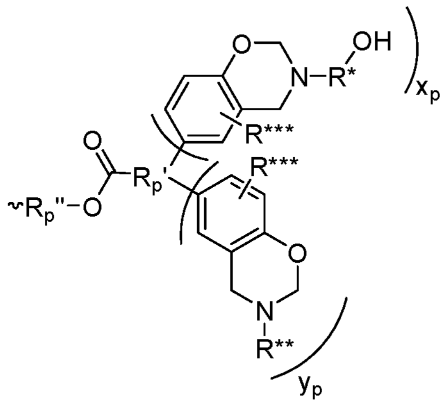

où,

$R_1$ et $R_2$ de la formule (XX) sont identiques ou différents ;
$x_1$, $x_2$ et $x_p$, indépendamment, sont de 0 à 1 et ne font pas ensemble 1;
$y_1$ = 1-$x_1$ ; $y_2$ = 1-$x_2$ ; $y_p$ = 1-$x_p$ ;
p vaut de 1 à 100 ;
$R_1$', $R_2$' et $R_p$', indépendamment, sont choisis dans le groupe consistant en H, -C-groupe alkyle ou alcoxy en $C_1$-$C_6$ linéaire ou ramifié, -C-groupe alcényle ou alkylénoxy en $C_2$-$C_6$ linéaire ou ramifié, -C-groupe alcynyle en $C_2$-$C_6$ linéaire ou ramifié substitué ou non substitué, et -C-groupe phényle non substitué ou substitué par un groupe alkyle en $C_1$-$C_6$ ou alcényle en $C_2$-$C_6$ linéaire ou ramifié ;
$R_p$" est choisi dans le groupe constitué par H, un groupe alkyle ou alcoxy en $C_1$-$C_6$ linéaire ou ramifié, un groupe alcényle ou alkylénoxy en $C_2$-$C_6$ linéaire ou ramifié, un groupe alcynyle en $C_2$-$C_6$ linéaire ou ramifié

substitué ou non substitué, et un groupe phényle non substitué ou substitué par un groupe alkyle en $C_1$-$C_6$ ou alcényle en $C_2$-$C_6$ linéaire ou ramifié ;
R*, R** et R*** sont, indépendamment, comme définis selon l'une des revendications 1 à 3,
dans lequel
$x_1$, $x_2$, $x_p$, $y_1$, $y_2$, $y_p$ sont définis comme suit :

$$x_1 = \frac{n_{aminoalcohol(R1)}}{n_{amines(R1)}^{total}}$$

$$x_2 = \frac{n_{aminoalcohol(R2)}}{n_{amines(R2)}^{total}}$$

$$x_p = \frac{n_{aminoalcohol(Rp)}}{n_{amines(Rp)}^{total}}$$

$$y_1 = \frac{n_{amines\ (R1)}}{n_{amines\ (R1)}^{total}}$$

$$y_2 = \frac{n_{amines\ (R2)}}{n_{amines\ (R2)}^{total}}$$

$$y_p = \frac{n_{amines(Rp)}}{n_{amines\ (Rp)}^{total}}$$

dans lequel $n_{amine(R1)}^{total} = n_{amines(R1)} + n_{aminoalcohol(R1)}$ , et sont le nombre d'amino-alcool dans le groupe $R_1$, $n_{amines(R1)}$ représente le nombre d'amines (à l'exception du nombre d'amino-lacool) par groupe $R^1$ et $n_{amine(R1)}^{total} = n_{amines(R1)} + n_{aminoalcohol(R1)}$ est le nombre total de groupes amines par groupe $R_1$;

dans lequel $n_{amine(R2)}^{total} = n_{amines(R2)} + n_{aminoalcohol(R2)}$ , et $n_{aminoalcohol(R2)}$ sont le nombre d'amino-alcool dans le groupe $R_2$, $n_{amines(R2)}$ représente le nombre d'amines (à l'exception du nombre d'amino-lacool) par groupe $R_2$ et $n_{amine(R2)}^{total} = n_{amines(R2)} +$ $n_{aminoalcohol(R2)}$ est le nombre total de groupes amines par groupe $R_2$;

dans lequel $n_{amine(Rp)}^{total} = n_{amines(Rp)} + n_{aminoalcohol(Rp)}$ , et $n_{aminoalcohol(Rp)}$ sont le nombre d'amino-alcool dans le groupe Rp, $n_{amines(Rp)}$ représente le nombre d'amines (à l'exception du nombre d'amino-lacool) par groupe $R_p$ et $n_{amine(Rp)}^{total} = n_{amines(Rp)} +$ $n_{aminoalcohol(Rp)}$ est le nombre total de groupes amines par groupe $R_p$, en tant que catalyseur de polymérisation du benzoxazine.

**12.** Procédé (2) pour synthétiser un monomère du benzoxazine contenant un ester de formule (XX) comprenant les étapes suivantes consistant à :

a) faire réagir un dérivé d'acide phénolique de formule (XXI), comprenant au moins un groupe R *** sur le cycle phénolique,

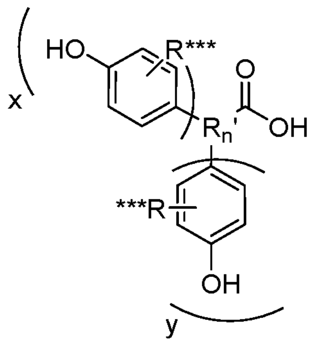

(XXI)

où x est de 0 à 1, et y = 1-x,
avec une molécule ou un oligomère polyfonctionnel de formule (XXII)

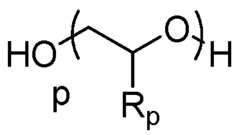

(XXII)

à une température de 25°C à 200°C, pendant 1h-72h, en présence d'un catalyseur de type acide de Bronsted, conduisant à un oligomère ou une molécule à terminaison phénolique (composé (XXIII))
et
b) faire réagir le composé (XXIII)) avec un mélange de

- un aminoalcool de formule (V)

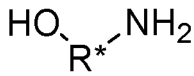

(V)

- un dérivé d'amine primaire de formule (VI)

$R^{**}\text{-}NH_2$ (VI),

et
- le paraformaldehyde de formule (VII)

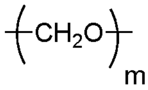

m=8-100,

à une plage de température de 80°C à 100°C, de 1h à 10h, sous agitation, pour l'obtention du composé de formule (XX),
où $R_p$, $R_1$ et $R_2$, R*, R**, R***, $R_n$' étant $R_1$' ou $R_2$', $X_1$, $x_2$, $x_p$, $y_1$, $y_2$, $y_p$ et p sont, indépendamment, tels que définis dans l'une quelconque des revendications 1 à 3 et 12, à condition que lorsque l'au moins un R*** du dérivé d'acide phénolique est en position *ortho* par rapport au groupe -OH, alors R*** est H.

**13.** Procédé (3) de préparation d'un dérivé de polybenzoxazine comprenant l'étape de polymérisation d'une composition comprenant une benzoxazine contenant des groupes hydroxyle aliphatiques libres et monoester de formule (I) ou comme pouvant être obtenue par le procédé (1) selon une des revendications 4-9, ou un monomère de benzoxazine contenant un ester de formule (XX) selon la revendication 3 ou comme pouvant être obtenu par le procédé (2) suivant la revendication 2, ou un mélange de ceux-ci, comme catalyseur de benzoxazine, et comprenant de 0% en

poids à 99% en poids d'un dérivé de benzoxazine, différent dudit catalyseur de benzoxazine, à des températures situées dans la gamme de 100°C à 250°C, pendant 1h à 24h, pour l'obtention de dérivés de polybenzoxazine.

**14.** Procédé (3) selon la revendication 13, dans lequel la proportion de chacun des benzoxazine contenant des groupes hydroxyles aliphatiques libres et monoester de formule (I) ou du monomère benzoxazine contenant un ester de formule (XX) dans leur composition est comprise entre 0,5% en poids et 95% en poids, mieux de 1 à 50% en poids, le plus préférablement de 5 à 10% en poids.

**15.** Procédé (3) selon la revendication 13 ou 14, dans lequel le dérivé de benzoxazine différent du catalyseur est une classe de composés choisis dans le groupe constitué par
un monomère 3,4-dihydro-2H-1,3-benzoxazine ayant la formule

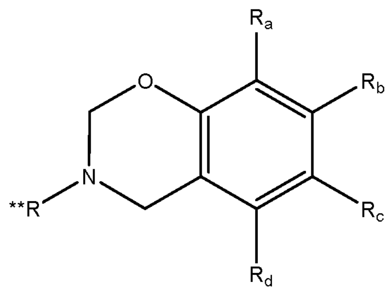

soit, dans laquelle

$R_a = CH_3$
$R_b$ et $R_c$ représentent conjointement

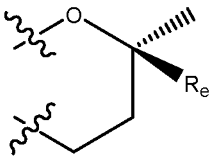

avec

$R_e$ =

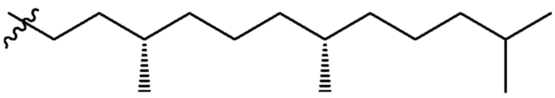

ou

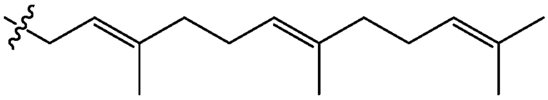

,

et
$R_d$ = H, ou $CH_3$,
soit dans laquelle
$R_d$ et $R_c$ représentent conjointement

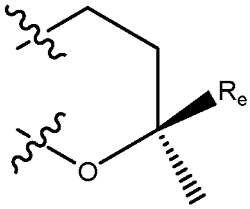

avec

$R_e$ = 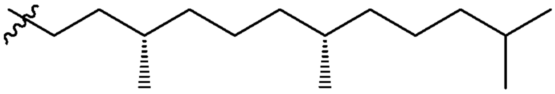

ou

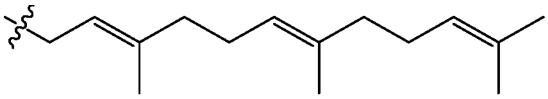,

et

$R_b = R_a = CH_3$,

soit dans laquelle

$R_c = R_d = H$,

$R_b$ = 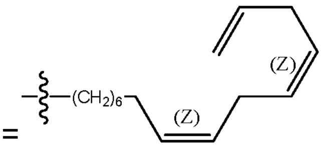, 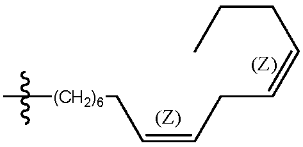, 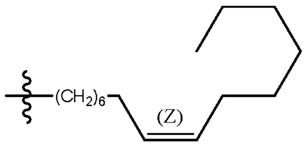

et/ou

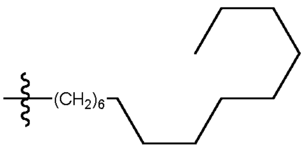

et $R_a$ = COOH,

soit dans laquelle

$R_a$ = H, $R_b$ = OH, $R_c$=H et

$R_d$ = 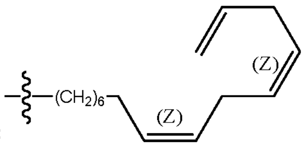, 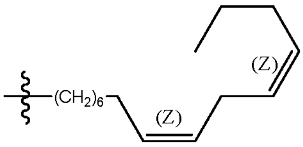, 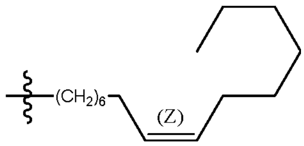

et/ou

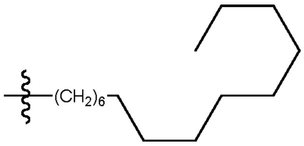
;

soit dans laquelle

$R_a$= H, et

$R_b$ = 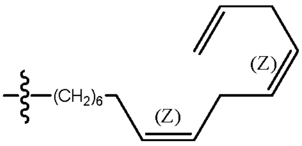 , 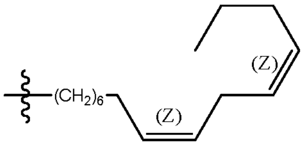 , 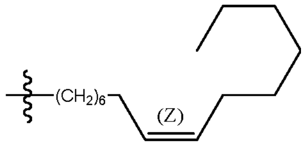

et/ou

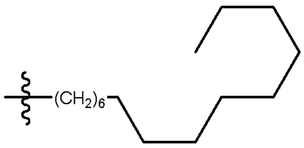

et $R_c$= H, $R_d$= OH,

soit dans laquelle

$R_a$ = $CH_3$, $R_b$ = OH, $R_c$ = H

$R_d$ = 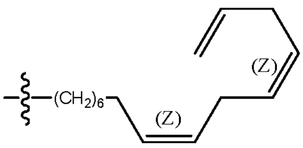 , 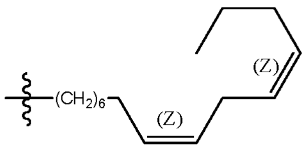 , 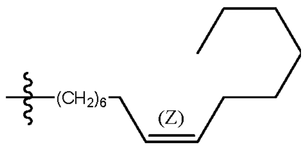

et/ou

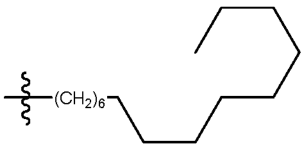

soit dans laquelle

$R_a$ = $R_c$ = $R_d$ = H, et

$R_b$ = 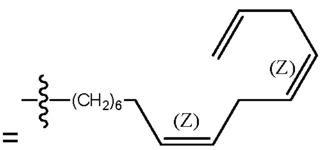 , 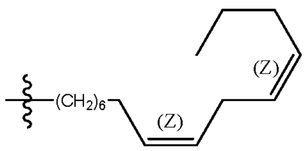 , 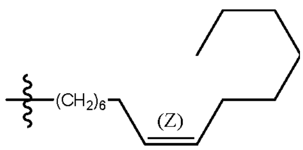

et/our

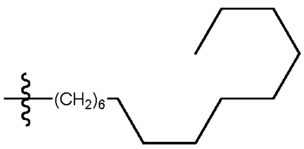

soit dans laquelle
$R_b = R_d = H$
$R_c = -CH_2-HC=CH_2$ et
$R_a = OCH_3$ ;
ou
$R_b = R_d = H$
$R_c= -CH=HC-CH_3$ et
$R_a = OCH_3$,
ou leurs mélanges, R** étant défini selon l'une des revendications 1-3; un composé de formule A, B ou C, ou leur mélange, comme suit Composé de formule A :

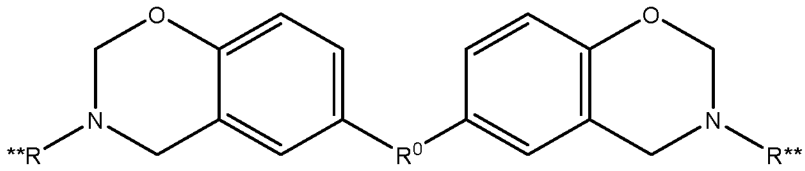

A

où
$R^0$ est un groupe $-CH_2-$, $-C(CH_3)_2-$, $SO_2$, $-C(CF_3)_2-$, $-C(CH_3)(C_6H_5)-$, $-C(CH_3)(C_2H_5)-$, $-C(C_6H_5)_2-$, $-CHCH_3-$, $-C_6H_{10}-$ ou $-C(CH_3)CH_2CH_2COOH-$ ;
R** est un groupe $-CH_2CH_2OH$, vinyle, méthyle, éthyle, propyle, isopropyle, butyle, hexyle, cyclohexyle, fluorène, phénylacétylène, éther phenyl-propargyle, benzonitrile, furfuryle, phénylène ou $-(CH_2)_{17}CH_3$ ;
Composé de formule B :

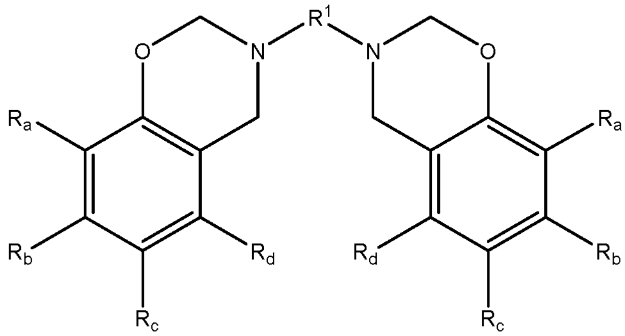

B

soit dans laquelle
$R^1$ est un $-(CH_2)_n-$, avec n=1-10 ou

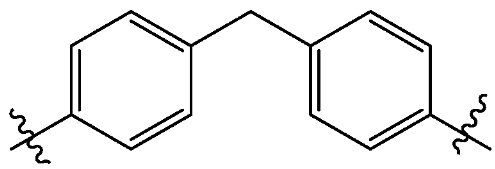

ou

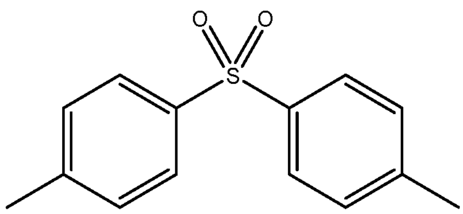

ou

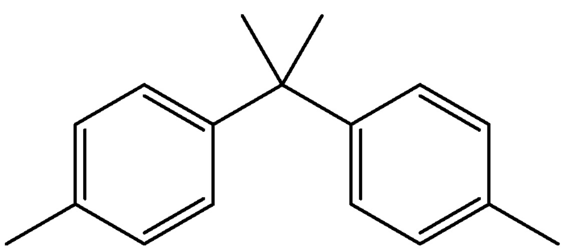

ou

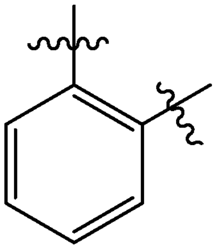 ou 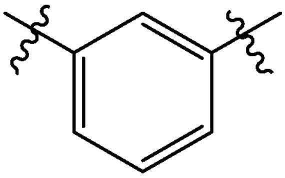 ou 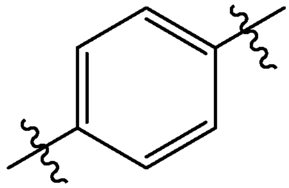

ou

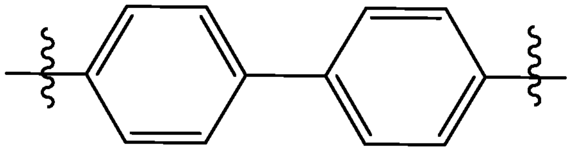

ou

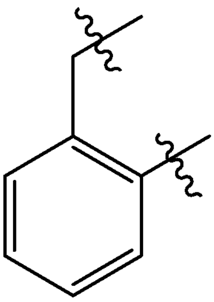 ou 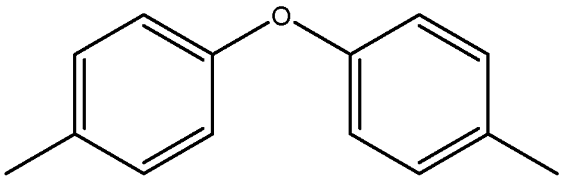

ou

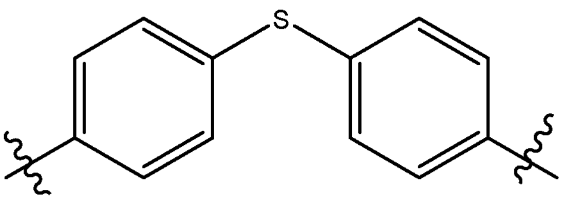 ou 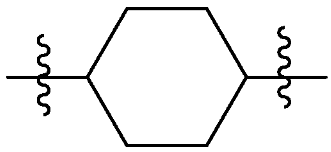 ,

et

où $R_a$, $R_b$, $R_c$, $R_d$ sont choisis dans le groupe constitué par

$R_a = R_b = R_c = R_d = H$,

$R_a = OCH_3$, $R_b = R_d = H$, $R_c = CH_2\text{-}CH\text{=}CH_2$,

$R_a = OCH_3$, $R_b = R_d = H$, $R_c = CH\text{=}CH\text{-}CH_3$,

$R_a = OCH_3$, $R_b = R_d = H$, $R_c = CHO$,

$R_a = OCH_3$, $R_b = R_c = R_d = H$,

$R_a = OCH_3$, $R_b = R_d = H$, $R_c = CHO$,

$R_a = OCH_3$, $R_b = R_d = H$, $R_c = CH_2CH_2COOH$,

$R_a = R_b = R_d = H$, $R_c = CH_2CH_2COOH$,

$R_a = R_b = R_d = H$, $R_c = CH\text{=}CHCOOH$, et

$R_a = OCH_3$, $R_b = R_d = H$, $R_c = CH\text{=}CHCOOH$,

soit dans laquelle

$R_c = R_d = H$,

$R_b$ = 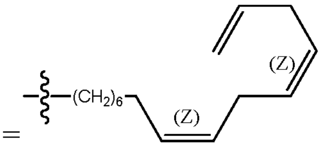, 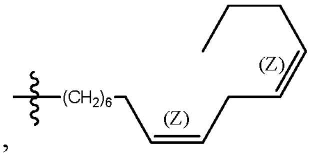, 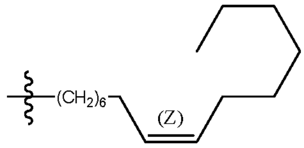

ou

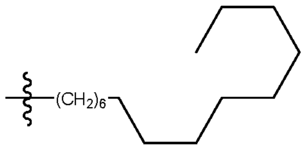,

et $R_a$ = COOH,
soit dans laquelle
$R_a$ = H, $R_b$ = OH, $R_c$=H et

$R_d$ = 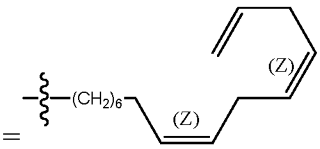, 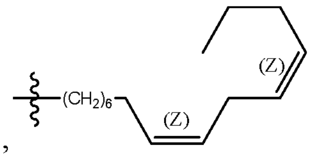, 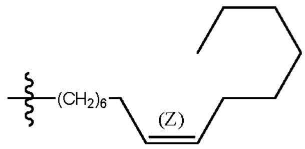

ou

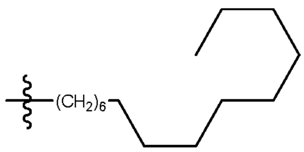,

soit dans laquelle
$R_a$= H, et

$R_b$ = 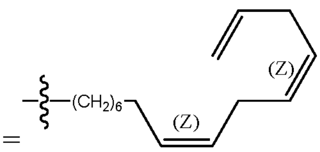, 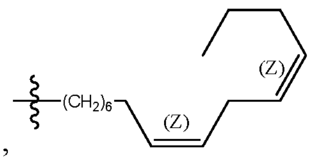, 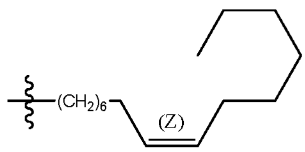

ou

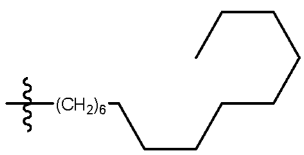,

et $R_c$= H, $R_d$= OH,
soit dans laquelle
$R_a$ = $CH_3$, $R_b$ = OH, $R_c$ = H

$R_d$ = 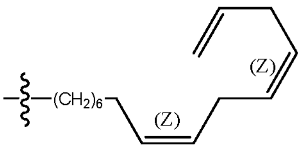 , 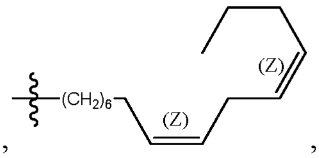 , 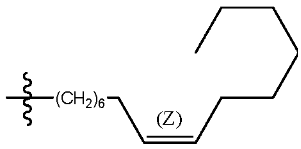

ou

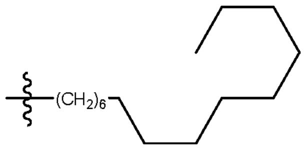 ,

soit dans laquelle
$R_a = R_c = R_d = H$, et

$R_b$ = 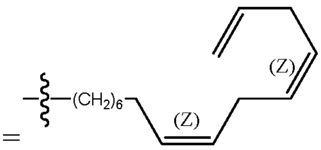 , 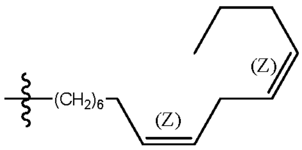 , 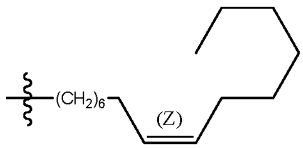

ou

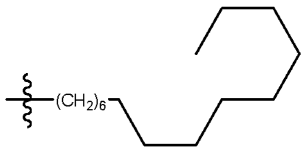 ,

soit dans laquelle
$R_a = R_c = H$
$R_b = -CH_2HC{=}CH_2$, et $R_d = OCH_3$ ;
ou
$R_a = R_c = H$
$R_b = -CH{=}HC\text{-}CH_3$ et
$R_d = OCH_3$,
ou $R_a = R_b = R_d = H$ et

$R_c$ = 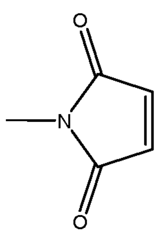 ,

ou $R_a = R_b = R_c = H$ et $R_d = CH_2CH{=}CH_2$
ou $R_a = R_b = R_d = H$ et $R_c = CH_2CH{=}CH_2$
ou $R_a = R_c = R_d = H$ et $R_b = CH_2CH{=}CH_2$; et
Composé C :

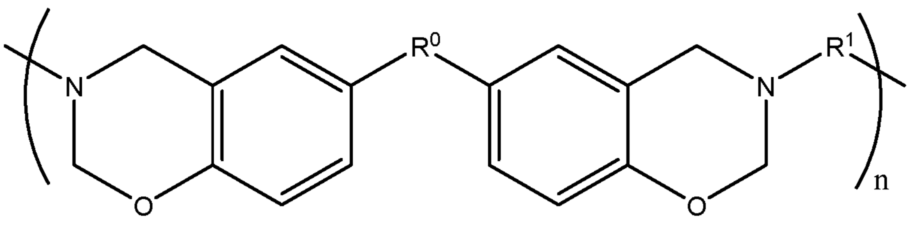

C

où $R^0$ est un groupe $-CH_2-$, $-C(CH_3)_2-$, $SO_2$, $-C(CF_3)_2-$, $-C(CH_3)(C_6H_5)-$, $-C(CH_3)(C_2H_5)-$, $- C(C_6H_5)_2-$, $-CHCH_3-$, $-C_6H_{10}-$ ou $-C(CH_3)CH_2CH_2COOH-$ ;

et soit dans laquelle

n est un nombre entier de 1 to 10 ;

$R^1$ est un groupe $-(CH_2)_n-$, avec n=1-10, ou

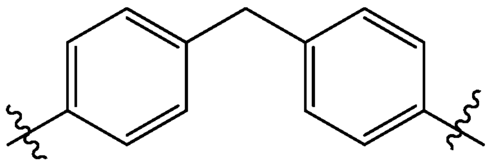

ou

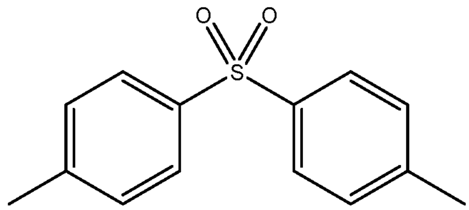

ou

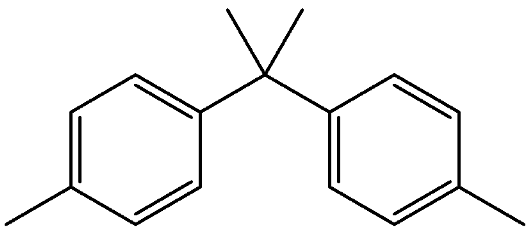

ou

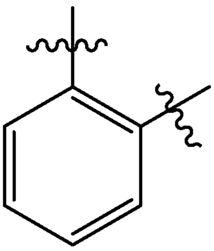

.

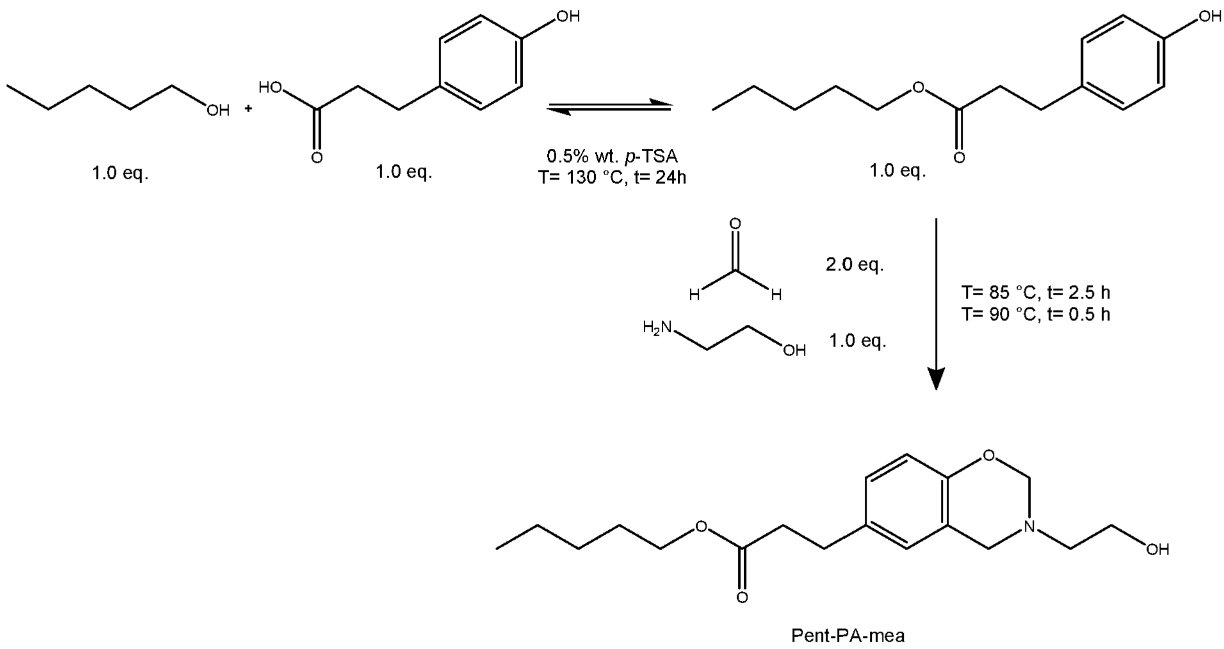
1.0 eq.
1.0 eq.
0.5% wt. p-TSA
T= 130 °C, t= 24h
1.0 eq.
2.0 eq.
1.0 eq.
T= 85 °C, t= 2.5 h
T= 90 °C, t= 0.5 h
Pent-PA-mea

Fig. 1

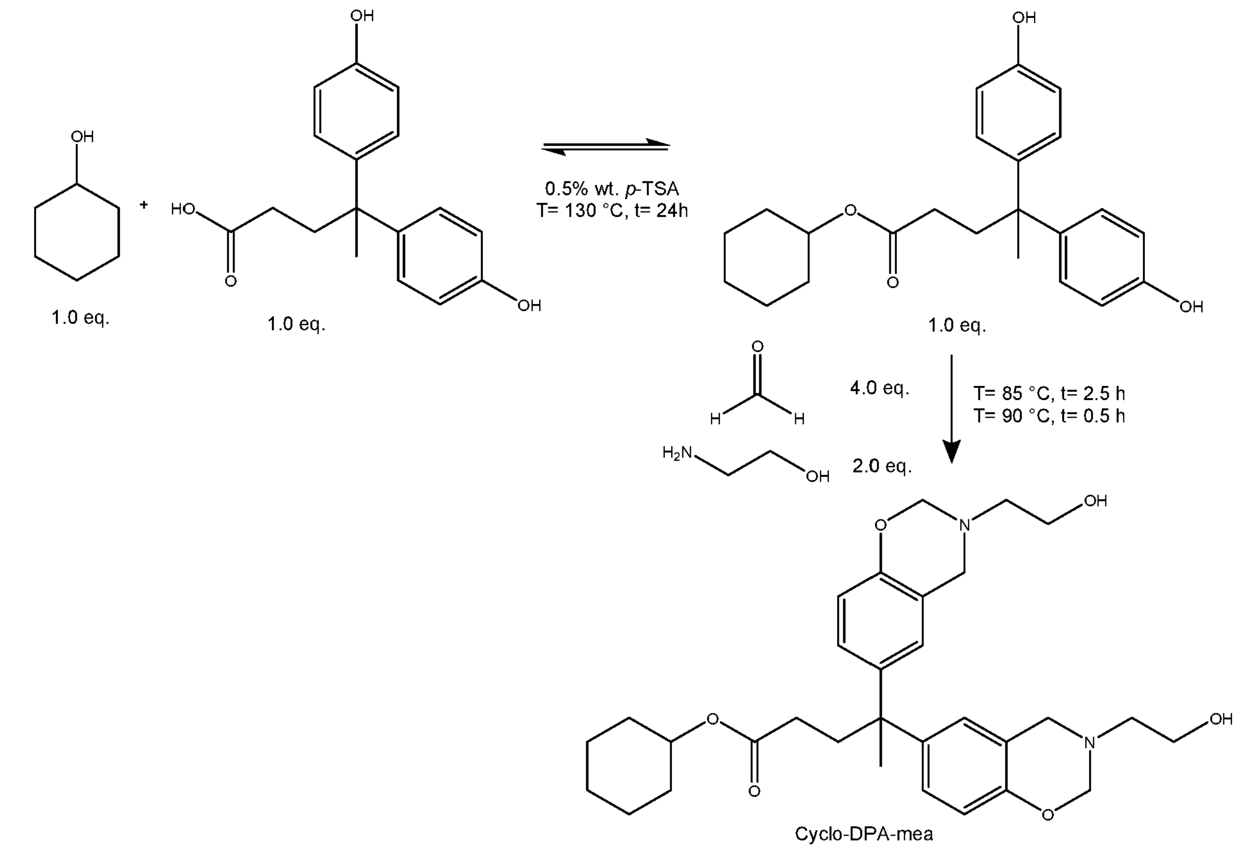


Cyclo-DPA-mea

Fig. 2

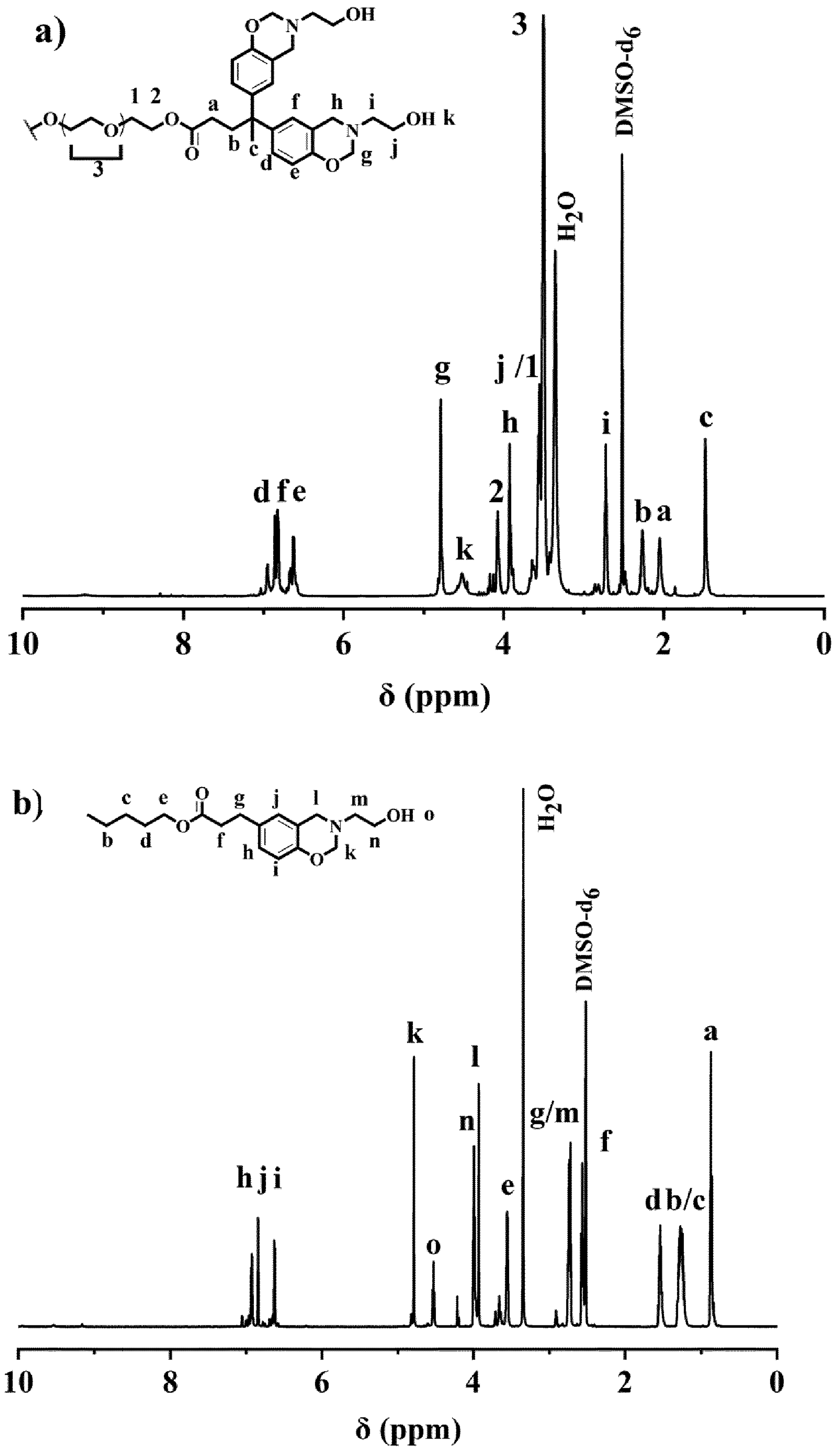
a)
3
DMSO-d$_6$
H$_2$O
g
j /1
h
i
c
2
b a
d f e
k
10
8
6
4
2
0
δ (ppm)
b)
H$_2$O
DMSO-d$_6$
k
a
l
n
g/m
f
e
h j i
d b/c
o
10
8
6
4
2
0
δ (ppm)

Fig. 3

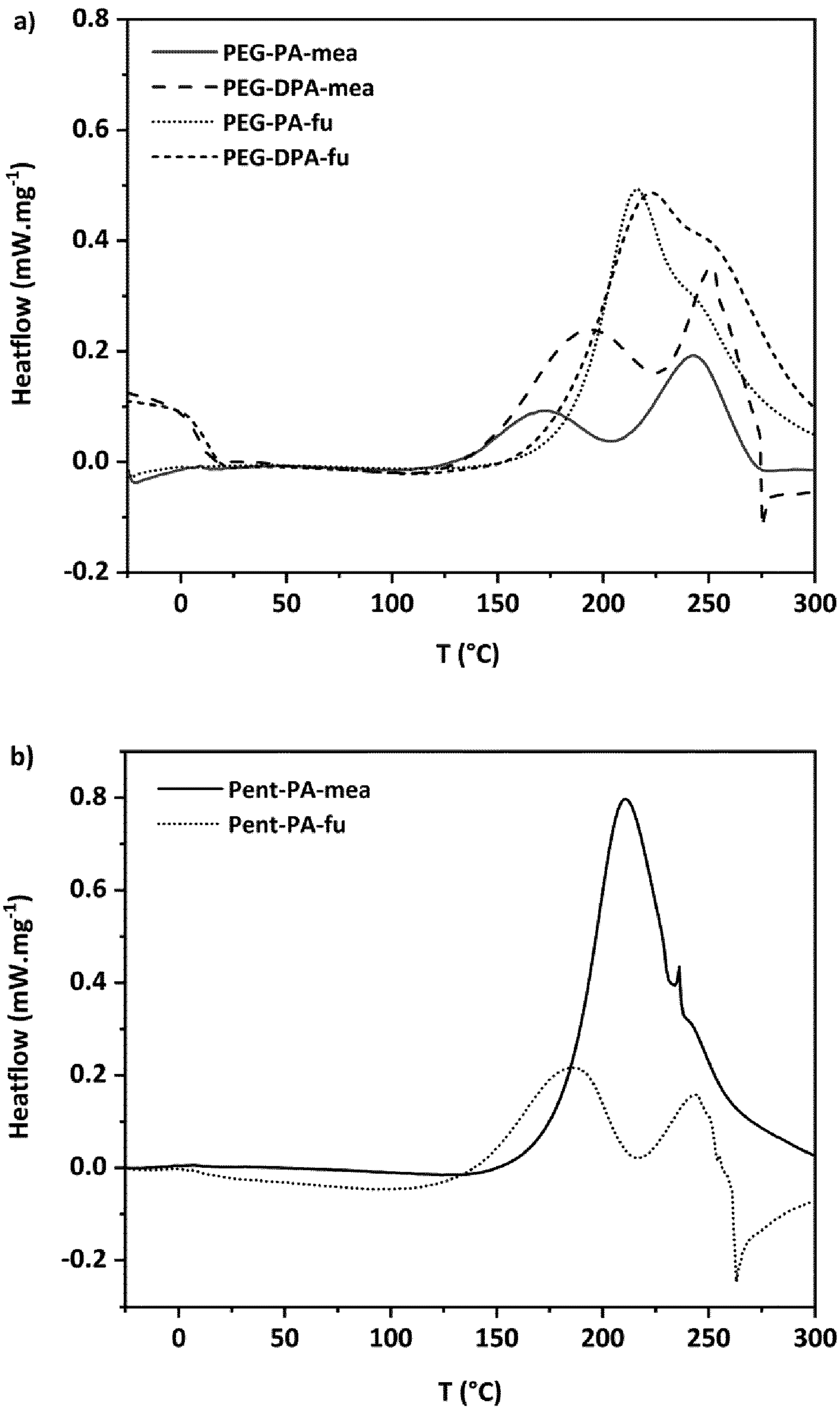
a)
0.8
0.6
0.4
0.2
0.0
-0.2
Heatflow (mW.mg-1)
PEG-PA-mea
PEG-DPA-mea
PEG-PA-fu
PEG-DPA-fu
0
50
100
150
200
250
300
T (°C)
b)
0.8
0.6
0.4
0.2
0.0
-0.2
Heatflow (mW.mg-1)
Pent-PA-mea
Pent-PA-fu
0
50
100
150
200
250
300
T (°C)

Fig. 4

a)

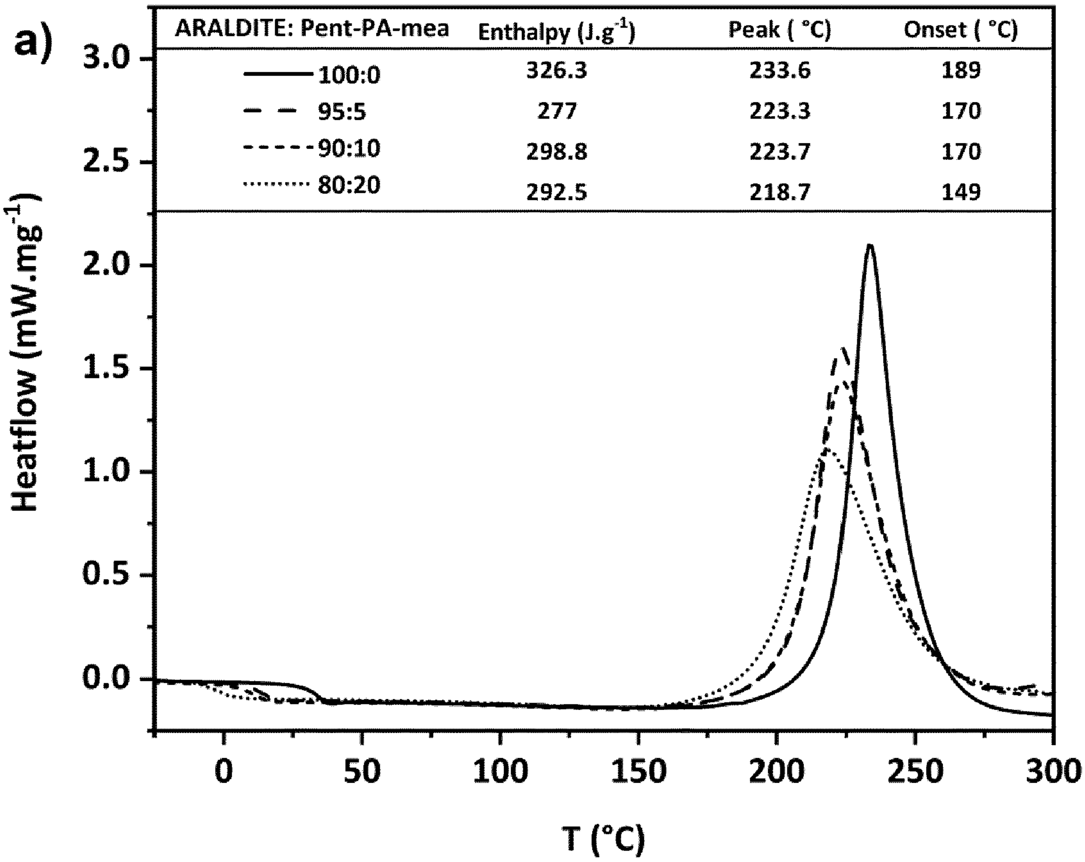
ARALDITE: Pent-PA-mea
Enthalpy (J.g-1)
Peak ( °C)
Onset ( °C)
100:0 326.3 233.6 189
95:5 277 223.3 170
90:10 298.8 223.7 170
80:20 292.5 218.7 149
Heatflow (mW.mg-1)
3.0
2.5
2.0
1.5
1.0
0.5
0.0
0
50
100
150
200
250
300
T (°C)

b)

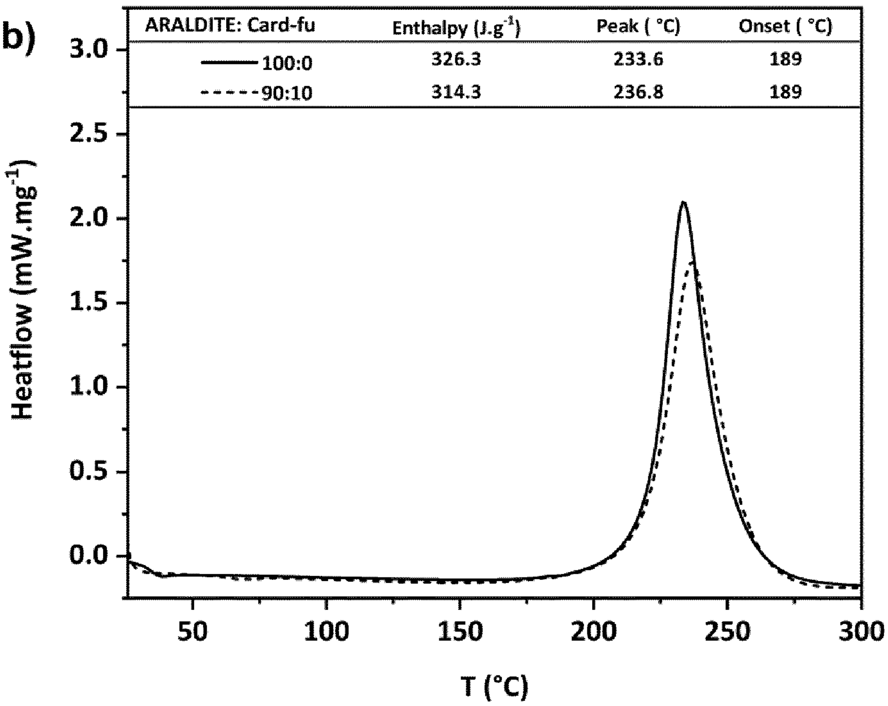
ARALDITE: Card-fu
Enthalpy (J.g-1)
Peak ( °C)
Onset ( °C)
100:0 326.3 233.6 189
90:10 314.3 236.8 189
Heatflow (mW.mg-1)
3.0
2.5
2.0
1.5
1.0
0.5
0.0
50
100
150
200
250
300
T (°C)

Fig. 5

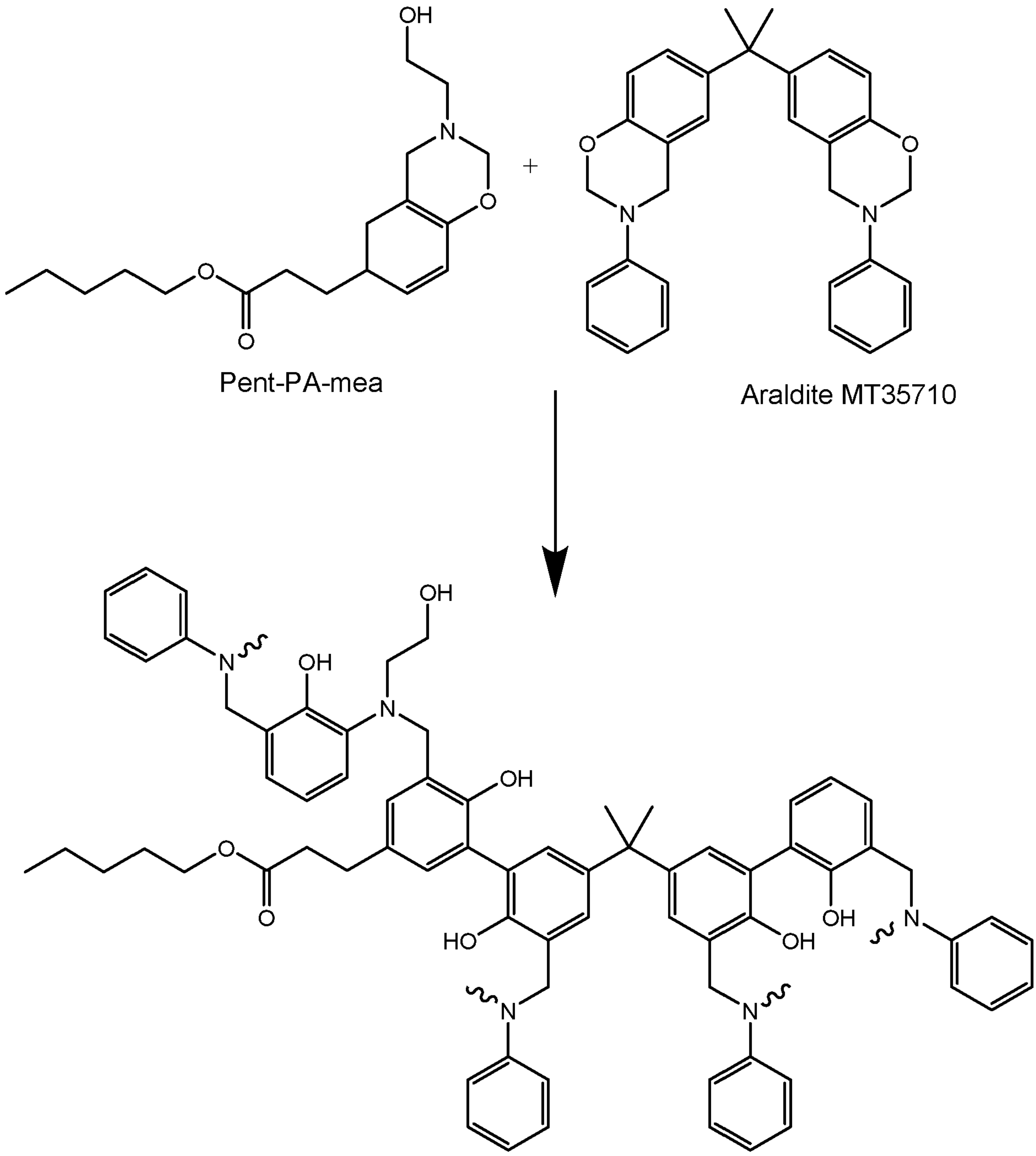

Pent-PA-mea

Araldite MT35710

Fig. 6

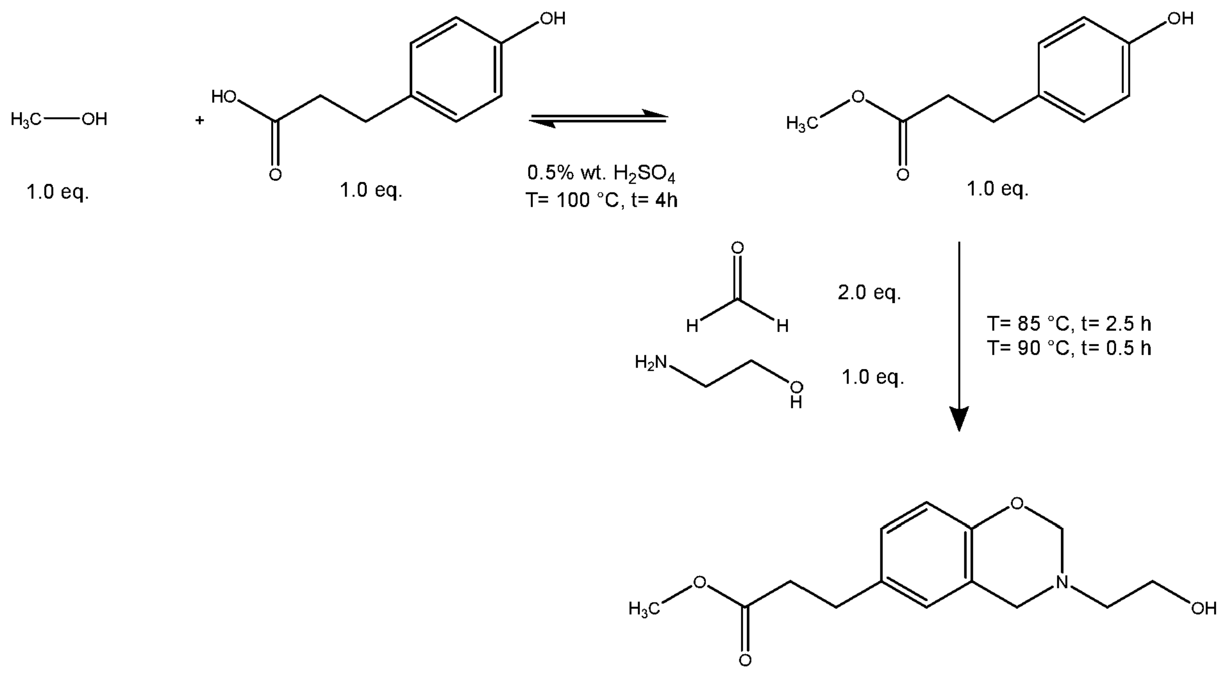


Me-PA-mea

Fig. 7

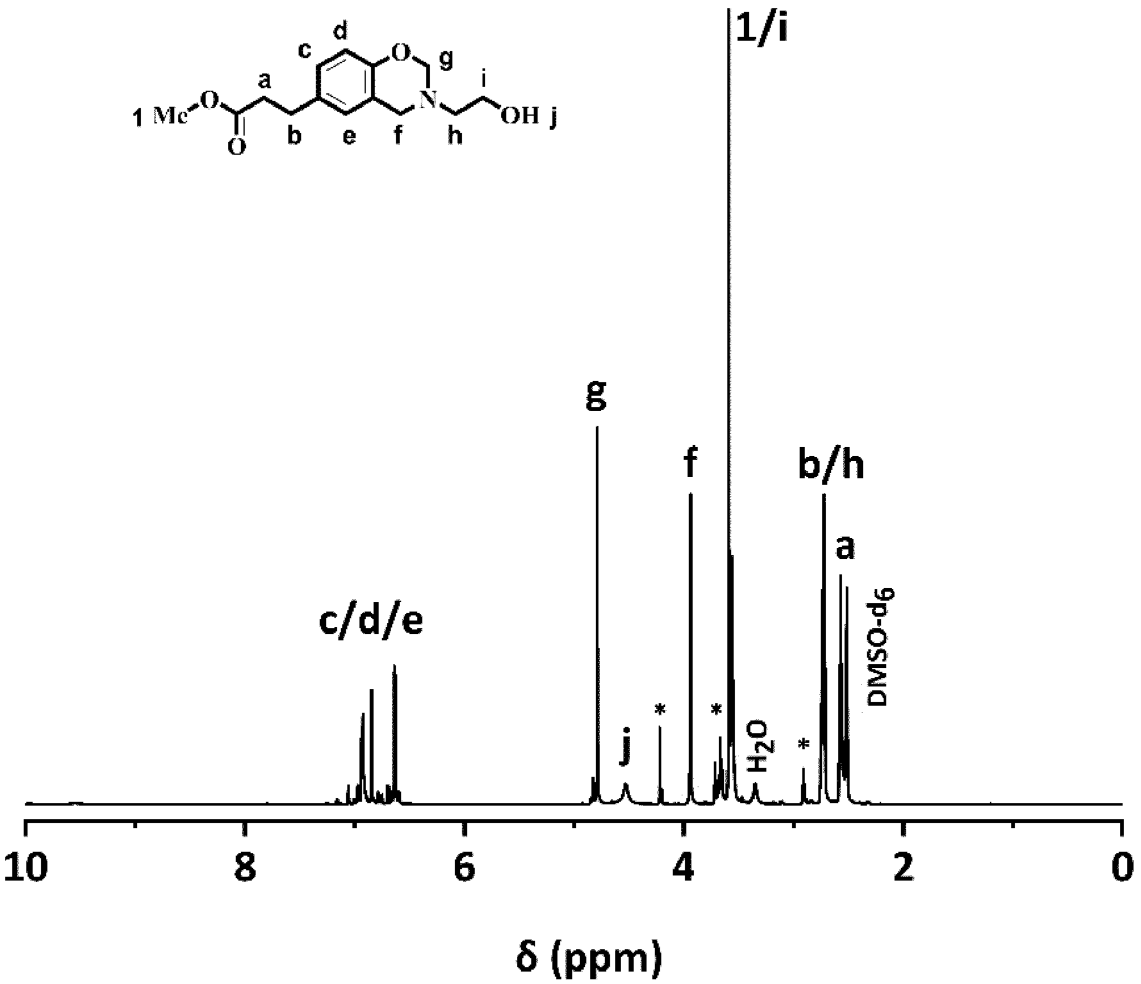


Fig. 8

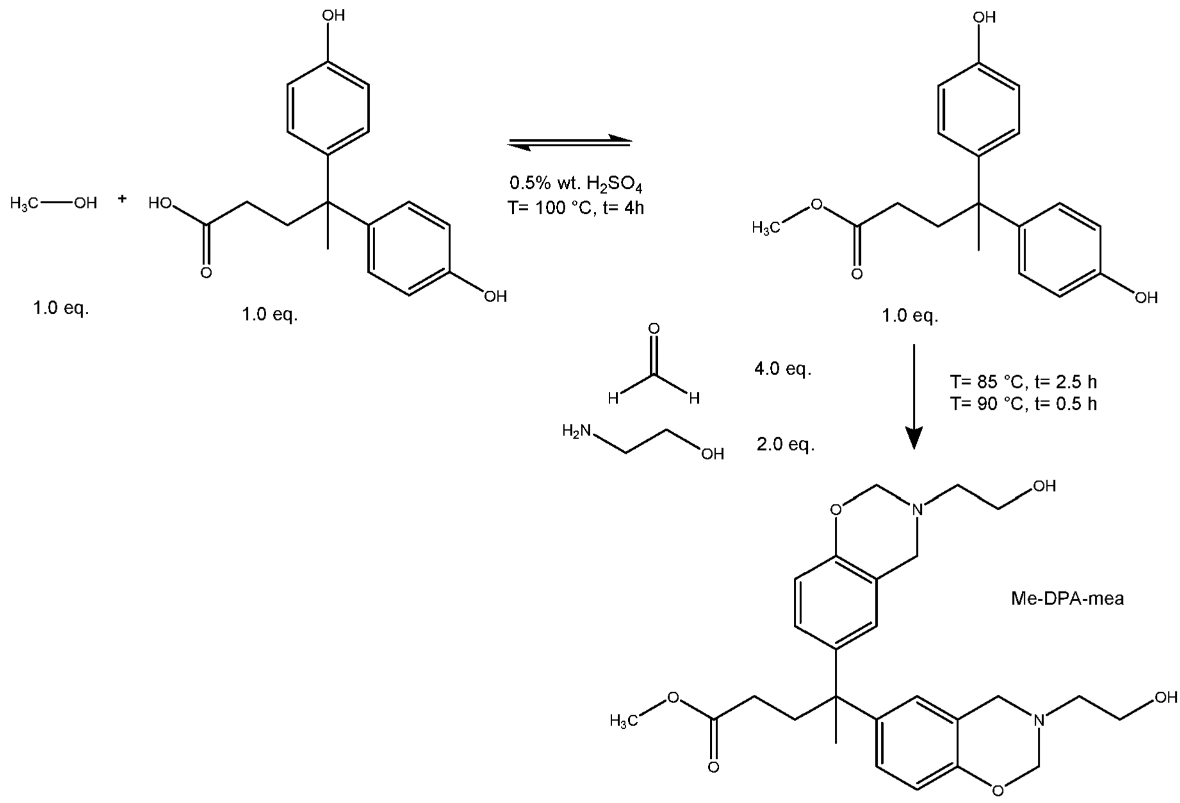
0.5% wt. $H_2SO_4$
T= 100 °C, t= 4h
1.0 eq.
1.0 eq.
1.0 eq.
4.0 eq.
2.0 eq.
T= 85 °C, t= 2.5 h
T= 90 °C, t= 0.5 h
Me-DPA-mea

Fig.9

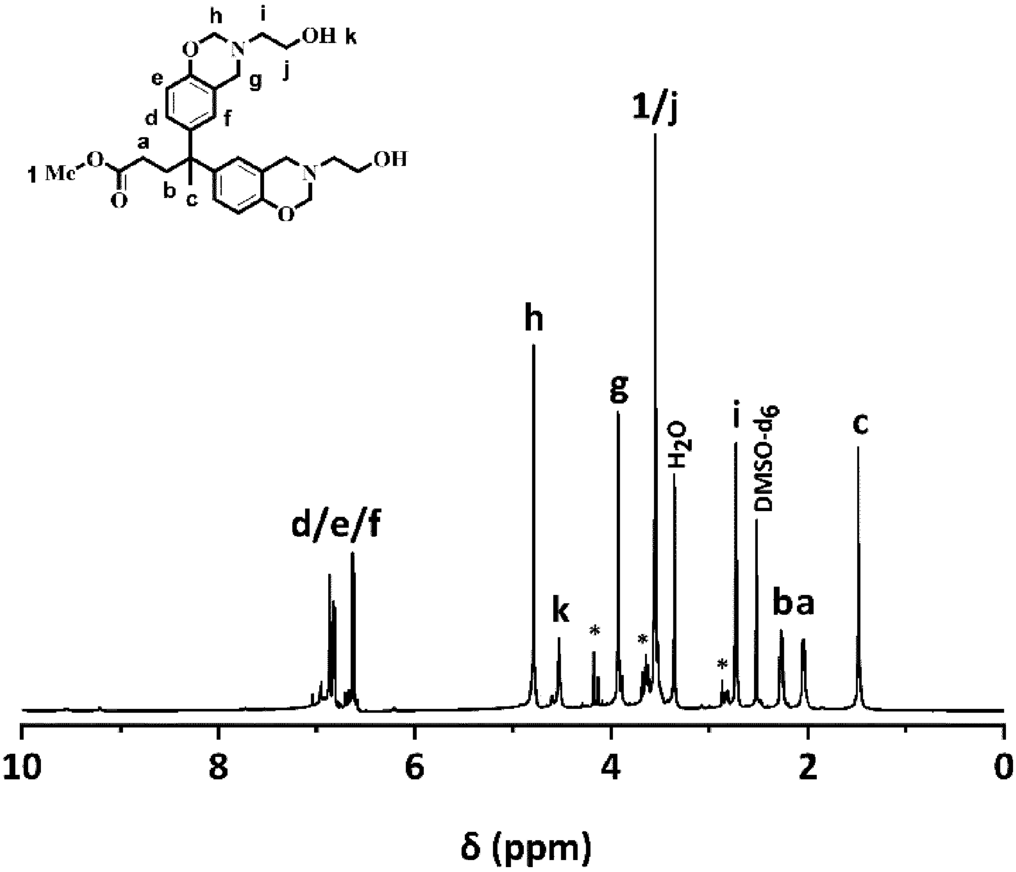


Fig. 10

a)

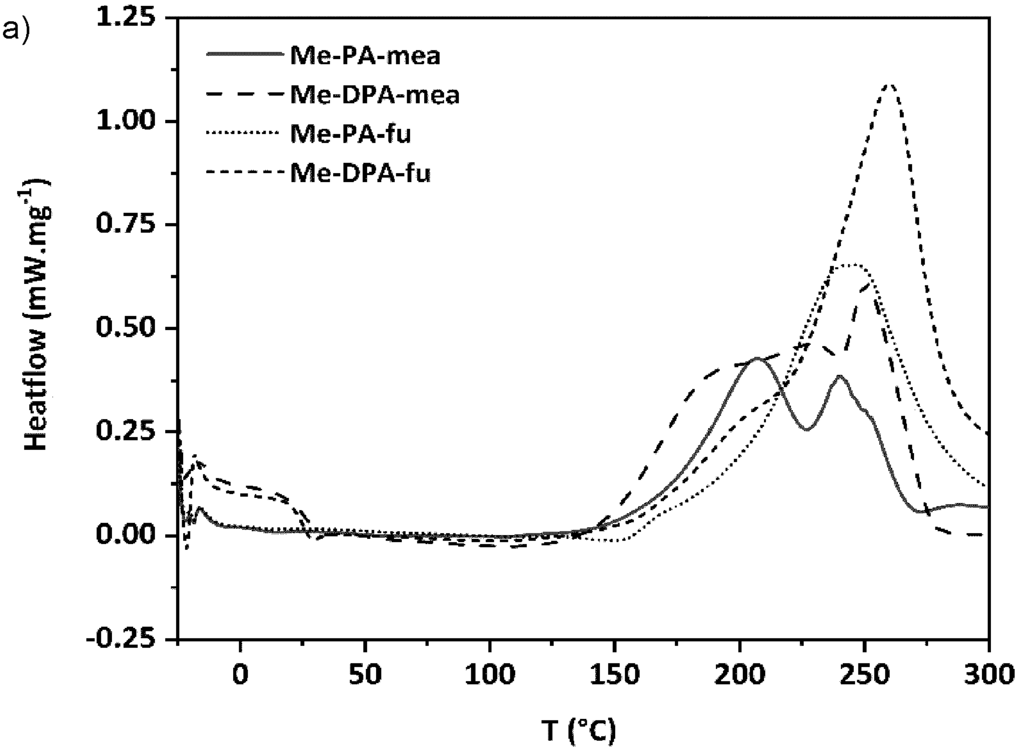
Me-PA-mea
Me-DPA-mea
Me-PA-fu
Me-DPA-fu
Heatflow (mW.mg-1)
T (°C)

b)

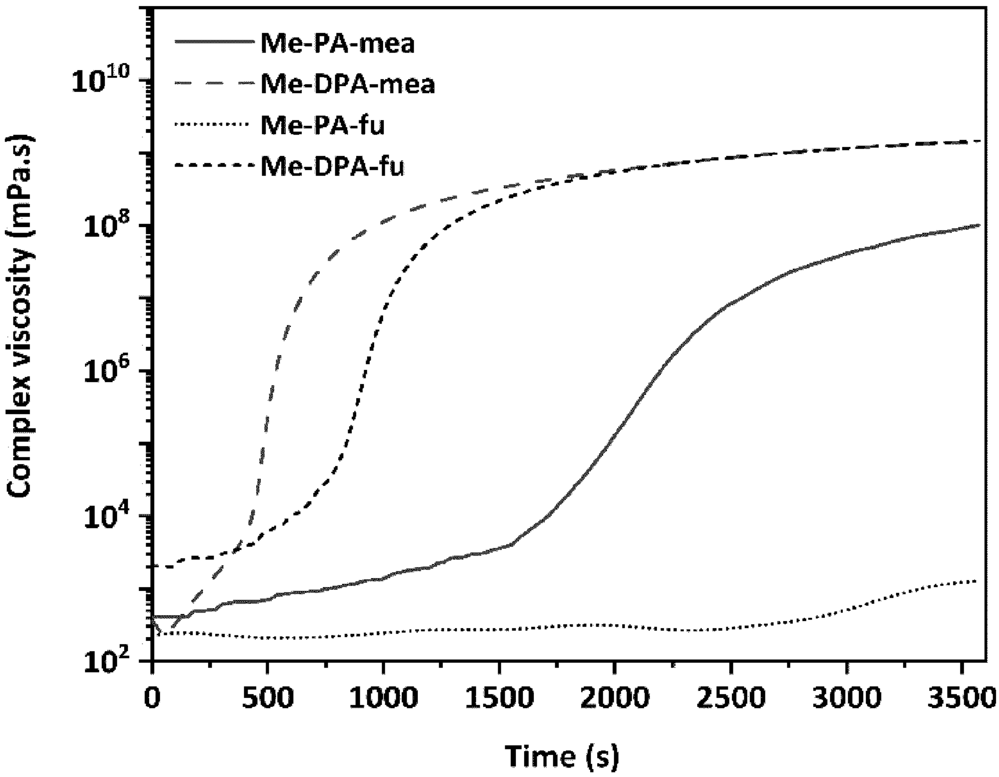
Me-PA-mea
Me-DPA-mea
Me-PA-fu
Me-DPA-fu
Complex viscosity (mPa.s)
Time (s)

Fig. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010018198 A1
- WO 2020193293 A1


### Non-patent literature cited in the description


- **LIU CHAO et al.** Catalyst effects on the ring-opening polymerization of 1,3-benzoxazine and on the polymer structure. *POLYMER,* 01 May 2013, vol. 54 (12), ISSN 0032-3861, 2873-2878